# EUROPEAN PATENT APPLICATION

(11) **EP 4 647 071 A1**
(43) Date of publication of application: **12.11.2025**
(21) Application number: 24175294.8
(22) Date of filing: 10.05.2024
(51) Int. Cl.: A61K 31/18, A61K 31/4402, A61K 31/505, A61P 19/02

(54) **MITOCHONDRIAL COMPLEX I MODULATOR FOR USE IN THE TREATMENT OF ARTHRITIS**

(71) Applicant: Istesso Therapeutics Limited, London N1C 4AG (GB)
(72) Inventor: FOSTER, Martyn Leslie, Benfleet, SS7 5JQ (GB); PATEL, Lisa, London, N1C 4AG (GB)
(74) Representative: Mewburn Ellis LLP

(57) **Abstract**

The invention provides mitochondrial complex I modulator (MCIM) compounds, which find utility in the treatment of arthritis, such as RA. The MCIM compounds find further utility in the treatment of physical functional decline and physical disabilities, such as age-related functional decline and disabilities. Treatment with MCIM compounds repair damage caused by arthritic disease and functional decline to improve patient quality of life.

## Description

### Field of the Invention

The present invention relates to mitochondrial complex I modulator (MCIM) compounds for use in treating arthritis and age-related musculoskeletal disabilities, and particularly, although not exclusively, to the use of MCIM compounds to reverse bone erosion and osteitis.

### Background

Rheumatoid arthritis is a chronic and systemic autoimmune disease characterised by synovial inflammation, peri-articular bone loss and cartilage destruction, leading to joint destruction, pain, progressive disability, fatigue and osteoporosis (Aletaha and Smolen, 2018; Guo et al, 2018; Smolen et al, 2018; Michaud et al, 2020), all of which negatively impact health-related quality of life. When compared with the general population, RA confers increased risk of mortality in the order of a 1.3- to 3-fold elevation (Riise et al, 2001). The prevalence of RA has been estimated at 51 per 10,000 worldwide (Almutairi et al, 2021), which based on the current world population of approximately 8 billion, provides an estimate of 41 million people affected by RA.

The pathogenesis of RA is not completely understood **(Error! Reference source not found,** and S chett, 2017; **Error! Reference source not found.,** et al 2018). Systemic features include cardiovascular **(Error! Reference source not found.,** 2019), pulmonary **(Error! Reference source no t found,** et al, 2019), psychological **(Error! Reference source not found.** et al, 2017) and skeletal disorders **(Error! Reference source not found,** and Humphery, 2017), and RA can also have a d evastating effect on patients' quality of life **(Error! Reference source not found.** et al, 2016; **Error! Reference source not found.** et al, 2019). Changes in functional capacity in RA as assessed using the Health Assessment Questionnaire-disability index (HAQ-DI) accompany progression of the disease but are also seen in normal ageing (Krishnan et al, 2004).

Treatment of RA is aimed at limiting and controlling disease activity, with the aim of preventing long-term functional loss and disability. A wide range of drugs are available (Ferrari et al, 2015). The current treatment regimen for RA consists of initial treatment with non-steroidal anti-inflammatory drugs (NSAIDs), low-dose glucocorticoids and non-biologic disease-modifying anti-rheumatic drugs (DMARDs) such as the immunosuppressant methotrexate (MTX) (Smolen et al, 2018). Second- and third-line therapy is increasingly focussed on the biologic and targeted synthetic disease-modifying anti-rheumatic drugs (bDMARDs and tsDMARDs, respectively) (Smolen et al, 2017; Aletaha and Smolen, 2018; Smolen et al, 2018; Fleischmann, 2020) , the most commonly used of which are antitumour necrosis factor alpha (TNFα) agents such as adalimumab, infliximab and etanercept (Smolen et al, 2014; Smolen et al, 2015; Jinesh, 2015) and the JAK inhibitors (JAKi), such as tofacitinib (Xeljanz^{®}) (Rakieh and Conaghan, 2013; Lundquist et al, 2014) , baricitinib (Olumiant^{®}) (Taylor, 2019; Fleischmann et al, 2017a; Fleischmann et al 2017b) and upadacitinib (Rinvoq^{®})(Serhal and Edwards, 2019).

Whilst these agents have demonstrated their efficacy in reducing the signs and symptoms of disease and the inhibition of progression of structural damage (Bizzi et al, 2014; Pierreisnard et al, 2013) , a high failure rate (Smolen et al 2015; Siebert et al, 2015; Rubbert-Roth et al, 2019) necessitates switching between agents (Bonafede et al, 2016; Nam et al, 2017; Morinobu, 2020; Smolen et al, 2020). In addition, these drugs predominantly act as anti-inflammatory agents (Guo et al 2018; Tanaka, 2019), and none directly address the mechanism of structural damage (Kleyer et al, 2014; Panagopoulos and Lambrou, 2018; Liu et al, 2020) despite disability stemming from bone and joint destruction being the most debilitating feature of the disease (Guidelli et al, 2015; Cipolletta et al 2020). Indeed, up to 70% of patients continue to report symptoms despite treatment (Taylor et al, 2019), including functional impairment, fatigue and pain. Furthermore, although prevention of disability progression relies on maintaining patients in sustained remission, the majority of patients do not achieve a clinical remission based on disease activity score (DAS28) (Einarsson et al, 2019) and even fewer achieve the more stringent targets required to achieve remission based on the ACR/EULAR-definition (Michaud et al, 2020).

Given the importance of inducing remission to prevent disability progression, remission is increasingly becoming a treatment goal in patients with RA. The "treat to target" (T2T) concept defines treatment goals of clinical remission based on composite assessments of tender and swollen joint counts, and patient or physician assessments of disease or pain, followed by long-term prevention of joint destruction to preserve physical function (Studenic et al, 2022). Examples of composite assessments are the Disease Activity Score (DAS) 28-C-reactive protein (CRP) [DAS28-CRP], the clinical disease activity index (CDAI) and the simplified disease activity index (SDAI), whilst joint destruction can be measured using the van der Heijde's modified total sharpness score (van der Heijde's-mTSS), RAMRIS or ultrasound methods.

It has also been proposed that remission should be maintained throughout the course of the disease (Smolen et al, 2010) since any increase in disease activity may reignite the destructive progress. However, there is limited evidence that sustained remission (SR) by itself halts progression of joint damage and disability long-term. One alternative approach to targeting structural progression in rheumatoid arthritis to improve disability has been the testing of agents that prevent non-inflammatory bone loss such as denosumab. However, whilst this agent was efficacious in preventing bone loss it had no effect on measures of cartilage loss or bone inflammation (osteitis/bone marrow oedema) and showed no effect on disability in multiple clinical trials (Hu et al, 2022).

Functional status measured by the Health Assessment Questionnaire (HAQ) is a major outcome in RA. The HAQ has been shown to be among the strongest predictors of long-term outcomes in RA, including work disability and mortality, even when compared to radiographic progression (Wolfe and Hawley, 1998; Wolfe et al, 2003). Indeed, functional remission is an independent predictor of sustained remission in RA. However, disability progression is not used routinely in clinical practise, despite preservation of function being the goal or treatment (Lee et al, 2017). Indeed, whilst the HAQ-disability index is monitored in clinical trials as a component of the American College of Rheumatology 20/50/70 response criteria, it is absent from other more routinely used clinical indices. Furthermore, it has been shown that reliance solely on traditional disease activity targets as treatment goals underestimates the impact of the disease on pain, function, and health for a meaningful proportion of patients (Michaud et al, 2021).

Thus, whilst current treatments for arthritis reduce the signs and symptoms of disease, such as tender and swollen joint counts, assessments of pain and systemic inflammation, they largely fail to directly address the structural progression of the disease, and its consequent impact on disability. Indeed, as well as failing to promote tissue repair by exerting a direct effector response on repair processes as a consequence of their immunosuppressive mode of action these agents also disrupt normal bone dynamics by suppressing signals required for repair. As such, their suppression of the pro-inflammatory effector cell and molecular response proceeds cyclically, perhaps for months or years. In rheumatoid arthritis, such persistent inflammation within the bone erosion prevents erosions from healing (Berardi *et al,* 2021). Indeed, it has been shown that suppression of synovitis and pannus formation does not result in complete suppression of erosions and bone loss, which is reflected in the continued structural progression and functional decline observed in patients with RA despite treatment and its impact on minimum thresholds of disability improvement (minimum clinically important difference).

A similar situation may occur in other chronic age-related musculoskeletal diseases and disabilities, such as osteoarthritis, or, indeed, in general ageing where the kinetic interplay between the drive to injury and sequential activation of repair may result in 'futile' repair, i.e., creation of maladapted restitution of tissue remodelling to a normal state. Indeed, disability is associated with many chronic conditions, where it has been assessed using the HAQ tool and variants thereof. These include rheumatoid arthritis associated cachexia (Lin et al, 2019), psoriatic arthritis (Leung et al, 2021), systemic sclerosis (Clements et al, 1999), cutaneous sclerosis (Allanore et al 2020), gout (Alvarez-Hernández et al, 2008), ankylosing spondylitis (Zochling, 2011), scleroderma (Khanna et al, 2008), systemic lupus erythematosus (Colangelo et al, 2009), polymyalgia rheumatica (Leung et al, 2023), tendinitis (Cota et al, 2023), inflammatory myopathies (Danieli et al, 2023), osteoarthritis (Romaniello et al, 2022) and fibromyalgia (Altintas and Melikoglu, 2022) and other conditions such as haemophilia (De la Corte-Rodriguez et al, 2017), AIDS/HIV (Sousa et al, 2008) and hypophosphatasia (Dahir et al, 2023). Progressive disability resulting from futile repair may also occur, analogously, in ageing itself where, both for men and women, functional disability as assessed by measures such as the HAQ-DI increases exponentially with age (Krishnan et al, 2004; Sokka et al, 2006). Indeed, it has been proposed that the HAQ-DI can be used to assess any type of condition including normal ageing (Health Assessment Questionnaire Disability Index (HAD-QI), 2023). The result of either failure to repair or futile repair is that most patients with chronic disease experience ongoing symptoms and disease progression despite treatment, as described above for rheumatoid arthritis. As such, there is a pressing need for therapeutics with a 'dual pharmacology' that can act directly to target the structural progression of the disease, but also improve health-related quality of life and function/disability. Such a therapeutic would:
- display a different profile of efficacy to conventional immunosuppressant anti-inflammatory drugs such as disease-modifying anti-rheumatic drugs.
- rapidly inhibit or arrest structural progression of disease in the joints.
- produce improvements in disability, fatigue, systemic inflammation and health-related quality of life without reliance on clinical composite scores or symptom assessments, such as tender or swollen joint counts.

The present invention has been devised in light of the above considerations.

### Summary of the Invention

Current treatments for chronic, progressive conditions, such as arthritis, inhibit disease progression but have failed to promote tissue repair. Consequently, patients with chronic disease experience ongoing symptoms and progression and poor quality of life. Despite the attractiveness of restoring normal tissue architecture as a means to treat chronic disease, pharmacological interventions to achieve this have not been studied, and as a result practical applications of this approach do not yet exist. One potential approach to achieve pharmacological tissue repair is through changes in mitochondrial function. However, the literature currently teaches that eliciting changes in mitochondrial function suppresses repair and promotes inflammation (Cai *et al,* 2022), and indeed, alterations in mitochondrial function to alter disease progression through control of inflammation, or tissue remodelling, have not been extensively studied. The mitochondrial Complex I modulator (MCIM) compounds used disclosed herein, and disclosed in PCT/EP2024/057086, modulate the activity of mitochondrial Complex I in a manner that differs from that of conventional complex I inhibitors. This elicits an adaptive phase which directs cell fate choices and mimics a wound repair-like microenvironment *in vivo.* Phenotypically, this can control inflammation, alter the activation response of resident cells and desensitizes the microenvironment to the effects of a pro-inflammatory cell infiltrate, and concurrently initiate repair signals in affected tissues, such as the lung and joint. In addition, the MCIM compounds can stimulate the production of key growth factors such as VEGF, and collagen I, and the basement collagen IV. Together, these mechanisms support a reduction in inflammation and restoration of tissue architecture in multiple organ/tissue settings. Therefore, treatment with MCIM compounds are the first compounds to demonstrate reversal of existing pathological damage with a concomitant increase in patient quality of life, representing the first curative treatment for arthritis and other diseases and disorders associated with physical functional decline and physical functional decline associated with aging.

In addition to the effects of MCIM compounds described in PCT/EP2024/057086, the inventors have now found that treatment of RA patients with MCIM compounds can inhibit or reverse structural progression in the joints, in particular with respect to bone erosion and cartilage loss and/or reduce osteitis (inflammation of the bone). Treatment with MCIM compounds can also lead to a reduction in inflammatory biomarkers, including C-reactive protein. Furthermore, treatment with MCIM compound has been found to improve patients' ability to perform activities of daily living (ADLs), and in particular to improve disability, which may be the result of tissue repair induced by MCIM compound. In addition, notable improvements in patient fatigue, and overall quality of life (QoL) are observed in patients treated with MCIM compound.

At its broadest, the invention provides MCIM compounds for use in treating arthritic diseases and/or treating or arresting progression of physical functional decline and/or treating or arresting progression of a physical disability. In some embodiments, the MCIM compounds may prevent or reduce the structural progression of arthritic diseases and/or physical functional decline and/or physical disability. In some embodiments, the use of MCIM compounds in the treatment of disease may improve disability measures, such as quality-of-life (QoL) measures.

In a first aspect, the invention provides an MCIM compound for use in the treatment of arthritis in a patient. In a related second aspect, the invention provides a method of treating arthritis in a patient, the method comprising administering an MCIM compound to the patient. In a further related third aspect, the invention provides an MCIM compound for use in the preparation of a medicament for use in the treatment of arthritis.

In fourth aspect, the invention provides an MCIM compound for use in the treatment of physical functional decline in a patient and/or treatment of a functional disability in a patient. In a related fifth aspect, the invention provides a method of treating physical functional decline in a patient and/or treating a functional disability in a patient, the method comprising administering an MCIM compound to the patient. In a further related sixth aspect, the invention provides an MCIM compound for use in the preparation of a medicament for use in the treatment of physical functional decline and/or treatment of a functional disability in a patient.

In some embodiments of the above aspects, the treatment may i) increase mobility in one or more joints of the patient, ii) inhibit the loss of mobility in one or more joints of the patient, iii) reduce patient fatigue, iv) reduce pain intensity experienced by the patients, and/or v) improve a patients' quality of life (QoL). Quality of life (QoL) relates to a patient's sense of well-being and ability to carry out activities of daily living and can be measured using various well-established clinical scoring methods/patient reported outcome measures. In some embodiments, clinical parameters i)-v) may be measured using any suitable clinical scoring method known in the art. Non-limiting examples for measuring clinical parameters i)-v) include the following clinical scoring methods: HAQ-disability index, EULAR-RAID, FACIT-F and EQ-5D-5L. In some embodiments, QoL may be assessed using or more of the clinical scoring systems selected from HAQ-disability index, EULAR-RAID, FACIT-F and EQ-5D-5L. In some embodiments, joint mobility is assessed using RAMRIS and/or EQ-5D-5L. In some embodiments, patient fatigue is measured using FACIT-F.

In some embodiments of the first to third aspects, the arthritis may have previously been treated using one or more DMARDs. For example, the arthritis may have been treated with one or more DMARDs selected from: csDMARDs, tsDMARDs and bDMARDs. In some embodiments, the arthritis may have demonstrated an inadequate response to previous treatment with a DMARD. Thus, in some embodiments, the arthritis may be classified as a DMARD-inadequate response (IR) arthritis. In some embodiments, the arthritis may be a csDMARD-IR arthritis, a bDMARD-IR arthritis, and/or a tsDMARD-IR arthritis. An arthritis may demonstrate an inadequate response to one or more classes of DMARD. Thus, in some embodiments, the arthritis may have demonstrated an inadequate response to multiple classes of DMARD selected from csDMARD, tsDMARD and bDMARD. In some embodiments, the arthritis may be methotrexate-IR arthritis. In some embodiments, the arthritis may have demonstrated an inadequate response to one or more drugs selected from the following: methotrexate, azathioprine, chloroquine, hydroxychloroquine, ciclosporin, D-penicillamine, gold salt, sodium aurothiomalate, auranofin, leflunomide, sulfasalazine, minocycline, adalimumab, certolizumab, etanercept, golimumab, infliximab, abatacept, anakinra, rituximab, sarilumab, tocilizumab, secukinumab, ixekizumab, bimekizumab, ustekinumab, risankizumab, guselkumab, belimumab, upadacitinib, baricitinib, filgotinib, tofacitinib, deucravacitinib, and apremilast.

In some embodiments of the first to third aspects, the arthritis may have previously responded to treatment with one or more DMARDs before demonstrating an inadequate continued response to said treatment. Thus, in some embodiments, the arthritis may be refractory to treatment with one or more DMARDs. In some embodiments, the arthritis may be refractory to treatment with one or more DMARDs selected from: csDMARDs, tsDMARDs and bDMARDs. In some embodiments, the arthritis is refractory to treatment with one or more drugs selected from the following list: methotrexate, azathioprine, chloroquine, hydroxychloroquine, ciclosporin, D-penicillamine, gold salt, sodium aurothiomalate, auranofin, leflunomide, sulfasalazine, minocycline, adalimumab, certolizumab, etanercept, golimumab, infliximab, abatacept, anakinra, rituximab, sarilumab, tocilizumab, secukinumab, ixekizumab, bimekizumab, ustekinumab, risankizumab, guselkumab, belimumab, upadacitinib, baricitinib, filgotinib, tofacitinib, deucravacitinib, and apremilast. In some embodiments, the arthritis is refractory to treatment with methotrexate.

Patients suffering from arthritis, for example RA, may show signs of active/progression disease after treatment with a csDMARD, for example methotrexate, and ≥2 b/tsDMARDs (e.g. failure with at least 1 bDMARD and 1 tsDMARD, or failure with at least 2 bDMARDs, or failure with at least 2 tsDMARDs). In such cases, the arthritis is classified as "difficult-to-treat" arthritis. In some embodiments of the first to third aspects, the arthritis may be a difficult-to-treat arthritis, for example the arthritis may be difficult-to-treat RA.

In some embodiments of the first to third aspects, the arthritis may be rheumatoid arthritis (RA), psoriatic arthritis, osteoarthritis, spondyloarthritis, reactive arthritis, infectious arthritis, or juvenile idiopathic arthritis. In some embodiments, the arthritis may be a symptom of another disease or disorder. For example, the arthritis may be caused by systemic lupus erythematosus, osteoporosis, osteopenia, ankylosing spondylitis, gout, scleroderma, or Sjogren's syndrome.

In some embodiments of aspects one to six, the treatment comprises the separate, sequential or simultaneous administration of at least one DMARD. In some embodiments, the treatment comprises the separate, sequential, or simultaneous administration of one or more DMARDs selected from: csDMARDs, tsDMARDs and bDMARDs. In some embodiments, the treatment comprises the separate, sequential or simultaneous administration of one or more DMARDs selected from the following: methotrexate, azathioprine, chloroquine, hydroxychloroquine, ciclosporin, D-penicillamine, gold salt, sodium aurothiomalate, auranofin, leflunomide, sulfasalazine, minocycline, adalimumab, certolizumab, etanercept, golimumab, infliximab, abatacept, anakinra, rituximab, sarilumab, tocilizumab, secukinumab, ixekizumab, bimekizumab, ustekinumab, risankizumab, guselkumab, belimumab, upadacitinib, baricitinib, filgotinib, tofacitinib, deucravacitinib, and apremilast. In some embodiments, the treatment comprises the separate, sequential or simultaneous administration of methotrexate.

In some embodiments of the above aspects, the MCIM compound is does not cause immunosuppression. In some embodiments, the MCIM compound is administered as an adjunct therapy for use with a primary immunosuppressant therapy. In some embodiments, the primary immunosuppressant therapy comprises a DMARD. For example, the immunosuppressant therapy may comprise one or more DMARDs selected from: csDMARDs, tsDMARDs and bDMARDs. For example, the primary immunosuppressant therapy may comprise one or more DMARDs selected from the following: methotrexate, azathioprine, chloroquine, hydroxychloroquine, ciclosporin, D-penicillamine, gold salt, sodium aurothiomalate, auranofin, leflunomide, sulfasalazine, minocycline, adalimumab, certolizumab, etanercept, golimumab, infliximab, abatacept, anakinra, rituximab, sarilumab, tocilizumab, secukinumab, ixekizumab, bimekizumab, ustekinumab, risankizumab, guselkumab, belimumab, upadacitinib, baricitinib, filgotinib, tofacitinib, deucravacitinib, and apremilast. In some embodiments, the primary immunosuppressant therapy comprises methotrexate. The primary and adjunct therapies may be administered separately, sequentially or simultaneously.

In some embodiments of the first to third aspects, before treatment with the MCIM compound, the arthritis may be moderate to severe arthritis. The severity of arthritis may be measured using any suitable scoring method known in the art. For example, the severity of arthritis may be measured using one or more clinical scoring methods selected from: HAQ-DI, EQ-5D, EQ-5D-3L, EQ-5D-5L, EULAR-RAID, and FACIT-F. In some embodiments, the patient prior to treatment with an MCIM compound may have a HAQ-DI score of 1-2 (moderate disability) or a score of ≥2 (severe disability). In some embodiments, the patient prior to treatment with an MCIM compound may have an EQ-5D, EQ-5D-5L and/or EQ-5D-3L score in one or more dimensions measured of 3 (moderate problems), 4 (severe problems), or 5 (extreme problems). In some embodiments, the patient prior to treatment with an MCIM compound may have a composite EULAR-RAID score of 3-6 (moderate impairment) or a score of ≥7 (severe impairment). In some embodiments, the patient prior to treatment with an MCIM compound may have a FACIT-F score of 20-40 (moderate fatigue) or a score of ≤20 (severe fatigue).

In some embodiments of the fourth to sixth aspects, the patient prior to treatment with an MCIM compound may have a HAQ-DI score of 1-2 (moderate disability) or a score of ≥2 (severe disability). In some embodiments, the patient prior to treatment with an MCIM compound may have an EQ-5D, EQ-5D-5L and/or EQ-5D-3L score in one or more dimensions measured of 3 (moderate problems), 4 (severe problems), or 5 (extreme problems). In some embodiments, the patient prior to treatment with an MCIM compound may have a composite EULAR-RAID score of 3-6 (moderate impairment) or a score of ≥7 (severe impairment). In some embodiments, the patient prior to treatment with an MCIM compound may have a FACIT-F score of 20-40 (moderate fatigue) or a score of ≤20 (severe fatigue).

In some embodiments of the above aspects, following treatment with an MCIM compound a patient may experience an increase in their quality of life. Quality of life of a patient may be measured using any suitable clinical scoring method known in the art. In some embodiments of the above aspects, following treatment with an MCIM compound a patient may have a lower HAQ-DI score compared to their HAQ-DI score before treatment with an MCIM compound. In some embodiments, following treatment with an MCIM compound the patient may achieve the minimal clinically important difference (MCID), for example, the patient HAQ-DI score may decrease by ≥0.22 compared to the patient HAQ-DI score prior to treatment with MCIM compound. In some embodiments, following treatment with an MCIM compound the patient may achieve a critical difference (i.e. the minimum change that can be reliably discriminated from random long-term variations), for example the patient HAQ-DI score may decrease by ≥0.68 compared to the patient HAQ-DI score prior to treatment with MCIM compound. In some embodiments, following treatment with MCIM compound a patient may achieve functional remission. Functional remission may be indicated by a HAQ-DI score of 50.5. In some embodiments, following treatment with an MCIM compound a patient may achieve a population normative score of 50.25.

In some embodiments of the above aspects, following treatment with an MCIM compound a patient may have a higher FACIT-F score compared to their FACIT-F score before treatment with an MCIM compound. In some embodiments, following treatment with an MCIM compound the patient may achieve the FACIT-F MCID, for example, the patient FACIT-F score may increase by ≥4 compared to the patient FACIT-F score prior to treatment with MCIM compound. In some embodiments, following treatment with an MCIM compound the patient may achieve a within-patient meaningful improvement (WPMI), for example the patient FACIT-F score may increase by ≥9.7 compared to the patient HAQ-DI score prior to treatment with MCIM compound. In some embodiments, following treatment with an MCIM compound a patient may achieve a healthy population normative score of ≤43.

In some embodiments of the above aspects, following treatment with an MCIM compound a patient may achieve a lower patient global assessment of disease (PGAD) and/or patient global assessment of health (PGAH) score compared to the patients PGAD and/or PGAH score prior to treatment with MCIM compound. In some embodiments, the patient may achieve the PGAD MCID and/or the PGAH MCID. In some embodiments, the patient may achieve a score decrease of ≥9.1 as measured using the PGAD. In some embodiments, the patient may achieve a score decrease of ≥9.1 as measured using the PGAH. In some embodiments, the patient may achieve a score decrease of ≥9.1 as measured using each of the PGAD and the PGAH.

In some embodiments of the above aspects, following treatment with an MCIM compound a patient may have a lower EULAR-RAID score compared to their EULAR-RAID score before treatment with an MCIM compound. In some embodiments, following treatment with an MCIM compound the patient may achieve a patient acceptable state (PAS) in one or more EULAR domains selected from: pain, physical disability (functional), fatigue, sleep disturbances, coping, physical wellbeing, and emotional well-being. In some embodiments, the EULAR score for one or more domains may decrease by ≥2 points, for example by at least 2, 3, 4, 5, 6, 7, 8, 9 or 10 points compared to the respective domain score prior to treatment with the MCIM compound. In some embodiments, the composite EULAR-RAID score may decrease by ≥2, for example at least 2, 3, 4, 5, 6, 7, 8, 9 or 10 points compared to the composite EULAR-RAID score prior to treatment with an MCIM compound.

In some embodiments of the above aspects, following treatment with an MCIM compound one or more of the EQ-5D-5L descriptive dimensions (mobility, self-care, usual activities, pain/discomfort, and anxiety/depression) may be decreased by 1, 2, 3, or 4 points compared to the descriptive dimension score prior to treatment with and MCIM compound. In some embodiments, following treatment with an MCIM compound the EQ-5D-5L visual analogue scale (VAS) score may be increased following MCIM treatment compared the VAS score prior to MCIM treatment. In some embodiments, following treatment with an MCIM compound the subject may have an increase in EQ-VAS of ≥5 points or ≥7.5.

Any suitable clinical scoring method known in the art, or any combination of scoring methods, may be used to determine the effectiveness of treatment with an MCIM compound according to any one of the above aspects. In some embodiments, one or more clinical scoring methods may be used to assess efficacy of treatment with an MCIM compound. In some embodiments, one or more clinical scoring methods may be used to assess efficacy of treatment with an MCIM compound selected from the following list: ACR/EULAR 2010 scoring criteria (ACR/EULAR score), "disease activity score at 28 joints" criteria (DAS28 score), DAS28-CRP (Das28 calculated with CRP), "health assessment questionnaire disability index" (HAQ-DI score), "clinical disease activity index" (CDAI score), "simplified disease activity index" (SDAI score), "American College of Rheumatology response criteria" (also known as "ACR20/50/70 response"; ACR20/50/70 score), European league against rheumatism response criteria (EULAR score), "Modified total Sharp/van der Heijde score" (mTSS score), "routine assessment of patient index data 3 score" (RAPID3 score), Pain Visual Analogue Scale (Pain VAS), EULAR-RA Impact of Disease (EULAR-RAID), patient global assessment of health (PGAH), Patient global assessment of disease (PGAD), EQ-5D, EQ-5D-3L, EQ-5D-5L, EQ-5D VAS, EQ-5D-Y-3L, ATHLOS scale, SAGE scale, the disability index, and the SF-36 survey, Barthel Index (BI), Groningen Activity Restriction Scale (GARS), Lawton and Brody Instrumental Activities of Daily Living Scale (LB-IADL) and the elderly mobility scale.

In some embodiments of the above aspects, treatment with the MCIM compound inhibits or reverses structural progression of arthritis, inhibits or reverses bone erosion, inhibits or reverses cartilage loss, reduces osteitis, reduces rheumatoid factor in patient blood, and/or reduces C-reactive protein in patient blood. Structural progression of arthritis, including bone erosion, cartilage loss and osteitis may be measured using OMERACT RAMRIS (RAMRIS). In some embodiments, RAMRIS may be used to assess joints of one or more of the distal radius, distal ulna, carpal bones, metacarpal bases, metacarpal heads, and phalangeal bases. In some embodiments, following treatment with an MCIM compound a patient may have a RAMRIS bone erosion score of 0. In some embodiments, following treatment with an MCIM compound a patient may have a RAMRIS bone erosion score of 0. In some embodiments, following treatment with an MCIM compound a patient may have a RAMRIS bone erosion score of less than 0. In some embodiments, following treatment with an MCIM compound a patient RAMRIS osteitis/bone marrow edema score may be decreased compared to osteitis score prior to treatment. In some embodiments, following treatment with an MCIM compound a patient RAMRIS osteitis score may decrease from 3 to 2, 1 or 0, or decrease from 2 to 1 or 0, or decrease from 1 to 0. In some embodiments, following treatment with an MCIM compound the joint space narrowing score for one or more joints of a patient may decrease from 4 to 3, 2, 1, or 0, or from 3 to 2, 1, or 0, or from 2 to 1 or 0, or from 1 to 0. In some embodiments, following treatment with an MCIM compound the serum concentration of CRP in a subject may be decrease by ≥20% compared to the serum CRP concentration prior to treatment with an MICIM compound. In some embodiments, the serum CRP concentration of a subject may be decreased by at least 20%, at least 30%, at least 40%, at least 50%, at least 60%, at least 70%, at least 80%, at least 90% after treatment with an MCIM compound the serum concentration prior to treatment with an MCIM compound.

In some embodiments according to the fourth to sixth aspect above, the physical functional decline comprises musculoskeletal decline and/or the functional disability comprises a musculoskeletal disability. In some embodiments, the physical functional decline comprises degradation or damage of one or more bones and/or degradation or damage to one or more joints. In some embodiments, the functional disability comprises degradation or damage of one or more bones and/or degradation or damage to one or more joints. In some embodiments, the physical functional decline and/or the functional disability is the result of one or more of; RA, osteoarthritis; osteoporosis; osteopenia, inflammatory bowel disease, sarcopenia, cachexia relating to cancer or fibrosis, idiopathic pulmonary fibrosis and heart failure.

In some embodiments according to the fourth to sixth aspect, the physical function decline is age-related physical functional decline and/or the functional disability is an age-related functional disability. In some embodiments, the age-related functional decline/age-related functional disability is associated with one or more diseases selected from the following list: RA, osteoarthritis; osteoporosis; osteopenia, inflammatory bowel disease, sarcopenia, cachexia relating to cancer or fibrosis, idiopathic pulmonary fibrosis and heart failure. In some embodiments, the MCIM compounds may improve fatigue symptoms associated with arthritis, RA, osteoarthritis, osteoporosis; osteopenia, inflammatory bowel disease, sarcopenia, cachexia relating to cancer or fibrosis, idiopathic pulmonary fibrosis and heart failure. In some embodiments, the functional disability is associated with a fatigue disorder. In some embodiments, the fatigue disorder is one or more diseases selected from: myalgic encephalopathy/chronic fatigue syndrome (ME/VFS), post-viral fatigue syndrome, long-COVID and fibromyalgia.

In the embodiments of the above aspects, the MCIM compound is a compound, or a pharmaceutically acceptable salt, hydrate, or solvate thereof, of the following formula: 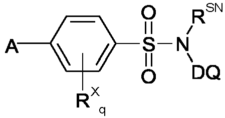 wherein:
-A is independently: 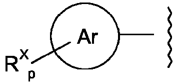
-Ar is independently phenyl, pyridinyl, or pyrimidinyl; and
p is independently an integer from 0 to 3;
and wherein:
q is independently an integer from 0 to 3;
and wherein:
-R^{SN} is independently -H or saturated aliphatic C₁₋₄alkyl;
and wherein:
-DQ is independently -D¹-Q¹ or -D²=O;
-D¹- is independently cyclopentane-di-yl, cyclohexane-di-yl, cycloheptane-di-yl, bicyclo[3.1.1]heptane-di-yl, or bicyclo[3.2.1]octane-di-yl, and is optionally substituted with one or more groups -R^{D};
-D²= is independently cyclopentane-yl-ylidene, cyclohexane-yl-ylidene, cycloheptane-yl-ylidene, bicyclo[3.1.1]heptane-yl-ylidene, or bicyclo[3.2.1]octane-yl-ylidene, and is optionally substituted with one or more groups -R^{D};
each -R^{D} is independently selected
   from -F, -Cl, -Br, -I, -R^{DD}, -CF₃, -OH, -OR^{DD}, -NH₂, -NHR^{DD}, and -NR^{DD}₂; and
each -R^{DD} is independently saturated aliphatic C₁₋₄alkyl;
and wherein -Q¹ is independently selected from: 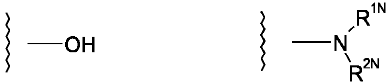 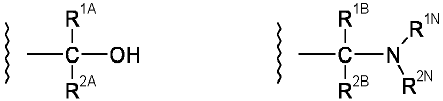 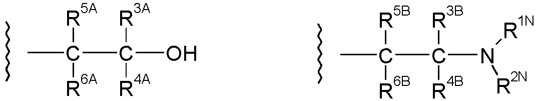 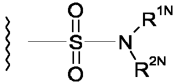 wherein:
each -R^{1N} is independently -H, -R^{CN}, or -R^{CF};
each -R^{2N} is independently -H, -R^{CN}, or -R^{CF};
each -R^{CN} is independently saturated aliphatic C₁₋₄alkyl; and
each -R^{CF}, if present, is independently saturated aliphatic C₁₋₄fluoroalkyl;
   or:
   -NR^{1N}R^{2N} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
   -R^{1A} is independently -H, -R^{C}, or -R^{F}; and
   -R^{2A} is independently -H, -R^{C}, or -R^{F};
   or -R^{1A} and -R^{2A} together form a saturated aliphatic C₂₋₄alkylene group;
   -R^{1B} is independently -H, -R^{C}, or -R^{F}; and
   -R^{2B} is independently -H, -R^{C}, or -R^{F};
   or -R^{1B} and -R^{2B} together form a saturated aliphatic C₂₋₄alkylene group;
   or -R^{1B} and -R^{2B} together form =O;
   -R^{3A} is independently -H, -R^{C}, or -R^{F}; and
   -R^{4A} is independently -H, -R^{C}, or -R^{F};
   or -R^{3A} and -R^{4A} together form a saturated aliphatic C₂₋₄alkylene group;
   -R^{5A} is independently -H, -R^{C}, -R^{F}, or -R^{J}; and
   -R^{6A} is independently -H, -R^{C}, or -R^{F};
   or -R^{5A} and -R^{6A} together form a saturated aliphatic C₂₋₄alkylene group;
   -R^{3B} is independently -H, -R^{C}, or -R^{F}; and
   -R^{4B} is independently -H, -R^{C}, or -R^{F};
   or -R^{3B} and -R^{4B} together form a saturated aliphatic C₂₋₄alkylene group;
   -R^{5B} is independently -H, -R^{C}, -R^{F}, -OH, or -OR^{O}; and
   -R^{6B} is independently -H, -R^{C}, or -R^{F};
   or -R^{5B} and -R^{6B} together form a saturated aliphatic C₂₋₄alkylene group;
   each -R^{C} is independently saturated aliphatic C₁₋₄alkyl;
   each -R^{F} is independently saturated aliphatic C₁₋₄fluoroalkyl;
   -R^{O} is independently saturated aliphatic C₁₋₄alkyl;
   -R^{J} is independently -NHz, -NHR^{JN1}, -NR^{JN1}2, or -NR^{JN2}R^{JN3};
   each -R^{JN1} is independently saturated aliphatic C₁₋₄alkyl; and
   -NR^{JN2}R^{JN3} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
and wherein each -R^{X} is independently:

-F, -Cl, -Br, -I,

-R^{XX},

-OH, -OR^{XX},

-SH, -SR^{XX},

-CF₃, -OCF₃, -SCF₃,

-NH₂, -NHR^{XX}, -NR^{XX}₂, -NR^{YY}R^{ZZ},

-C(=O)R^{XX}, -OC(=O)R^{XX},

-C(=O)OH, -C(=O)OR^{XX},

-C(=O)NH₂, -C(=O)NHR^{XX}, -C(=O)NR^{XX}₂, -C(=O)NR^{YY}R^{ZZ},

-OC(=O)NH₂, -OC(=O)NHR^{XX}, -OC(=O)NR^{XX}₂, -OC(=O)NR^{YY}R^{ZZ},

-NHC(=O)R^{XX}, -NR^{XX}C(=O)R^{XX},

-NHC(=O)OR^{XX}, -NR^{XX}C(=O)OR^{XX},

-NHC(=O)NH₂, -NHC(=O)NHR^{XX}, -NHC(=O)NR^{XX}₂, -NHC(=O)NR^{YY}R^{ZZ},

-NR^{XX}C(=O)NH₂, -NR^{XX}C(=O)NHR^{XX}, -NR^{XX}C(=O)NR^{XX}₂, -NR^{XX}C(=O)NR^{YY}R^{ZZ},

-CN,

-NO₂,

-S(=O)₂NH₂, -S(=O)₂NHR^{XX}, -S(=O)₂NR^{XX}₂, -S(=O)₂NR^{YY}R^{ZZ},

-S(=O)R^{XX}, -S(=O)₂R^{XX}, -OS(=O)₂R^{XX}, -S(=O)₂OH, or -S(=O)₂OR^{XX};

wherein:
each -R^{XX} is independently saturated aliphatic C₁₋₆alkyl, phenyl, or benzyl, wherein said phenyl and benzyl are optionally substituted with one or more groups selected from: -F, -Cl, -Br, -I, -CF₃, -OCF₃, -R^{XXX}, -OH, -OR^{XXX}, or -SR^{XXX}, wherein each -R^{XXX} is independently saturated aliphatic C₁₋₄alkyl; and
each -NR^{YY}R^{ZZ} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl.

In some embodiments:
-A is: 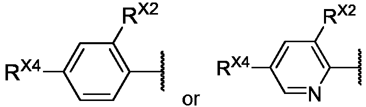
   q is independently 0 or 1;
   -R^{SN} is -H;
-DQ is: 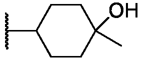
   -R^{X} is independently -F or -Cl;
   -R^{X2} is independently -F, -Cl, -CN, or -CF₃;
   -R^{X4} is independently -F, -Cl, or -CN.

In some embodiments, the MCIM compound is selected from compounds of the following formulae: 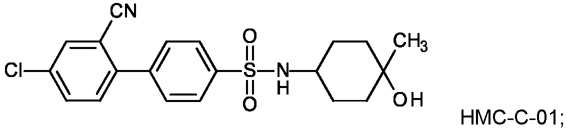 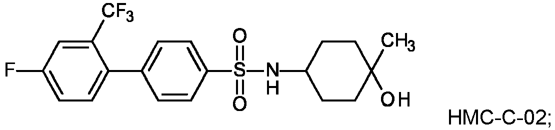 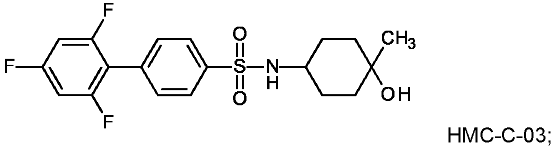 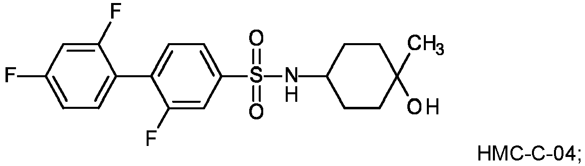 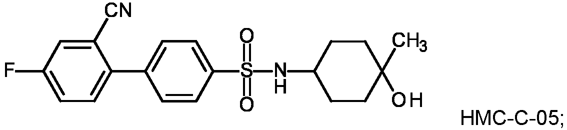 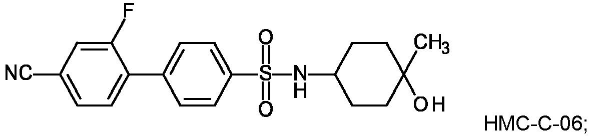 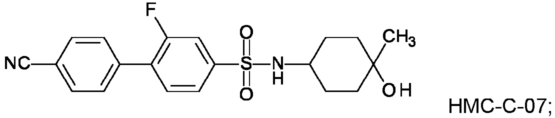 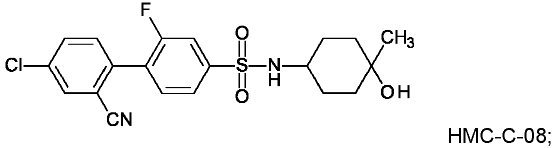 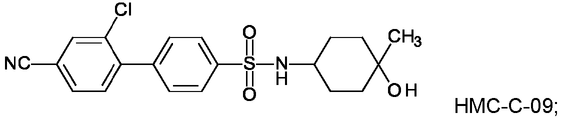 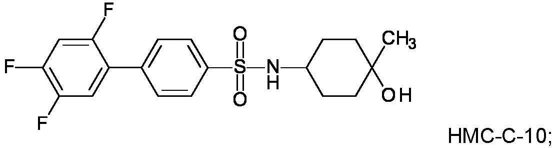 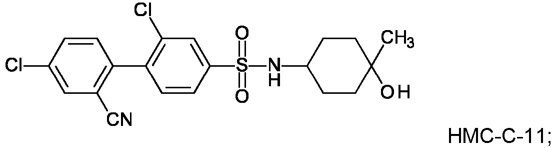 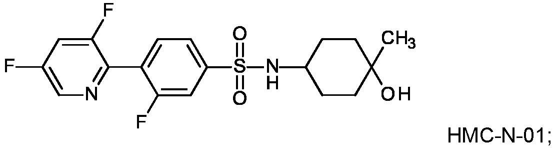 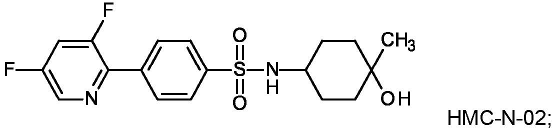 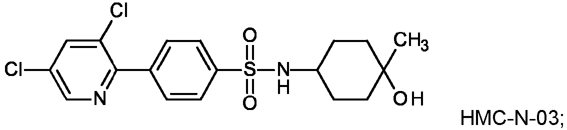 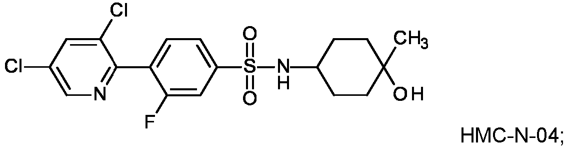 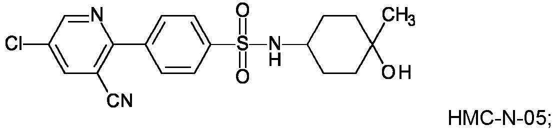 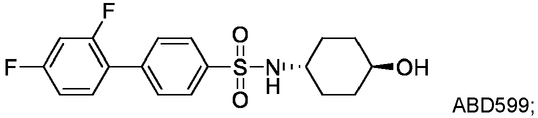 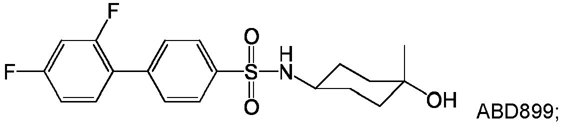 and 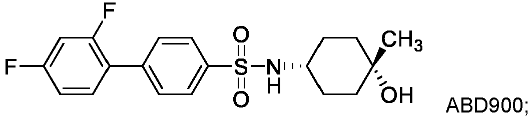 or a pharmaceutically acceptable salt, hydrate, or solvate thereof.

In some embodiments, the MCIM compound is selected from compounds of the following formulae: 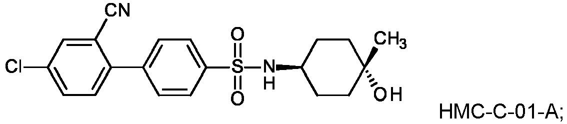 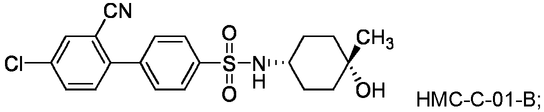 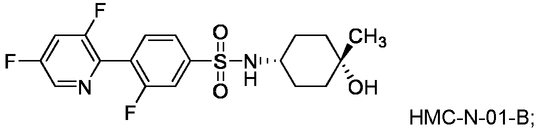 and 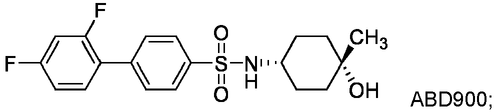 or a pharmaceutically acceptable salt, hydrate, or solvate thereof.

In some embodiments, the MCIM compound is a compound of the following formula, or a pharmaceutically acceptable salt, hydrate, or solvate thereof:

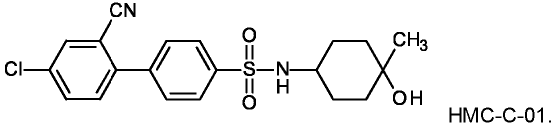

In some embodiments, the MCIM compound is a compound of the following formula, or a pharmaceutically acceptable salt, hydrate, or solvate thereof:

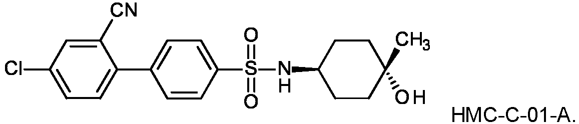

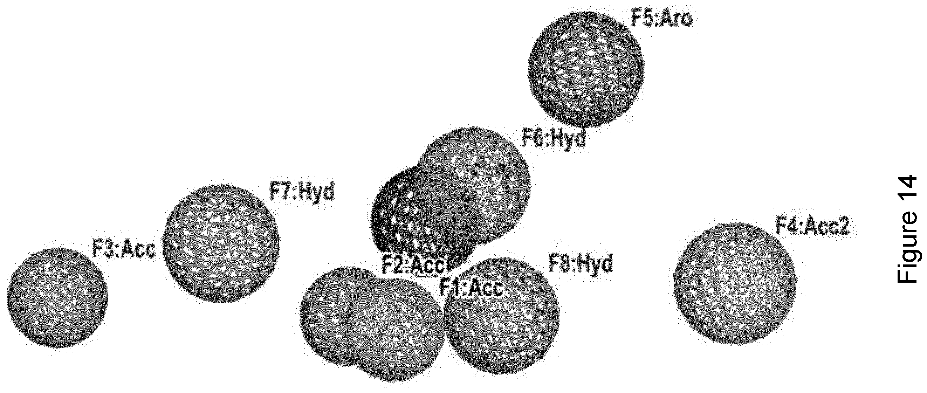

In some embodiments, the MCIM compound comprises four or more of the pharmacophore features of the pharmacophore model represented in Figure 14. The three-dimensional arrangement of the pharmacophore features may be as described in Tables 12-A, 12-B and 12-C.

In some embodiments, the MCIM compound may be administered at a dose of 0.05-1000 mg/day, 0.1-900 mg/day, 0.2-800 mg/day, 0.3-700 mg/day, 0.4-600 mg/day, 0.5-500 mg/day, 1-250 mg/day, 2-100 mg/day, 3 to 75 mg/day, 4-50 mg/day, or 5-40 mg/day. In some embodiments, the MCIM compound is administered at a dose of 5, 20 or 40 mg/day.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1****.** Areas assessed for bone erosion and joint space narrowing (left hand), osteitis (middle hand) and synovitis and tenosynovitis (right hand) of wrist and metacarpophalangeal joints used in the OMERACT MRI scoring system. Reproduced from Ostergaard, 2017. IC-CM: intercarpal-carpometacarpal joints; RC: radiocarpal joint; RU: distal radioulnar joint; I-VI and 1-3: extensor respectively flexor tendon compartments of the wrist; MCP: metacarpophalangeal joints; PIP: proximal interphalangeal joints.
**Figure 2** **.** (A) Difference in erosion score, (B) osteitis score, (C) cartilage loss, and (D) synovitis score assessed using MRI in patients treated with either placebo, 5 mg HMC-C-01-A, 20 mg HMC-C-01-A, or 40 mg HMC-C-01-A. MRI was performed prior to treatment and repeated at 12 weeks. Data presented is Least Squares Means (LSM) from baseline at week 12. * p<0.05, ANCOVA.
**Figure 3****.** (A) Change in HAQ-DI score in patients treated with 5 mg and 40 mg HMC-C-01-A, and (B) percentage of patients treated with either placebo, 5 mg HMC-C-01-A, 20 mg HMC-C-01-A, or 40 mg HMC-C-01-A achieving threshold at week 12 of treatment. * p<0.05, logistic regression model for critical change, Chi² test for remission- missing data assumed non-responder.

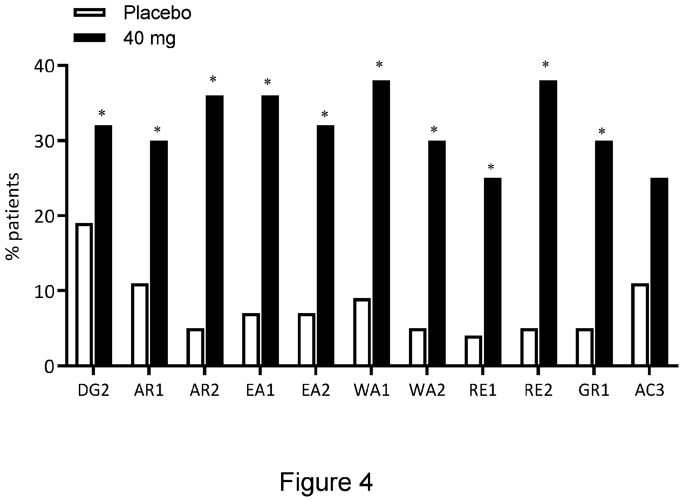

**Figure 4****.** Percentage of patients reporting "no problems" in response to questions relating to individual disability components of the HAQ-DI following 12 weeks of treatment with either placebo or 40 mg HMC-C-01-A. DG2 'Are you able to shampoo your hair?', AR1 'Are you able to stand up from a straight chair?', AR2 'Are you able to get in and out of bed?', EA1 'Are you able to cut your meat?', EA2 'Are you able to lift a full cup or glass to your mouth?', WA1 'Are you able to walk outdoors on flat ground?', WA2 'Are you able to climb up five steps?', RE1 'Are you able to reach and get down a 5 pound object (such as a bag of sugar) from just above your head?', RE2 'Are you able to bend down to pick up clothing from the floor?', GR1 'Are you able to open car doors?', AC3 'Are you able to do chores such as vacuuming or yardwork?'. * p<0.05, Chi² test, full analysis set- missing data assumed non-responder.
**Figure 5****.** Percentage of patients reporting no pain related to functions on the HAQ-DI after 12 weeks of treatment with placebo or 40 mg HMC-C-01-A. * p<0.05, Chi² test, full analysis set- missing data assumed non-responder.
**Figure 6****.** Percentage of patients achieving within-patient meaningful improvement (WPMI) assessed using FACIT reporting method, following 12 weeks of treatment with either placebo, 5 mg HMC-C-01-A, 20 mg HMC-C-01-A, or 40 mg HMC-C-01-A. * p<0.05, logistic regression model, modified full analysis set for FACIT WPMI.
**Figure 7****.** Percentage of patients achieving a score of <2 for fatigue components on the FACIT-F scale following 12 weeks of treatment with placebo or 40 mg HMC-C-01-A. * p<0.05 Chi² test, full analysis set- missing data assumed non-responder.
**Figure 8** **.** (A) percentage of patients achieving patient acceptable state (PAS; PAS <2) in components of the EULAR-RAID patient-reported outcome measure following 12 weeks of treatment with either placebo or HMC-C-01-A. (B) Percentage of patients reporting no problems (score = 1) with individual health-related quality of life domains of the EQ-5D instrument following 12 weeks of treatment with either placebo or HMC-C-01-A. * p<0.05, Chi² test, full analysis set- missing data assumed non-responder.

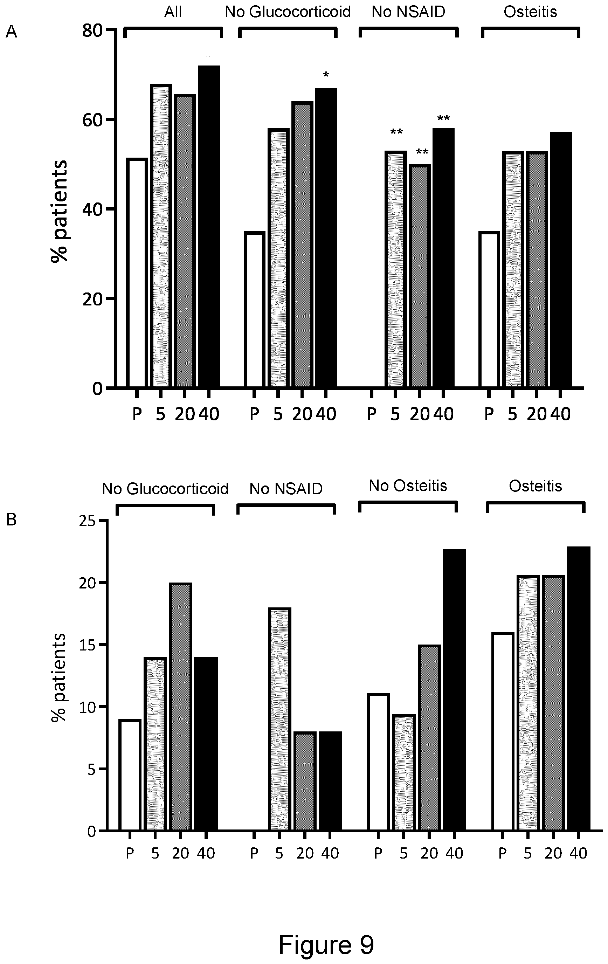

**Figure 9** **.** (A) Percentage of patients showing no progression of erosion following treatment for 12 weeks with placebo ("P"), 5 mg HMC-C-01-A, 20 mg HMC-C-01-A, or 40 mg HMC-C-01-A. "All" show all patients receiving treatment with either placebo or HMC-C-01-A, "No glucocorticoid" shows a subgroup of patients receiving treatment with placebo or HMC-C-01-A without co-treatment with steroids, "No NSAID" shows a subgroup of patients receiving treatment with placebo or HMC-C-01-A without co-administration of non-steriodal anti-inflammatory drugs (NSAIDs), "Osteitis" shows a subgroup of patients receiving treatment with placebo or HMC-C-01-A who showed evidence of subclinical inflammation of the bone (osteitis) at baseline. * p<0.05, logistic regression model, modified full analysis set for all, no steroid, no NSAID and osteitis subgroups; * p<0.05, ** p<0.01, Chi² test, full analysis set- missing data assumed non-responder. (B) Percentage of patients receiving no steroids who demonstrate repair/reversal of erosion following 12 weeks of treatment with placebo or HMC-C-01-A, "No osteitis" shows a subgroup of patients receiving treatment with placebo or HMC-C-01-A who showed no evidence of subclinical inflammation of the bone (osteitis) at baseline.
**Figure 10****.** Homology model of the complete Complex I constructed from publicly available structures for 5 different organisms. The putative targets were resolved in all 5 structures.
**Figure 11****.** *In silico* homology model of NDUFS2. Druggability assessment was performed using SiteMap and identified two binding pockets in the Complex I subunit NDUFS2 (spheres).
**Figure 12****.** *In silico* SiteFinder model of NDUFS2 when in Complex I. A narrow channel was identified for Q10 and drug-like compound binding in the Q-tunnel. Spheres are used to illustrate the space/channels around NDUFS2 when in the Complex I structure.
**Figure 13****.** *In silico* model of a MCIM compound of the invention docked in the Q-site of Complex I. This model reveals interactions between a Complex I inhibitor of the invention and NDUFS2 and additional interactions with the neighbouring Complex I subunit NDUFS7.
**Figure 14****.** 3D representation of a pharmacophore model built from *in silico* modelling of drug docking in Complex I.
**Figure 15****.** Overlay of a MCIM compound of the invention on the ligand-protein pharmacophore model illustrating successful docking of the MCIM compound of the invention in the Q-tunnel of Complex I.
**Figure 16****.** Overlay of a MCIM compound of the invention, CHMSA-02-A, on the ligand-protein pharmacophore model (top panel) and the chemical structure of CHMSA-02-A (bottom panel). This illustrates successful docking of CHMSA-02-A in the Q-tunnel of Complex I.
**Figure 17****.** Graph showing the quantitative structure-activity relationship (QSAR) model used to identify further Complex I binders of the invention and to predict their activity in vivo. Predicted pAct of Complex I binders of the invention correlates well with their experimentally validated pAct, with a coefficient of determination (R²) value of 0.8322 demonstrating that this QSAR model can be used to accurately identify Complex I binders of the invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

The present disclosure provides a pharmaceutical composition comprising an MCIM compound as defined herein for use in a method of treating arthritis and/or physical functional decline, for example an age-related musculoskeletal disease or disability. The method of treatment may inhibit or reverse bone erosion, reduce osteitis, reduce cartilage loss, reduce rheumatoid factor in patient blood and/or reduce C-reactive protein (CRP) in patient blood. Treatment with an MCIM compound can increase mobility in one or more joints of a patient, prevent or inhibit loss of mobility in one or more joints of a patient, increase patient mobility, prevent or inhibit loss of patient mobility, reduce patient fatigue, and/or improve patient quality of life (QoL). It will be understood that where the term "patient mobility" is used hereon in, this term refers to a global assessment of joint mobility in a patient. Thus, an increase in patient mobility indicates an overall improvement in joint mobility.

Also provided is a method of treating arthritis and/or physical functional decline, for example an age-related musculoskeletal disease or disability, the method comprising administering an MCIM compound as disclosed herein to a patient. Also provided is the use of an MCIM compound in the manufacture of a medicament for use in a method of treating arthritis and/or physical functional decline, for example an age-related musculoskeletal disease or disability.

### Arthritides

The MCIM compounds disclosed herein may be used to treat athritides caused by a broad range of aetiologies. For example, the arthritis to be treated may be, or be a symptom of, rheumatoid arthritis (RA), psoriatic arthritis, osteoarthritis, osteoporosis, osteopenia, ankylosing spondylitis, spondylarthritis, reactive arthritis, infectious arthritis, systemic lupus erythematosus, juvenile idiopathic arthritis, gout, scleroderma, or Sjogren's syndrome. MCIM compounds as described herein may be used to treat arthritis, including but not limited to any of the foregoing disease, disorders or syndromes and may increase patient mobility, inhibit loss of patient mobility, reduce fatigue, reduce pain intensity, and/or improve patient quality of life. Treatment with MCIM compound may inhibit or reverse structural progression of disease, inhibit bone and/or cartilage erosion, and/or reduce inflammation in bone tissue (osteitis). Structural progression of disease may comprise one or more of cartilage loss, bone erosion, osteitis, and joint space narrowing. Treatment with an MCIM compound may also increase mobility in one or more joints of a patient and/or inhibit loss of mobility in one or more joints of a patient and/or reduce patient fatigue and/or reduce pain intensity experience by a patient, for example pain associated with daily activities, and/or improve the quality of life of a patient. Any suitable clinical scoring methods known in the art may be used to measure one or more of the foregoing, including those discussed hereinbelow.

The MCIM compounds disclosed herein may be used to treat a patient with arthritis in remission. For example, the MCIM compounds disclosed herein may be used as a maintenance therapy to prolong arthritis remission in a patient. It will be understood that patients with arthritis in remission generally refers to a disease state wherein some or several of the symptoms of the arthritis are under control, symptoms are no longer present, or symptoms are reduced to such an extent that a patient's quality of life is not significantly impacted (e.g. as measured by any suitable clinical or health-related quality of life scoring method described herein). Any suitable clinical scoring methods may be used to determine if a patient is in remission, for example any of the scoring methods disclosed herein. Patients in remission may experience less than 15 minutes of stiffness in the morning, have little to no joint pain, little to no joint tenderness, little to no joint swelling, have little to no reporting of impact of disease and/or have low biomarkers for inflammation (e.g. low levels of C-reactive protein in patient serum).

### Functional declined and functional disability

Physical functional decline is one of the most common and serious clinical problems associated with ageing. Within the general population, from the age of 55 onwards a dramatic increase in the rates of disability is observed (Krishnan et al, 2004). However, it will be appreciated that physical functional decline (also referred to herein simply as 'functional decline') can progress at varying rates between subjects, with some subjects demonstrating earlier or later onset of serious functional decline compared to the population average. Functional decline can decrease a subject's ability to perform every-day tasks and lead to a general decrease in patient mobility, dexterity and QoL. Several factors can contribute to functional decline associated with ageing, including wear and tear of joints and osteoporosis. Thus, MCIM compounds as disclosed herein may be useful in targeting functional decline, and in particular age-related functional decline, and improve quality of life of a subject. The MCIM compounds disclosed herein may be useful in treating functional decline, and diseases and disabilities associated with functional decline, including age-related functional decline, and age-related diseases and disabilities associated with functional decline. The MCIM compounds disclosed herein may reduce disability as measured by anyone or more of the clinical scoring/assessment measures described herein. In particular, the present invention may be useful in treating functional decline, disabilities and diseases associated with bone disorders or bone aging. For example, treatment with an MCIM compound may increase mobility in one or more joints of a patient and/or inhibit loss of mobility in one or more joints of a patient and/or reduce patient fatigue and/or reduce pain intensity experience by a patient, for example pain associated with daily activities, and/or improve the quality of life of a patient. Any suitable clinical scoring methods known in the art may be used to measure one or more of the foregoing, including those discussed hereinbelow.

The present invention may be useful in treating osteoporosis, osteopenia, and/or osteoarthritis. Osteoarthritis is a condition characterised by a breakdown of the cartilage at a joint, causing pain, swelling and reduced joint mobility. Osteoarthritis can be the result of, for example, previous joint injury, damage caused by RA or gout, and/or excess strain (e.g. as a result of obesity). Osteoarthritis can result in osteitis, joint pain, joint stiffness, swelling around the joint, and loss of joint mobility. MCIM compounds as described herein may be used to treat osteoarthritis, wherein the treatment increases joint mobility, inhibits loss of joint mobility, reduces fatigue, reduce pain intensity, and/or improves patient quality of life. Treatment with MCIM compound may inhibit bone and/or cartilage erosion, and/or reduce inflammation in bone tissue (osteitis).

The MCIM compounds disclosed herein may also find use in treating age-related diseases and disorders such as inflammatory bowel disease, sarcopenia, cachexia relating to cancer or fibrosis, idiopathic pulmonary fibrosis and heart failure. It will be appreciated however that while these diseases are typically more common in the aging population, they may be present in younger individuals (e.g. under the age of 55) and that the MCIM compounds disclosed herein will also be useful in treating these younger individuals. The MCIM compounds disclosed herein may improve symptoms associated with the aforementioned diseases/disorders, for example, fatigue associated with inflammatory bowel disease, sarcopenia, cachexia relating to cancer or fibrosis, idiopathic pulmonary fibrosis and heart failure.

The MCIM compounds disclosed herein may also find use in treating fatigue disorders. For example, the MCIM compounds may be used to treat myalgic encephalopathy/chronic fatigue syndrome (ME/VFS), post-viral fatigue syndrome, long-COVID and fibromyalgia.

The MCIM compounds may be used in the treatment of patients over the age of 55, 60, 65, 70, 75, 80, 85 or 90. In particular, the MCIM compounds may be used to treat physical functional decline (e.g. age-related function declined) and/or a functional disability (e.g. an age-related functional disability) in a patient aged 55 and above, 60 and above, 65 and above, 70 and above, 75 and above, 80 and above, 85 and above or 90 and above. In further examples, the MCIM compounds may be administered in order to prevent physical functional decline (e.g. age-related function declined) and/or a functional disability (e.g. an age-related functional disability) in patients aged 55 and above, 60 and above, 65 and above, 70 and above, 75 and above, 80 and above, 85 and above or 90 and above.

### Pain

Pain is a debilitating symptom of many diseases and disorders, including arthritis such as RA, and functional decline, for example age-related functional decline, of bones and joints. The MCIM compounds for use in the methods of the present invention may reduce pain associated with daily activities, in patients suffering from a disease, disorder and/or functional decline. Patients may have an arthritis, such as RA, and/or have age-related functional disability. For example, treatment with an MCIM as disclosed herein may reduce the pain associated with dressing, arising, eating, walking, hygiene, reach, grip and/or activities. Pain associated with these functions may be assessed using any known scoring methodology common in the art, for example the pain visual analogue scale, HAQ-DI, EULAR-RAID, and/or EQ-5D-5L scoring systems as described herein below. The reduction in pain associated with daily activities may be accompanied by a restoration of bone and/or cartilage in affected joints.

### Quality of life and clinical scoring methods

Quality of life (QoL) relates to a patient's sense of well-being and ability to carry out activities of daily living. Several methodologies are known in the art and may be used to assess QoL in patients suffering from an arthritis, a musculoskeletal disease or disorder, and age-related disability. Examples of QoL indexes which may be used to measure QoL in patients suffering from arthritis include EQ-5D-5L, HAQ-DI and EULAR-RAID, and FACIT-F each of which is described in more detail below. MCIM compounds disclosed herein, for example HMC-C-01-A, may improve patient QoL as measured by any suitable scoring method or methods.

It will be appreciated that it is not necessary outside of clinical trials for each patient treated with the MCIMs as disclosed herein to undergo or undertake one or more the aforementioned clinical assessments before and/or after treatment. Such an approach may be unduly burdensome on patients and physicians. Assessment of an individual patient's response to treatment is down to the discretion of the treating physician.

### HAQ-DI

The Health Assessment Questionnaire (HAQ) Disability Index (DI) may be carried out as described in Wolfe F et al (1994) and "THE HEALTH ASSESSMENT QUESTIONNAIRE (HAQ) DISABILITY INDEX (DI) OF THE CLINICAL HEALTH ASSESSMENT QUESTIONNAIRE (VERSION 96.4)", both of which is hereby incorporated by reference in their entirety. Briefly, the HAQ comprises 8 sections relating to dressing, arising, eating, walking, hygiene, reach, grip, and activities. Each section comprises 2 or 3 questions, with scoring for each section being given on a scale of 0 (without any difficulty) to 3 (unable to do). The score given to each section is the highest score given for any given question in each section. The 8 scores of the 8 sections are summed and divided by 8 (or if, for example, only 7 sections are completed by a subject divided by 7). A score of ≤1 indicates mild disability, a score of 1-2 indicates moderate disability and a score of ≥2 indicates severe disability (Krishnan et al., 2004). Scores indicative of a minimally important difference, critical important difference, functional remission and population normative are described in Table 1, below. Subjects treated with an MCIM as described herein, for example HMC-C-01-A, may demonstrate a decrease in the HAQ-DI score of ≥0.22, indicating the subject has achieved a MCID. Subjects treated with an MCIM as described herein, for example HMC-C-01-A, may also demonstrate a decrease in the HAQ-DI score of z0.68, indicating the subject has achieved a critical difference (i.e. the minimum change that can be reliably discriminated from random long-term variations). Subjects treated with an MCIM as described herein, for example HMC-C-01-A, may achieve functional remission, indicated by a HAQ-DI score of ≤0.5, and in some cases a subject may achieve a population normative score of ≤0.25.

### EULAR-RAID score

The Rheumatoid Arthritis Impact of Disease (RAID) score is a global composite measure of the impact of RA that takes into account seven "domains" of impact of RA considered of utmost relevance by patients: pain, physical disability (functional), fatigue, sleep disturbances, coping, physical wellbeing and emotional well-being. The EUALR-RAID score may be determined as described in Gossec et al (2011), which is hereby incorporated by reference in its entirety. Briefly, each domain is evaluated by patients using a single 0 (no symptom) to 10 numeric rating scale and has a specific weight used to calculate a single composite RAID score using an online calculator (https://rheumcalc.com/raid/). A score of ≤2 indicates mild impairment, a score of 3-6 indicates moderate impairment and a score of ≥7 indicates severe impairment (Mistry et al., 2020). Following treatment with an MCIM compound as described herein, one or more of the domain scores selected from pain, physical disability (functional), fatigue, sleep disturbances, coping, physical wellbeing and emotional well-being may decrease by ≥2 points, for example by at least 2, 3, 4, 5, 6, 7, 8, 9 or 10 points compared to the respective domain score prior to treatment with an MCIM compound. Or the patient may achieve a score of ≤2, reflecting a patient acceptable state (PAS), as discussed in Dougados et al (2012) which is hereby incorporated in its entirety by reference.

Following treatment with an MCIM compound as described herein, a subject's composite RAID score may decrease by ≥2, for example at least 2, 3, 4, 5, 6, 7, 8, 9 or 10 points compared to the RAID score prior to treatment with an MCIM compound.

### EQ-5D-5L

The EQ-5D-5L is a standardised measure of health status which provides a simple, generic measure of health for clinical and economic appraisal (The EuroQol Group, 1990) and may be carried out as described in the EQ-5D-5L User Guide, version 2 (Oemar and Janssen, 2013) which is hereby incorporated by reference in its entirety. The EQ-5D-5L consists of two components, a descriptive system and a visual analogue scale (EQ VAS). The descriptive component comprises 5 "dimensions" to be measured (mobility, self-care, usual activities, pain/discomfort, anxiety/depression), with each dimension being scored on a 5-level scale (1 = no problem, 2 = slight problems, 3 = moderate problems, 4 = severe problems, 5 = extreme problems). Following treatment with an MCIM compound as disclosed herein, one or more of the EQ-5D-5L dimensions (mobility, self-care, usual activities, pain/discomfort, and anxiety/depression) may be decreased by 1, 2, 3, or 4 points compared to the score prior to treatment with and MCIM compound.

The EQ-VAS component records a subjects self-rated health on a vertical, visual analogue scale with endpoints labelled "the best health you can imagine" (100) and "the worst health you can imagine" (0). EQ-VAS is measured at the time the test is administered (i.e. on the day of clinical visit). Following treatment with an MCIM compound as described herein, a subject may have a higher EQ-VAS score compared to their EQ-VAS score prior to treatment with an MCIM compound. For example, the subject may have an increase in EQ-VAS of ≥5 points or ≥7.5.

### FACIT-F

The "functional assessment of chronic illness therapy - fatigue" (FACIT-F) is a measure that assesses self-reported patient fatigue and the impact said fatigue has on a patient's ability to perform daily activities and functions and can be performed as disclosed in Hewlett et al (2011), which is hereby incorporated by reference in its entirety. Originally developed for assessment of fatigue in cancer and anaemia patients, it has since become a standard test for assessment of fatigue in various diseases and disorders. As fatigue is also common to all rheumatic conditions, and a consistent feature of aging, FACIT-F is a commonly used scoring method for assessing fatigue in patients with various conditions, including age-related fatigue and arthritis such as RA.

Physical fatigue, functional fatigue, emotional fatigue and social consequences of fatigue are measured. 13 measures are taken to evaluate a subject's level of fatigue during their usual daily activities over the past week and reported on a Likert scale from 4 (not at all fatigued) to 0 (very much fatigued). Where measures are taken as negatively stated items, the scores are reversed by subtracting the response from 4. The scores for each of the 13 measures may then be summed to derive an overall score. A subject scoring less than 20 in the FACIT-F measure is considered to have severe fatigue. An increase of 4 points in FACIT-F score is the minimal clinically important difference (MCID). An increase of 9.7 points in FACIT-F score is the within-patient meaningful improvement (WPMI). Following treatment with an MCIM compound as described herein, a subject may have an increased FACIT-F score compared to their FACIT-F score prior to treatment with an MCIM compound. For example, the FACIT-F score may be increased by ≥4 compared to the subjects FACIT-F score prior to treatment, or, for example, the FACIT-F score may be increased by ≥9.7 compared to the subjects FACIT-F score prior to treatment.

### OMERACT RAMRIS

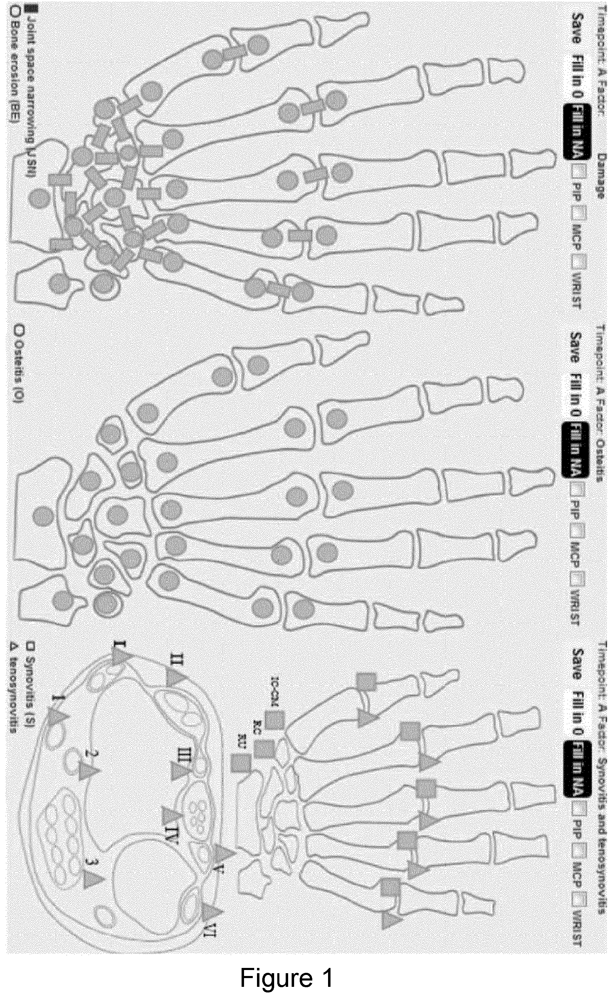

The OMERACT RAMRIS system is a validated, MRI imaging-based scoring system for evaluating bone erosion, bone marrow edema (osteitis), synovitis, and joint space narrowing. The OMERACT RAMRIS system assessment can be performed, and patients scored, as described in Østergaard (2017) and version 2.1 of The OMERACT Handbook (Beaton et al, 2021), each of which are hereby incorporated by reference in their entirety, and as discussed below. Figure 1, reproduced from Østergaard (2017), shows the areas, joint and bone regions assessed in the OMERACT RAMRIS scoring system.

Briefly, bone erosion is scored separately for each joint assessed. Joints assessment may include the distal radius, distal ulna, carpal bones, metacarpal bases, metacarpal heads, and phalangeal bases. Bone erosion is scored on a scale of 0-10, based on the proportion of eroded bone compared to the "assessed bone volume" judged on all available images: 0 = no erosion; 1 = 1-10% of bone eroded; 2 = 11-20%, 3 = 21-30%, 4 = 31-40%, 5= 41-50%, 6 = 51-60%, 7 = 31-70%, 8 = 71-80, 9 = 81-90%, 10 = 91-100%. In case a bone is fused with another bone, bone erosion is scored as 10 in the bone. For long bones, the "assessed bone volume" is from the articular surface (or its best estimated position if absent) to a depth of 1 cm, while in carpal bones it is the whole bone. In subjects treated with the MCIM compounds disclosed herein, for example HMC-C-01-A, subjects may have a RAMRIS erosion score of 0%, indicating no erosion progression. In some subjects, bone erosion progression may be less than 0%, indicating potential erosion repair due to osteogenesis/ossification.

Osteitis/bone marrow edema is scored on a scale of 0-3 based on the proportion of bone with osteitis: 0 = no osteitis; 1 = 1-33% of bone with osteitis; 2 = 34-66%; 3 = 67-100%. Each bone is scored separately as for bone erosion. In patients treated with MCIM compounds as described herein, osteitis score may decrease compared to osteitis score prior to treatment. For example, following treatment with an MCIM osteitis score may decrease from 3 to 2, 1 or 0, or osteitis score may decrease from 2 to 1 or 0, or osteitis score may decrease from 1 to 0.

Synovitis may be assessed in 3 wrist regions (e.g., the distal radioulnar joint; the radiocarpal joint; and the intercarpal and carpometacarpal joints) and in each MCP joint. The degree of synovitis is scored on a scale of 0-3. Score 0 is normal. A score of 1 (mild) is an increase of the presumed maximum volume of the synovial compartment by up to 33%. A score of 2 (moderate) is an increase of the presumed maximum volume of the synovial compartment by 34-66%. A score of 3 (severe) is an increase of the presumed maximum volume of the synovial compartment by 66%.

### Cartilage loss and joint space narrowing (JSN)

Cartilage Loss/joint space narrowing is assessed at 17 locations in the wrist, between distal radius and carpal bones (2 sites), between the carpal bones (except the pisiform; 10 sites), and between carpal bones and each metacarpal bone (5 sites), and in each MCP joint. The degree of narrowing is classified on a scale of 0-4, as follows: 0.0, no cartilage loss or JSN; 0.5, equivocal cartilage loss or JSN; 1.0, minimal (< 10%) but definitive cartilage loss or JSN; 1.5, mild (10% to 25%) cartilage loss or JSN; 2.0, moderate cartilage loss or JSN (26% to 75%, including unilaterally denuded areas but no bilaterally denuded areas or bone-on-bone contact); 2.5, moderate-severe cartilage loss or JSN (> 75%, including focal denuding or focal bone-on-bone contact); 3.0, complete cartilage denuding or diffuse bone-on-bone contact; 3.5, partial ankylosis; 4.0, complete ankylosis. Following treatment with an MCIM compound as described herein, joint space narrowing may be alleviated and/or cartilage loss may be reduced or reversed. For example, following treatment with an MCIM compound, the joint space narrowing score may decrease from 4 to 3, 2, 1, or 0, or the joint space narrowing score may decrease from 3 to 2, 1, or 0, or the joint space narrowing score may decrease from 2 to 1 or 0, or the joint space narrowing score may decrease from 1 to 0.

### C-reactive protein

In addition to, or in place of one or more of the clinical scoring methods which can be used to determine effectiveness of a treatment for RA, biomarkers indicative of arthritis can be used to assess effectiveness of treatment, for example C-reactive protein (CRP). C-reactive protein (CRP) is an acute phase reactant which is increased during systemic inflammation. It is a well-recognised biomarker of inflammation and forms a key component of clinical assessments of arthritis such as the ACR score and the disease activity score (DAS28)-CRP. A concentration of CRP of greater than 0.3 mg/dL is considered to be above the upper limit of normal (Nehring et al., 2023; hereby incorporated in its entirety by reference). Following treatment, a decrease in serum CRP may be observed in subjects treated with an MCIM compound as disclosed herein, for example HMC-C-01-A. For example, following treatment with an MCIM compound the serum concentration of CRP in a subject may be decrease by ≥20% compared to the serum CRP concentration prior to treatment with an MICIM compound. For example, the serum CRP concentration of a subject may be decreased by at least 20%, at least 30%, at least 40%, at least 50%, at least 60%, at least 70%, at least 80%, at least 90% after treatment with an MCIM compound the serum concentration prior to treatment with an MCIM compound.

Any suitable clinical scoring method known in the art, or any combination of scoring methods, may be used to determine the effectiveness of a treatment of arthritis, including those described above. Other clinical scoring methods known in the art include the ACR/EULAR 2010 scoring criteria (ACR/EULAR score), "disease activity score at 28 joints" criteria (DAS28 score), DAS28-CRP (Das28 calculated with CRP), "health assessment questionnaire disability index" (HAQ-DI score), "clinical disease activity index" (CDAI score), "simplified disease activity index" (SDAI score), "American College of Rheumatology response criteria" (also known as "ACR20/50/70 response"; ACR20/50/70 score), European league against rheumatism response criteria (EULAR score), "Modified total Sharp/van der Heijde score" (mTSS score), "routine assessment of patient index data 3 score" (RAPID3 score), Pain Visual Analogue Scale (Pain VAS), EULAR-RA Impact of Disease (EULAR-RAID), patient global assessment of health (PGAH), Patient global assessment of disease (PGAD), EQ-5D, EQ-5D-3L, EQ-5D-5L, EQ-5D VAS, and/or EQ-5D-Y-3L. Other suitable scoring methods include the ATHLOS scale, the SAGE scale, the disability index, and the SF-36 survey (Sanchez-Niubo et al, 2020; Spence et al, 2021; Zhang et al, 2023; Brazier et al, 1992; each of which is hereby incorporated by reference in their entirety). Other questionnaires that assess activities of daily living and instrumental activities of daily living which may be used to assess patient outcomes following treatment with the MCIM compounds disclosed herein are the Barthel Index (BI), Groningen Activity Restriction Scale (GARS), and Lawton and Brody Instrumental Activities of Daily Living Scale (LB-IADL) and the elderly mobility scale (version 2, June 2012).

Inhibition of disease progression may be determined by comparing the results of one or more of ACR/EULAR score, DAS28 score, DAS28-CRP, HAQ-DI score, CDAI score, SDAI score, ACR20/50/70 score, EULAR score, mTSS score, RAPID3 score, Pain VAS, EULAR score, EULAR-RAID, FACIT-F, PGAH, PGAD, EQ-5D, EQ5D-3L, EQ-5D-5L, EQ-5D VAS, and/or EQ-5D-Y-3L score of a subject before, during and/or after receiving treatment with an MCIM compound. Inhibition of disease progression may be indicated by the ACR/EULAR score, DAS28 score, DAS28-CRP, HAQ-DI score, CDAI score, SDAI score, ACR20/50/70 score, EULAR score, mTSS score, RAPID3 score, Pain VAS, EULAR-RAID, FACIT-F, PGAH, PGAD, EQ-5D, EQ5D-3L, EQ-5D-5L, EQ-5D VAS, and/or EQ-5D-Y-3L score being stable (i.e. not changing) over time following treatment.

### Minimal clinically important difference

In the clinical scoring methods described above and elsewhere, the minimal clinically important difference (MCID) is the smallest improvement considered to be worthwhile by a patient. Minimally important clinical differences may also be supplemented by the assessment of patients achieving critical differences (Behrens et al, 2019) or meaningful improvements (Rendas-Baum et al , 2024), as well as assessments of patients showing reductions in severe outcomes, or improvements in their health such that they report minimal, or no, problems, or that they have achieved a patient acceptable state. Table 1 provides a summary of the cutoff values considered significant in the clinical scoring methods described above and in the following examples.

| Table 1 - Values indicative of clinical improvement | | | |
|---|---|---|---|
| Clinical scoring method/clinic al scoring method dimension | Cutoff | Value | Source (each of which are incorporated in their entirety by reference) |
| RAMRIS/RA MRIS erosion | Patients showing no erosion progression | ≤0% | Peterfy et al (2016) |
| CRP responders | Reduction | 20% | American College of Rheumatology response criteria "ACR20" |
| HAQ-DI | Minimal clinically important difference (MCID) | ≥-0.22 | Kitchen et al (2013) |
| | Critical difference | ≥-0.68 | Behrens et al (2019) |
| | Functional remission | ≤0.5 | Strand et al (2019) and Miwa et al (2017) |
| | Population normative | ≤0.25 | Strand et al (2019) and Krishnan et al (2004) |
| PGAD | MCID | -9.1 | Kitchen et al (2013) |
| PGAH | MCID | -9.1 | Kitchen et al (2013) and Nikiphorou (2016) |
| FACIT-F | MCID | ≥4 | Strand et al (2019) and Strand et al (2021) |
| | WPMI | ≥9.7 | Rendas-Baum et al (2024) |
| | Severe | <20 | Fazaa et al (2021) |
| RAID | Patient acceptable state (PAS) | <2 | Mistry et al (2020) |
| RAID/RAID components | Patient acceptable state (PAS) | <2 | Duarte et al (2021) |
| EQ-5D-5L/EQ-5D dimensions | No problems | =1 | EQ-5D-5L User Guide, version 2.0 prepared by Oemar and Jansen (2013) |

### Pharm acophores

MCIM compounds for use in the methods of treatment disclosed herein are not particularly limited and may be any MCIM that conforms to the pharmacophore model described herein.

The term "pharmacophore" is used herein as defined in Wermuth, C.G., Ganellin, C.R., Lindberg, P., Mitscher, L.A.; Glossary of Terms Used in Medicinal Chemistry (IUPAC Recommendations 1998); Pure & Appl. Chem. 70:5 (1998) 1129-1143: A pharmacophore is an ensemble of aromatic steric and electronic features that is necessary to ensure the optimal supramolecular interactions with a specific biological target and to trigger (or block) its biological response. The ensemble of aromatic steric and electronic features represent so-called "pharmacophoric features". Typical pharmacophoric features include, for example, hydrogen bond donor, hydrogen bond acceptor, hydrophobic, aromatic, and positively and negatively ionized areas.

As used herein, the term "pharmacophore model" relates to a pharmacophore hypothesis for the binding interactions in a particular active site. A pharmacophore model is made up of a set of annotation points which are interrelated in 3D space. The annotation points show the location and type of biologically important atoms and groups, i.e., each annotation point relates to a pharmacophore feature of the model. Each annotation point is associated with a radius that describes the permissible variation in 3D space for the location of the given pharmacophoric feature.

As used herein, the phrase "conform to a pharmacophore model" means that a compound described herein binds to the target binding site (i.e., the NDUSF2 and/or NDUSF7 binding site) in a 3D conformation (i.e., "pose") whereby, 4 or more of the annotation points of the pharmacophore model described herein are occupied by corresponding features of the MCIM compound described herein, as determined the unified annotation scheme in Molecular Operating Environment (MOE), 2022.02

Chemical Computing Group ULC, 1010 Sherbooke St. West, Suite #910, Montreal, QC, Canada, H3A 2R7, 2022. In some embodiments, four or more of the annotation points of the pharmacophore model described herein are occupied by corresponding features of the MCIM compound described herein. In some embodiments, five or more, six or more, seven or more, eight or more, or all nine of the annotation points of the pharmacophore model described herein are occupied by corresponding features of the MCIM compound described herein. The conformance of a compound described herein with the pharmacophore features is as determined using the unified annotation scheme in Molecular Operating Environment (MOE), 2022.02 Chemical Computing Group ULC, 1010 Sherbooke St. West, Suite #910, Montreal, QC, Canada, H3A 2R7, 2022.

Annotation points can be broadly divided into three categories: *atom, projected,* and *centroid.* Annotation points are determined for a given compound by the unified annotation scheme in Molecular Operating Environment (MOE), 2022.02 Chemical Computing Group ULC, 1010 Sherbooke St. West, Suite #910, Montreal, QC, Canada, H3A 2R7, 2022.

The annotation points used herein are described below.

### Atom

### Don: H-bond Donor

### Acc: H-bond Acceptor

*Atom* annotations are located directly on an atom of a molecule and typically indicate a function related to protein-ligand binding.

**Don** annotates an H-bond donor heavy atom. A **Don** annotation is added to all oxygen and nitrogen atoms with at least one (possibly implicit) hydrogen attached.

**Acc** annotates an H-bond acceptor heavy atom. O, S, and N elements can be hydrogen bond acceptors provided that they conform to the following rules:
1. Sulfur atoms are not acceptors except that sulfur in S=C groups and anionic sulfurs are acceptors and are given **Acc** annotations.
2. Nitrogen atoms are acceptors and given **Acc** annotations provided that they are not buried. A buried nitrogen is one of {=N=, -N#, >N=, >N<} or a non-3-ring conjugated nitrogen of the form {>N-π, >N-[C+], >N-B, >N-S=O, >N-P=O}.
3. Oxygen atoms are acceptors and are given **Acc** annotations provided that they are not buried and provided that they are not in certain exception groups. A buried oxygen is one of {=O=, -O#; >O=, >O<} or a non-3-ring conjugated oxygen of the form {>O-π, >O-B, >O-[C+]}. Non-buried oxygen atoms are acceptors unless they are part of the following exception groups:

| | |
|---|---|
| 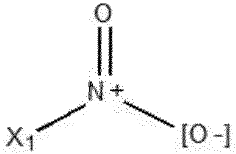 | 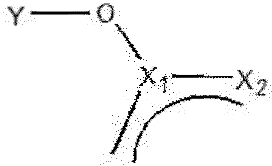 |
| 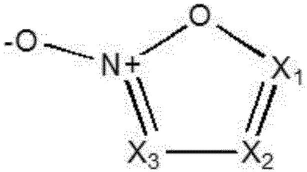 not Acc; Xi = any | 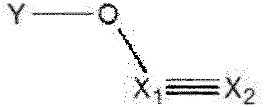 |
| | 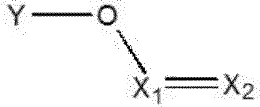 not Acc if any Xi = het; Y ≠ H |
| 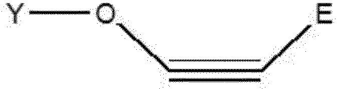 | |
| 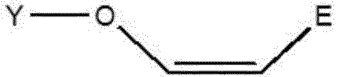 | 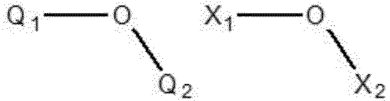 not Acc if both {Q1 ,Q2} are π |
| 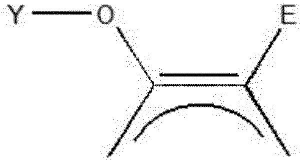 not Acc if Y ≠ H, E = {C#N, N#C, >[N,S,P]=O, Q+, CF₃} | |
| | not Acc if both {X1 ,X2} = het |

### Projected

### Don2: Projected Donor

### Acc2: Projected Acceptor

*Projected* annotations are (typically) located along implicit lone pair or implicit hydrogen directions and are used to annotate the location of possible hydrogen bond or metal ligation partners, or possible R-group atom locations.

Projected **Don2** annotations are added according to the hybridization and the heavy atom coordination of the donor. In the following table the **d** denotes a **Don2** feature.

| **Table 1** | | |
|---|---|---|
| Projected donors | | |
| 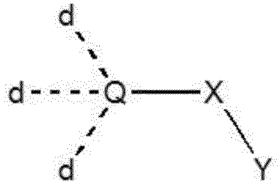 | 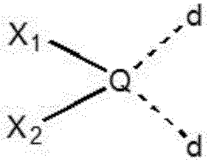 | 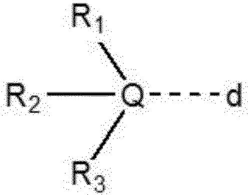 |
| Q = 1° *sp*³, X >1-coord, Y ≠ H | Q = 2° *sp*3 | Q = 3° *sp*³ |
| 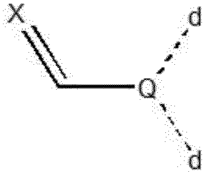 | 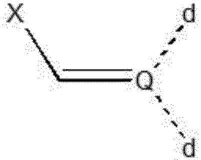 | 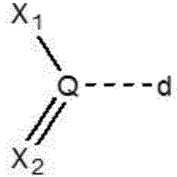 |
| Q = 1 ° *sp²* | Q = 1 °*sp*², X ≠ H | Q = 2° *sp²* |
| 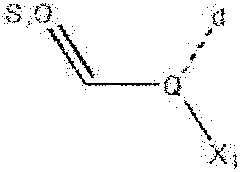 | 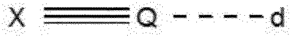 | |
| Q : 2° *sp²* | Q = 1° *sp* | |

Hydrogen bond **Acc2** projected annotations are added to those heavy atoms that qualify as H-bond acceptors (see above) and are given **Acc** annotations. The **Acc2** projected annotations are added in the same locations as those for the **Don2** projected annotations and according to the same rules. (That is, the donors and the acceptors are projected using the same angles and the same distances.) An atom that is both a **Don** and an **Acc** will be annotated with "Don2&Acc2" projected annotations.

Projected annotations such as **Don2,** and **Acc2** are located at potential heavy atom positions. For example, **Don2** indicates a potential hydrogen bond partner heavy atom. Realistically, this partner atom cannot have too much overlap with any of the atoms of the molecule generating the projected annotation. This condition depends on the particular conformation of a molecule and cannot be reliably predicted by topological means. Consequently, a *solvent exposure test* must be applied to validate any hits resulting from a Pharmacophore Search, *i.e*., a test to verify that applicable projected features are not covered by other parts of the conformation (that would prevent the putative projection atom from occupying the intended position).

### Centroid

### Aro Aromatic

### Hyd Hydrophobic

*Centroid* annotations (Aro, Hyd) are located at the geometric center of a subset of the atoms of a molecule.

**Aro** annotation centroids are used for aromatic and pseudo aromatic rings. The **Aro** annotation centroid is placed at the centroid of each aromatic ring (e.g. two centroids in naphthalene).

The definition of aromaticity is generous (a Daylight-style definition) in which each ring is treated in isolation and a Hückel 4n+2 rule is applied. C=O carbons count 0 electrons, otherwise C=R count as 1; N=X nitrogens count 1 and >N- nitrogens and -O- oxygens count 2 electrons. Thus, the following are treated as (pseudo) aromatic and are given an **Aro** annotation centroid in the center of the ring:

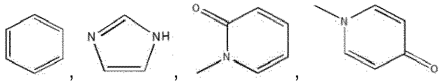

Hydrophobic atoms are annotated with **HydA** and hydrophobic centroids are annotated with **Hyd.** Hydrophobic groups are determined by graph theoretic algorithms.

The fundamental rules for deciding whether an atom is hydrophobic are summarized below:
1. Nitro nitrogen atoms (not in nitrate anions) are hydrophobic.
2. Divalent sulfur atoms with two heavy neighbors bonded only to carbon or sulfur are hydrophobic.
3. Halogens are hydrophobic.
4. Carbon atoms are hydrophobic except
   a) aliphatic carbons π bonded to non-carbon atoms; or
   b) π carbon atoms adjacent to univalent oxygen; or
   c) aromatic carbon adjacent to aromatic oxygen in 5-rings; or
   d) carbon atoms adjacent to two or more {N,O} atoms; or
   e) anionic carbons in c1cccc1;

The assignment of hydrophobic annotations proceeds by first applying the preceding hydrophobic atom typing rules but leaving out fluorine atoms on the grounds that they are small and should not affect annotation placement.

The Unified scheme provides an atom-centered hydrophobic annotation, **HydA** and a centroid hydrophobic feature **Hyd.** The **HydA** annotation is used for hydrophobic atoms that are deemed to have sufficiently high (potential) exposure to a potential receptor. This means that, for example, *sp*3 carbons with 4 heavy neighbors are not marked (since they are buried) and aromatic carbons with two heavy neighbors and two *ortho* substituents are not annotated.

The **Hyd** annotations are assigned by a procedure that groups connected hydrophobic atoms and assigns centroids weighted by an estimate of the likely exposed surface area of each hydrophobic atom; that is, the **Hyd** centroid will be placed closer to more exposed hydrophobic atoms in a hydrophobic group.

The following grouping algorithm is used:
**1. Strip Fluorines.** Remove all fluorines that are not bonded to {H,F} from further consideration. Note that estimates of exposed surface area use a fluorine suppressed molecule.
**2. Rings.** Find all 5-, 6-, 7-, and 8-member rings that are not composed of smaller rings. For each such ring, extract each contiguous stretch of hydrophobic atoms with at least three atoms that have a sufficiently high total exposed surface area, and generate a surface area weighted centroid annotation. Remove all annotated ring atoms from further consideration.
**3. Components.** Find all connected components (single-linkage clusters) among the remaining hydrophobes and remove from consideration those clusters with an exposed surface area sum deemed too small (less than a -CH2- group).
**4. Small Components.** For the remaining hydrophobic components with three or fewer atoms, generate an exposed surface area weighted centroid annotation.
**5. Large Components.** Identify the center of the component graph and generate a weighted centroid at the center including its neighbors. Remove the annotated atoms, splitting the component and apply the Small Components step and/or the Large Components step (recursively) to group the hydrophobes.

MCIM compounds, or a pharmaceutically acceptable salt, hydrate, or solvate thereof which conforms to the pharmacophore model defined herein may be used to treat arthritis as defined herein.

### MCIM compounds

Exemplary MCIM compounds suitable for use in the methods of treatment disclosed herein may be described by the following formula, as disclosed in WO2010/032009 which is hereby incorporated by reference in its entirety: 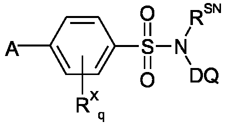 wherein:
-A is independently: 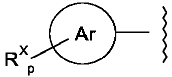
-Ar is independently phenyl, pyridinyl, or pyrimidinyl; and
p is independently an integer from 0 to 3;
and wherein:
q is independently an integer from 0 to 3;
and wherein:
-R^{SN} is independently -H or saturated aliphatic C₁₋₄alkyl;
and wherein:
-DQ is independently -D¹-Q¹ or -D²=O;
-D¹- is independently cyclopentane-di-yl, cyclohexane-di-yl, cycloheptane-di-yl, bicyclo[3.1.1]heptane-di-yl, or bicyclo[3.2.1]octane-di-yl, and is optionally substituted with one or more groups -R^{D};
-D²= is independently cyclopentane-yl-ylidene, cyclohexane-yl-ylidene, cycloheptane-yl-ylidene, bicyclo[3.1.1]heptane-yl-ylidene, or bicyclo[3.2.1]octane-yl-ylidene, and is optionally substituted with one or more groups -R^{D};
each -R^{D} is independently selected
   from -F, -Cl, -Br, -I, -R^{DD}, -CF₃, -OH, -OR^{DD}, -NH₂, -NHR^{DD}, and -NR^{DD}₂; and
each -R^{DD} is independently saturated aliphatic C₁₋₄alkyl;
and wherein -Q¹ is independently selected from: 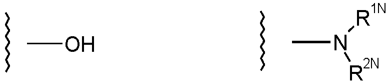 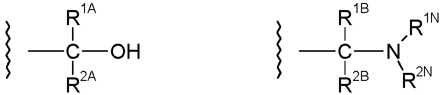 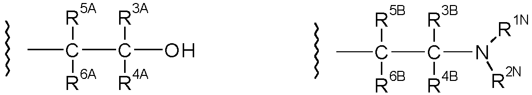 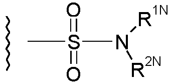 wherein:
each -R^{1N} is independently -H, -R^{CN}, or -R^{CF};
each -R^{2N} is independently -H, -R^{CN}, or -R^{CF};
each -R^{CN} is independently saturated aliphatic C₁₋₄alkyl;
each -R^{CF} is independently saturated aliphatic C₁₋₄fluoroalkyl;
   or:
   -NR^{1N}R^{2N} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
   -R^{1A} is independently -H, -R^{C}, or -R^{F}; and
   -R^{2A} is independently -H, -R^{C}, or -R^{F};
   or -R^{1A} and -R^{2A} together form a saturated aliphatic C₂₋₄alkylene group;
   -R^{1B} is independently -H, -R^{C}, or -R^{F}; and
   -R^{2B} is independently -H, -R^{C}, or -R^{F};
   or -R^{1B} and -R^{2B} together form a saturated aliphatic C₂₋₄alkylene group;
   or -R^{1B} and -R^{2B} together form =O;
   -R^{3A} is independently -H, -R^{C}, or -R^{F}; and
   -R^{4A} is independently -H, -R^{C}, or -R^{F};
   or -R^{3A} and -R^{4A} together form a saturated aliphatic C₂₋₄alkylene group;
   -R^{5A} is independently -H, -R^{C}, -R^{F}, or -R^{J}; and
   -R^{6A} is independently -H, -R^{C}, or -R^{F};
   or -R^{5A} and -R^{6A} together form a saturated aliphatic C₂₋₄alkylene group;
   -R^{3B} is independently -H, -R^{C}, or -R^{F}; and
   -R^{4B} is independently -H, -R^{C}, or -R^{F};
   or -R^{3B} and -R^{4B} together form a saturated aliphatic C₂₋₄alkylene group;
   -R^{5B} is independently -H, -R^{C}, -R^{F}, -OH, or -OR^{O}; and
   -R^{6B} is independently -H, -R^{C}, or -R^{F};
   or -R^{5B} and -R^{6B} together form a saturated aliphatic C₂₋₄alkylene group;
   each -R^{C} is independently saturated aliphatic C₁₋₄alkyl;
   each -R^{F} is independently saturated aliphatic C₁₋₄fluoroalkyl;
   -R^{O} is independently saturated aliphatic C₁₋₄alkyl;
   -R^{J} is independently -NH₂, -NHR^{JN1}, -NR^{JN1}₂, or -NR^{JN2}R^{JN3};
   each -R^{JN1} is independently saturated aliphatic C₁₋₄alkyl; and
   -NR^{JN2}R^{JN3} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
   and wherein each -R^{X} is independently:

   -F, -Cl, -Br, -I,

   -R^{XX},

   -OH, -OR^{XX},

   -SH, -SR^{XX},

   -CF₃, -OCF₃, -SCF₃,

   -NH₂, -NHR^{XX}, -NR^{XX}₂, -NR^{YY}R^{ZZ},

   -C(=O)R^{XX}, -OC(=O)R^{XX},

   -C(=O)OH, -C(=O)OR^{XX},

   -C(=O)NH₂, -C(=O)NHR^{XX}, -C(=O)NR^{XX}₂, -C(=O)NR^{YY}R^{ZZ},

   -OC(=O)NH₂, -OC(=O)NHR^{XX}, -OC(=O)NR^{XX}₂, -OC(=O)NR^{YY}R^{ZZ},

   -NHC(=O)R^{XX}, -NR^{XX}C(=O)R^{XX},

   -NHC(=O)OR^{XX}, -NR^{XX}C(=O)OR^{XX},

   -NHC(=O)NH₂, -NHC(=O)NHR^{XX}, -NHC(=O)NR^{XX}₂, -NHC(=O)NR^{YY}R^{ZZ},

   -NR^{XX}C(=O)NH₂, -NR^{XX}C(=O)NHR^{XX}, -NR^{XX}C(=O)NR^{XX}_{Z}, -NR^{XX}C(=O)NR^{YY}R^{ZZ},

   -CN,

   -NO₂,

   -S(=O)₂NH₂, -S(=O)₂NHR^{XX}, -S(=O)₂NR^{XX}₂, -S(=O)₂NR^{YY}R^{ZZ},

   -S(=O)R^{XX}, -S(=O)₂R^{XX}, -OS(=O)₂R^{XX}, -S(=O)₂OH, or -S(=O)₂OR^{XX};
wherein:
each -R^{XX} is independently saturated aliphatic C₁₋₆alkyl, phenyl, or benzyl, wherein said phenyl and benzyl are optionally substituted with one or more groups selected from: -F, -Cl, -Br, -I, -CF₃, -OCF₃, -R^{XXX}, -OH, -OR^{XXX}, or -SR^{XXX}, wherein each -R^{XXX} is independently saturated aliphatic C₁₋₄alkyl; and
each -NR^{YY}R^{ZZ} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl.

In some embodiments, -Ar is independently phenyl or pyridinyl; p is independently an integer from 1 to 3; q is independently 0 or 1; -R^{SN} is -H; -DQ is -D¹-Q¹; -D¹- is cyclohexane-di-yl and is optionally substituted with one group -R^{D}; -R^{D} is -R^{DD}; and -R^{DD} is methyl; each -R^{X} is independently: -F, -Cl, -Br, -R^{XX}, -OR^{XX}, -CF₃, or -CN; each -R^{XX} is independently methyl and -Q¹ is:

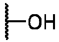

In some embodiments:
-A is: 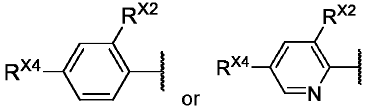
   q is independently 0 or 1;
   -R^{SN} is -H;
-DQ is: 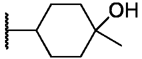
   -R^{X} is independently -F or -Cl;
   -R^{X2} is independently -F, -Cl, -CN, or -CF₃;
   -R^{X4} is independently -F, -Cl, or -CN.

The following exemplary MCIM compounds suitable for use in the methods of treatment disclosed herein may be described by the following chemical structures, as disclosed in WO2014/207445, which is hereby incorporated by reference in its entirety: 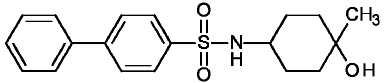
*N*-(4-hydroxy-4-methyl-cyclohexyl)-4-phenyl-benzenesulfonamide 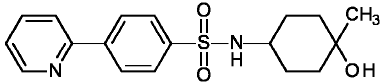
*N*-(4-hydroxy-4-methyl-cyclohexyl)-4-(2-pyridyl)benzenesulfonamide

The MCIM compound may be selected from compounds of the following formulae, or a pharmaceutically acceptable salt, hydrate, or solvate thereof (for convenience, collectively referred to herein as "HMC compounds"):

| | |
|---|---|
| 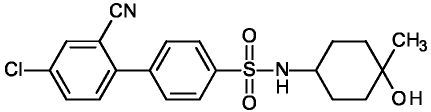 | HMC-C-01 |
| 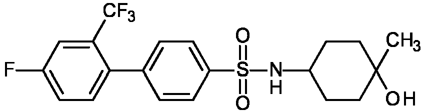 | HMC-C-02 |
| 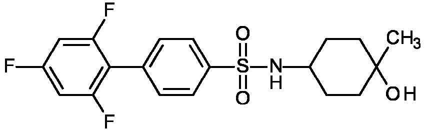 | HMC-C-03 |
| 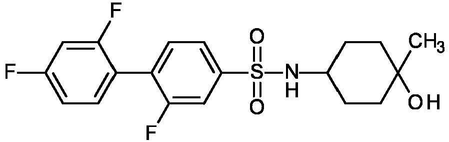 | HMC-C-04 |
| 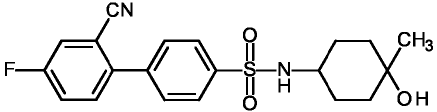 | HMC-C-05 |
| 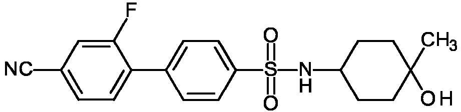 | HMC-C-06 |
| 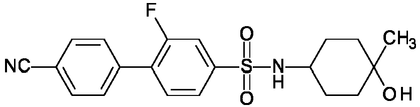 | HMC-C-07 |
| 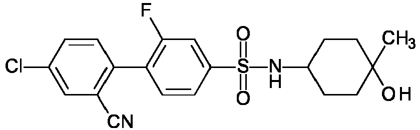 | HMC-C-08 |
| 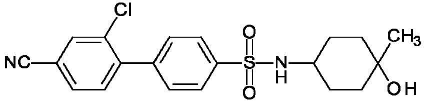 | HMC-C-09 |
| 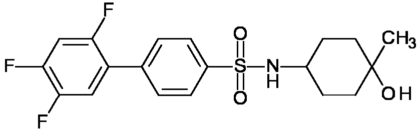 | HMC-C-10 |
| 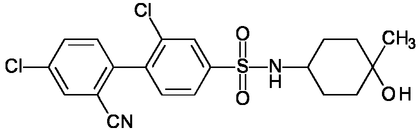 | HMC-C-11 |
| 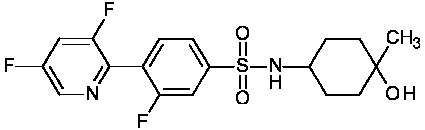 | HMC-N-01 |
| 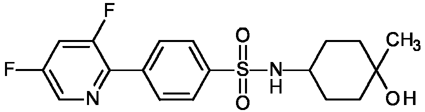 | HMC-N-02 |
| 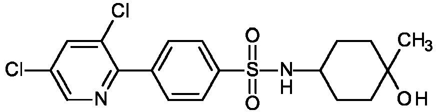 | HMC-N-03 |
| 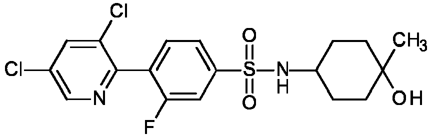 | HMC-N-04 |
| 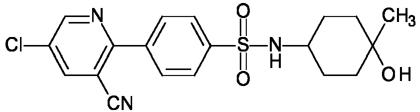 | HMC-N-05 |
| 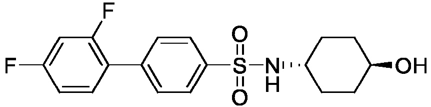 | ABD599 |
| 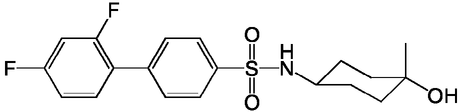 | ABD899 |
| 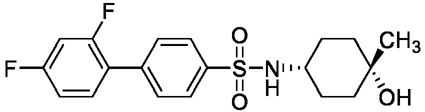 | ABD900 |
| 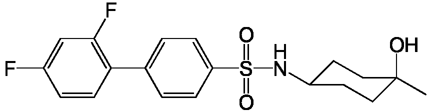 | |

Note that the substituents on one side of the cyclohexyl ring (i.e., -OH and -CH₃ on the right-hand side) may be positioned *"trans"* / *"cis"* or *"cis"* / *"trans"* with respect to the rest of the molecule (that is, on the cyclohexyl ring to which they attached, with respect to the rest of the compound which is attached at the para position of the cyclohexyl ring).

| | |
|---|---|
| 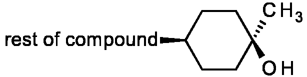 *"cis-OH"* | 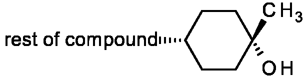 "*cis*-OH" |
| 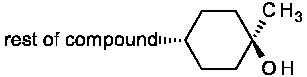 *"trans-OH"* | 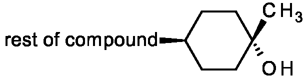 *"trans-OH"* |

Unless otherwise indicated, it is intended that all such conformations are encompassed by a reference to a compound that does not specify a particular conformation.

The compound may be in the "*trans*-OH" conformation, as in, for example, the following compounds:

| | |
|---|---|
| 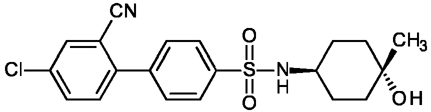 | HMC-C-01-A |
| 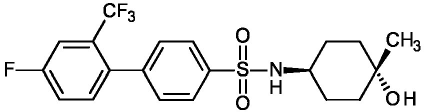 | HMC-C-02-A |
| 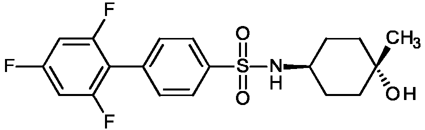 | HMC-C-03-A |
| 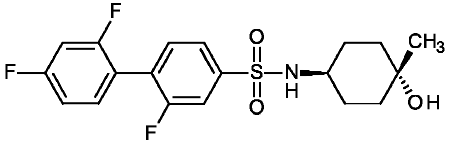 | HMC-C-04-A |
| 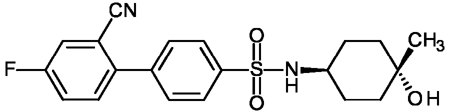 | HMC-C-05-A |
| 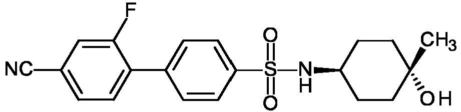 | HMC-C-06-A |
| 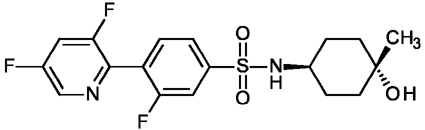 | HMC-N-01-A |
| 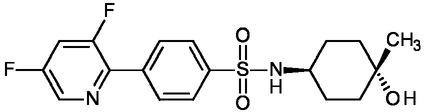 | HMC-N-02-A |
| 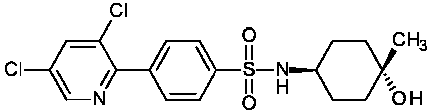 | HMC-N-03-A |
| 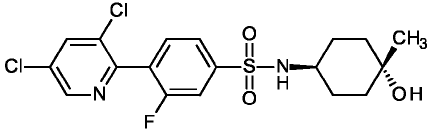 | HMC-N-04-A |

Alternatively, the compound may be in the "*cis*-OH" conformation, as in, for example, the following compounds:

| | |
|---|---|
| 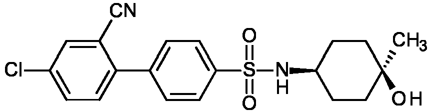 | HMC-C-01-B |
| 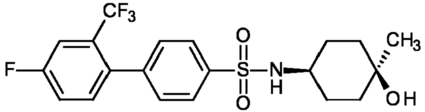 | HMC-C-02-B |
| 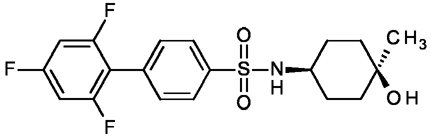 | HMC-C-03-B |
| 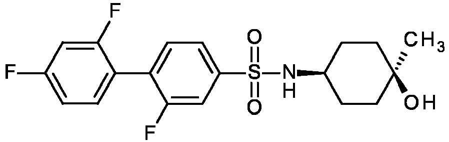 | HMC-C-04-B |
| 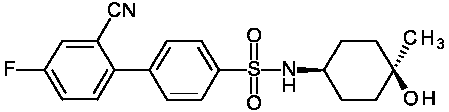 | HMC-C-05-B |
| 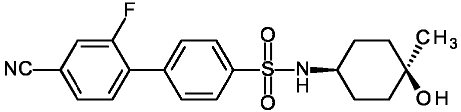 | HMC-C-06-B |
| 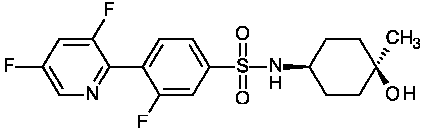 | HMC-N-01-B |
| 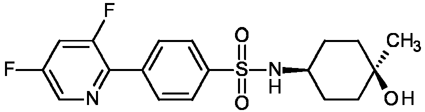 | HMC-N-02-B |
| 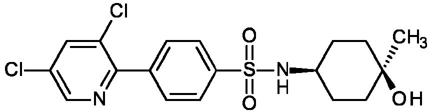 | HMC-N-03-B |

| | |
|---|---|
| 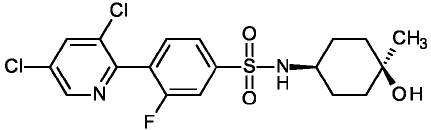 | HMC-N-04-B |

Note also that the cyclohexane ring may take a "chair", "boat", or "twist" conformation, and that interconversion between the conformations is possible. Unless otherwise indicated, it is intended that all such conformations (*e.g*., "chair", "boat", "twist", "OH is axial", "OH is equatorial", etc.) are encompassed by a reference to a compound that does not specify a particular conformation.

In some embodiments, the compound has the following formula:

| | |
|---|---|
| 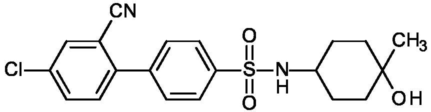 | HMC-C-01 |

In some embodiments, the compound has the following formula:

| | |
|---|---|
| 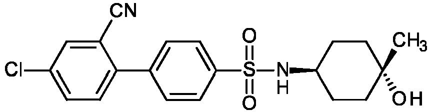 | HMC-C-01-A |

### Disease-modifying antirheumatic drugs

Disease-modifying antirheumatic drugs (DMARDs) are a class of drugs defined by their use in treating inflammatory arthritides, including rheumatoid arthritis (RA), psoriatic arthritis (PsA) and ankylosing spondylitis (AS). DMARDs do not all share a related structure or mechanism of action and can be subdivided into categories. DMARDs include conventional synthetic DMARDs (csDMARDs), targeted synthetic DMARDs (tsDMARDs), biological DMARDs (bDMARDs).

Particular DMARDs may also be used to treat a range of connective tissue diseases, inflammatory diseases and cancers. In particular, some DMARDs may be used to treat one or more of inflammatory bowel diseases (IBD), such as Crohn's disease and ulcerative colitis, systemic lupus erythematosus, psoriasis, gout, Sjögren syndrome, myasthenia gravis, and sarcoidosis.

Conventional synthetic DMARDs (csDMARDs) are small molecule drugs which demonstrate anti-inflammatory function and include, for example, methotrexate, azathioprine, chloroquine, D-penicillamine ciclosporin, gold salts such as sodium aurothiomalate and auranofin, hydroxychloroquine, leflunomide, minocycline and sulfasalazine. Each of the aforementioned csDMARDs may be used, either alone or in combination with another DMARD, in the treatment of RA.

An MCIM as disclosed herein may be used in combination with one or more of methotrexate, azathioprine, chloroquine, D-penicillamine ciclosporin, gold salts such as sodium aurothiomalate and auranofin, hydroxychloroquine, leflunomide, minocycline and sulfasalazine in the treatment of RA. In an example, HMC-C-01-A may be used in combination with methotrexate in the treatment of arthritis, for example RA.

An MCIM may be used in combination, for example, leflunomide, methotrexate and sulfasalazine to treat psoriatic arthritis. An MCIM may be used in combination with methotrexate and sulfasalazine to treat reactive arthritis.

Patients treated with a csDMARD, for example methotrexate which is often given as a first line drug for treatment of RA (often in combination with a second csDMARD), may show an inadequate response to treatment. An adequate response to treatment with a csDMARD may not be achieved from the outset of treatment (i.e. the subject may not achieve a low disease activity and the treatment is inadequate), or a subject may initially respond to treatment, achieving a low disease activity before the effectiveness of the treatment reduces leading to a worsening of symptoms (i.e. the arthritis is refractory). In some embodiments, the subject may have csDMARD-inadequate response (IR) arthritis and/or csDMARD-refractory arthritis. In some embodiments, the subject may have methotrexate-inadequate response (IR) arthritis, for example methotrexate-IR RA. In some embodiments, the subject may have methotrexate-refractory arthritis, for example, methotrexate-refractory arthritis. In some embodiments, the subject may have an inadequate response to a csDMARD, for example methotrexate, for at least 3 months prior to treatment with an MCIM as disclosed herein.

tsDMARDs are small molecule drugs. In contrast to csDMARDs, tsDMARDs are developed to target particular molecular structures, such as particular protein targets. tsDMARDs include, for example, the Janus kinase (JAK) inhibitor compounds baricitinib, filgotinib, and tofacitinib, the Tyk2 inhibitor, deucravacitinib, and the phosphodiesterase 4 (PDE4) inhibitor apremilast.

bDMARDs include monoclonal antibodies and receptor constructs which target either membrane bound or soluble proteins. For example, bDMARDs include the tumor necrosis factor (TNF) inhibitors adalimumab, certolizumab, etanercept (a decoy TNF receptor), golimumab, and infliximab. bDMARDs also include the CTLA4-Ig, abatacept, the IL-1 receptor antagonist, anakinra, the CD20 inhibitor, rituximab, the IL-6 inhibitors sarilumab and tocilizumab, the IL17A inhibitors, secukinumab, ixekizumab and bimekizumab, the IL23 inhibitors ustekinumab, risankizumab and guselkumab, and the BAFF Inhibitor belimumab.

tsDMARDs and bDMARDs are typically second-line drugs for treatment of RA and are administered when a subjects RA is non-responsive to, or becomes refractory to, csDMARDs such as
methotrexate. However, in addition to a spectrum of adverse events, up to a third of patients fail to respond to therapy with the most commonly used bDMARDs, the anti-TNFα agents. Thus, in some embodiments, the subject may have tsDMARD- IR arthritis and/or bDMARD- IR arthritis. In some embodiments, the subject may have tsDMARD-refractory arthritis and/or bDMARD-refractory arthritis. For example, the subject may have an inadequate response to, or be refractory to, treatment with an anti-TNFα inhibitor, for example adalimumab, certolizumab, etanercept (a decoy TNF receptor), golimumab, and infliximab. In some embodiments, the subject may have methotrexate-refractory arthritis, for example, methotrexate-refractory arthritis. Difficult-to-treat rheumatoid arthritis (D2T RA) is defined by the European League Against Rheumatism (EULAR) Task Force as RA which shows signs of active/progressive disease after failing treatment with csDMARD therapy and ≥ 2 b/tsDMARDs therapies with different mechanisms of action (Watanabe, 2022). Such a disease state is associated with uncontrolled disease activity and a significant decrease in the quality of life of a subject suffering from D2T RA. An MCIM as disclosed herein may be used to treat D2T RA.

### Co-administration

Adjunct therapies are used together with the 'primary' treatment to assist the primary treatment in achieving disease control. In some cases, treatment with DMARDs may only achieve low or moderate control of disease activity. In such cases, MCIM compounds as disclosed herein may be used as an adjunct therapy to increase control of disease activity and thereby improve structural progression, reduced disability progression, and increase health-related quality of life in a subject.

In some embodiments, the MCIM compound is an adjunct therapy. For example, the MCIM compound may be administered with a primary treatment. The primary treatment may comprise a DMARD, csDMARD, bDMARD and/or a tsDMARD. For example, the primary treatment may comprise one or more of csDMARDS selected from the following list: methotrexate azathioprine, chloroquine, D-penicillamine, cyclosporin, gold salts such as sodium aurothiomalate and auranofin, hydroxychloroquine, leflunomide, minocycline and sulfasalazine. Alternatively, or in addition to, the primary treatment may comprise one or more of the bDMARDs selected from the following list: adalimumab, certolizumab-pegol, etanercept, golimumab, infliximab, abatacept, anakinra, rituximab, sarilumab, tocilizumab, secukinumab, ixekizumab, bimekizumab, guselkumab, and belimumab. Alternatively, or in addition to, the primary treatment may comprise one or more of the tsDMARDs selected from the following list: upadacitinib, baricitinib, filgotinib, tofacitinib, deucravacitinib, and apremilast. In particular, the primary therapy may comprise one or more primary therapies selected from the following list: methotrexate, a tsDMARD, Upadacitinib, baricitinib, tofacitinib, deucravacitinib and filgotinib.

Alternatively to an MCIM compound acting as an adjunct therapy, the MCIM compound may be used as part of a primary treatment in combination with a DMARD. The method of treating arthritis and/or an age-related musculoskeletal disease or disability may therefore further comprises administering a disease modifying antirheumatic drug (DMARD). Thus, the present disclosure also provides an MCIM as disclosed herein for use in a method of treating arthritis and/or an age-related musculoskeletal disease or disability, wherein the method further comprises administering a DMARD. The present disclosure further provides a pharmaceutical composition comprising an MCIM compound for use in a method of treating arthritis and/or an age-related musculoskeletal disease or disability, wherein the method further comprises administering a DMARD. Also provided is a DMARD for use in a method of treating arthritis and/or an age-related musculoskeletal disease or disability, wherein the method further comprises administering an MCIM compound.

In the methods disclosed herein, administration of the MCIM compound and the DMARD may be separate, sequential, or simultaneous. Also provided is a pharmaceutical composition comprising an MCIM compound and a DMARD for use in a method of treating arthritis and/or an age-related musculoskeletal disease or disability. Also provided is the use of an MCIM compound in the manufacture of a medicament for use in a method of treating arthritis and/or an age-related musculoskeletal disease or disability, wherein the method further comprises administering a DMARD. Also provided is the use of a DMARD in the manufacture of a medicament for use in a method of treating arthritis and/or an age-related musculoskeletal disease or disability, wherein the method further comprises administering an MCIM compound.

Further provided is a method of treating or preventing an arthritis and/or functional decline, for example age-related musculoskeletal disease or disability, the method comprising administering a therapeutically- or prophylactically-effective amount of (i) MCIM compound and (ii) a DMARD to a subject in need of treatment.

The present disclosure also provides an MCIM compound and DMARD (e.g. in the form of a pharmaceutical combination or pharmaceutical composition comprising an MCIM compound and a DMARD) for use in a method of treating or preventing arthritis and/or an age-related musculoskeletal disease or disability. Also provided is the use of an MCIM compound and a DMARD (e.g. in the form of a pharmaceutical combination or pharmaceutical composition comprising an MCIM compound and a DMARD) in the manufacture of a medicament for use in a method of treating or preventing arthritis and/or an age-related musculoskeletal disease or disability. Also provided is a method of treating or preventing arthritis and/or an age-related musculoskeletal disease or disability, the method comprising administering a therapeutically- or prophylactically-effective amount of an MCIM compound and a DMARD (e.g. in the form of a pharmaceutical combination or pharmaceutical composition comprising an MCIM compound and DMARD) to a subject in need of treatment.

In some aspects and embodiments, the MCIM compound and the DMARD may be provided as a combination therapy. In some embodiments, the MCIM compound and the DMARD may be administered simultaneously or sequentially. Simultaneous administration refers to administration of the two or more agents together, for example as a pharmaceutical composition containing both agents (i.e. as a combined preparation), or immediately after one another (e.g. within 1, 4, 6, 8 or 12 hours), and optionally via the same route of administration, e.g. to the same artery, vein or other blood vessel. Sequential administration refers to administration of one of the agents followed after a given time interval by separate administration of another agent. It is not required that the agents are administered by the same route, although this is the case in some embodiments. The time interval may be any time interval.

Also provided is the use of an MCIM compound in the manufacture of a medicament for use in treating or preventing arthritis and/or an age-related musculoskeletal disease or disability, wherein treating or preventing the arthritis and/or an age-related musculoskeletal disease or disability further comprises administering a DMARD.

### Pharmaceutical compositions

The MCIM compounds disclosed herein may be in the form of a medicament or pharmaceutical composition. The medicaments or pharmaceutical composition may be formulated for administration by a number of routes, including but not limited to, parenteral i.e. non-oral route (for example, by injection: sub-cutaneous, intravenous, intra-arterial, intramuscular, or intratumoral; by topical or intradermal; by inhalation or intranasal; or by rectal), and peroral i.e. oral route. The medicaments and compositions may be formulated in a solid, semi-solid, or liquid dosage form. The pharmaceutical compositions according to this invention may be delivered by a route which facilitates exposure in the systemic circulation or by a route or method which gives rise to localised, targeted, delivery of the active compounds to a selected region in the body. The pharmaceutical compositions according to this invention may also be administered to humans or animals.

Administration is preferably in a "therapeutically effective amount", this being sufficient to show benefit to the individual. The actual amount administered, and rate and time-course of administration, will depend on the nature and severity of the disease being treated. For example, the rate of release of the active compounds from the pharmaceutical composition may be immediate, sustained, extended, controlled, pulsatile or follow a pattern that is optimal for the intended therapeutic application. The frequency of dosing can be fixed or variable depending on the rate of drug release, the required level in the systemic circulation or at the target site to provide the desired therapeutic effect. Prescription of treatment, e.g. decisions on dosage etc., is within the responsibility of physicians and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 23rd Edition, 2020, pub. Lippincott, Williams & Wilkins.

Pharmaceutical compositions may be prepared using a pharmaceutically acceptable "carrier" composed of materials, also known as excipients, that are generally considered safe and pharmacologically inert. "Pharmaceutically acceptable" refers to molecular entities and compositions that are "generally regarded as safe - GRAS", e.g., that are physiologically tolerable and do not typically produce adverse or untoward effects when administered to a human. In some embodiments, this term refers to molecular entities and compositions approved by a regulatory agency of the US federal or a state government, as the GRAS list under section 204(s) and 409 of the Federal Food, Drug and Cosmetic Act, that is subject to premarket review and approval by the FDA or similar lists, the U.S. Pharmacopeia or another generally recognised pharmacopeia for use in animals, and more particularly in humans.

The term "carrier" refers to excipients that serve one or more functions in the pharmaceutical compositions, for example, as diluents, binders, lubricants, glidants, disintegrants, solubilisers or solubility enhancers, stabilisers, preservatives, taste-masking or taste-modifying agents, flavourant, and/or colourant. The pharmaceutical compositions, when formulated as a solid dosage form, may further be uncoated or coated with an outer layer to impart aesthetic features, physical protection, enhance the physical and/or chemical stability of the active compounds or other constituents of the pharmaceutical compositions, and/or alter the rate of dissolution and release of the active compounds from the pharmaceutical compositions. The pharmaceutical compositions, when formulated as a parenteral injectable dosage form, may additionally contain aqueous or non-aqueous solvents, cosolvent mixtures, buffering agents, surfactants, tonicity modifying agents, chelating agents, pH modifiers, viscosity modifiers, and/or suspending agents. The pharmaceutical compositions, when formulated as a parenteral dosage form for inhalation, may additionally contain particle carriers for the pulmonary delivery of the active compounds, absorption penetration enhancers, and/or propellants, dependent on the inhalation delivery device to be used. Those with skill in the art are familiar with such pharmaceutical carriers, and excipients therein, and methods of compounding these carriers and excipients into pharmaceutical compositions. Suitable excipients for use in the pharmaceutical compositions can be found in standard pharmaceutical texts, for example, Handbook of Pharmaceutical Excipients, 9th edition, Pharmaceutical Press, American Pharmaceutical Association, 2020.

The choice of the pharmaceutical carrier is dependent on the dosage form type, the dose, intended route of delivery, the physicochemical properties of the active compounds, and the required bioavailability and/or exposure profile. When used, the excipients of the pharmaceutical compositions should not adversely affect the stability, bioavailability, safety, and/or therapeutic efficacy of the active compounds, i.e. the MCIM compound used in the pharmaceutical compositions. Thus, the skilled person will appreciate that pharmaceutical compositions are provided wherein there is no significant incompatibility between any of the components of the dosage form.

It may be convenient or desirable to prepare, purify, and/or handle a corresponding solvate of the active compound(s). The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a dihydrate, a tri-hydrate, etc.

Unless otherwise specified, a reference to a particular compound also includes the solvate forms thereof.

It may be convenient or desirable to prepare, purify, and/or handle a corresponding salt of the active compound(s), for example, a pharmaceutically-acceptable salt of an MCIM compound, for example HMC-C-01-A. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group which may be anionic (e.g., COOH may be COO ), then a salt may be formed with a suitable cation. If the compound is cationic, or has a functional group which may be cationic (e.g., NH2 may be NH3+), then a salt may be formed with a suitable anion.

### MCIM dose

Prescription of treatment, e.g. decisions on dosage etc., is within the responsibility of physicians and other medical doctors as discussed above. For example, a patient may be administered or prescribed between about 0.5-500 mg, about 1-250 mg, about 2-125 mg, about 4-60 mg, or about 5-40 mg MCIM compound per treatment. A patient may be administered or prescribed treatment with an MCIM compound daily. A patient may be administered or prescribed treatment with an MCIM compound daily for up to 12 weeks. In some embodiments, the patient may be administered or prescribed 5-40 mg MCIM per day for 12 weeks. In some embodiments, the patient may also be administered or prescribed methotrexate. In embodiments wherein the patient is being treated for arthritis, the arthritis may be methotrexate-refractory arthritis. In embodiments wherein the patient is being treated for RA, the RA may be methotrexate-refractory RA.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Methods of treatment

It is to be understood that where reference is made to an active compound for use in the treatment of a patient this also refers to a method of treating a patient, wherein the method comprises administering said active compound to the patient and vice versa. Likewise, it is to be understood that such references are to be considered as encompassing the manufacture of a medicament comprising the active compound for use in a method of treating a patient.

### Patient

The term patient and subject are used herein interchangeably. The subject to be treated may be any animal or human. The subject is preferably mammalian, more preferably human. The subject may be a non-human mammal, but is more preferably human. The subject may be male or female. The subject may be a patient. Therapeutic uses may be in human or animals (veterinary use).

### Examples

### EXAMPLE 1

HMC-C-01-A represents a way of treating RA, not only to reduce levels of inflammation but to also directly prevent bone damage. This clinical study was a randomised, double-blind, placebo-controlled, dose-ranging study to investigate the efficacy, safety and tolerability of HMC-C-01-A in patients with moderate to severe active rheumatoid arthritis, wherein the patients have previously shown an inadequate response to methotrexate alone.

248 patients with moderate to severe active RA, who had not responded to treatment with methotrexate for at least three months, were enrolled on the trial and randomly assigned to receive 1 of 3 doses of HMC-C-01-A (5 mg, 20 mg, or 40 mg) or placebo (a "dummy" drug). Following screening, patients were treated for a period of 12 weeks, and a follow-up Period of 6 weeks. Patients received HMC-C-01-A once daily and methotrexate once weekly during the treatment period.

During the treatment and follow-up periods, patients attended a hospital or clinic for regular visits during which they underwent planned study assessments to evaluate the effectiveness, tolerability and safety of HMC-C-01-A. Baseline values were acquired for each patient prior to commencement of treatment with HMC-C-01-A and then assessed again at weeks 4, 8, and 12 of treatment depending on the nature of the variable. Finally, a follow up appointment was carried out at week 18.

### Tender and swollen joint counts

Rheumatoid arthritis (RA) is characterized by joint tenderness and swelling and Tender and Swollen Joint Counts (TJC, SJC) are well established in RA as standard measures of joint involvement as disclosed in Sokka and Pincus (2009) which is hereby incorporated by reference in its entirety. Assessing this involves measuring the extent of tenderness and swelling. Joint swelling is soft tissue swelling that is detectable along the joint margins. Fluctuation is a characteristic feature of swollen joints and it may influence the range of joint movement. Joint tenderness is the presence of pain in a joint at rest with pressure or on movement of the joint, e.g. on movement of the hip joints, and is assessed by exerting pressure on the joint to elicit tenderness.

A number of joint counts have been developed; the principle two being the ACR 66/68 counts for swollen and tender joints, and the 28-joint count. The 66/68-joint count evaluates the following joints: upper-temporomandibular, sternoclavicular, acromioclavicular, shoulder, elbow, wrist, metacarpophalangeal, proximal interphalangeal and distal interphalangeal; lower-hip, knee, ankle, tarsus, metatarsophalangeal and interphalangeal, as described in Deandrade and Casagrande (1965) which is incorporated in its entirety by reference. The 28-joint count evaluates the following joints: upper-shoulder, elbow, wrist, metacarpophalangeal and proximal interphalangeal; lower-knee, as described in Fuchs et al (1989) which is incorporated in its entirety by reference.

The total swollen and tender joint counts are calculated as the sum of the swollen and the sum of the tender joint counts per patient and the change from baseline in calculated. The data are presented as the mean value for the group, and the mean change from the baseline value. A greater reduction from baseline reflects a greater impact of treatment.

### Patients' assessment of pain VAS

Pain is the most prominent symptom in the majority of people with arthritis and is measured using a visual analogue scale (VAS), as described in Mainland (1965) which is incorporated in its entirety by reference. The standard visual analog scale is a 10 cm scale with a border on each side. To the left of the "0" mark appears the indication "No pain at all", and to the right of the "10" mark "Pain as bad as it could be". The position of the mark is measured and the change in the position of the mark after time is calculated. The data are presented as the mean value for the group, and the mean change from the baseline value. A greater reduction from baseline reflects a greater impact of treatment.

### ACR20/50/70

The ACR20 is a composite measure defined as an improvement of 20% in the number of tender and swollen joints and a 20% improvement in three of the following five criteria: patient global assessment, physician global assessment, functional ability measure [most often Health Assessment Questionnaire (HAQ)], visual analog pain scale, and erythrocyte sedimentation rate or C-reactive protein (CRP). ACR50 and ACR70 are the same instruments with improvement levels defined as 50% and 70% respectively versus 20% for ACR20.

The data are calculated as the percentage of individuals showing this threshold effect across each treatment group.

### DAS28-CRP

The DAS index combines information relating to the number of swollen and tender joints, in addition to a measure of general health, and the acute phase response, such as change in C-reactive protein and is described in Wells et al (2009) and Prevoo (1995) et al, each of which are hereby incorporated in their entirety by reference. The DAS28 is based on a count of 28 swollen and tender joints, with a score ranging from 0 to 9.4 and can be used to objectively evaluate a patient's response to treatment. An absolute level of disease activity can be selected as a clinically meaningful goal for therapeutic intervention; with a value of ≤3.2 defined as the threshold for a low disease activity state and <2.6 as the threshold for remission. The DAS28-CRP method can be carried out as described in Leong et al (2020) which is hereby incorporated by reference in its entirety.

The DAS28 is calculated according to the formula below for each patient at each visit. The data are presented as the mean value for the group, and the mean change from the baseline value. A greater reduction from baseline reflects a greater impact of treatment.
DAS28 (CRP)=0.56*√(TJC28)+0.28*√(SJC28)+0.014*GH+0.36*In(CRP+1)+0.96

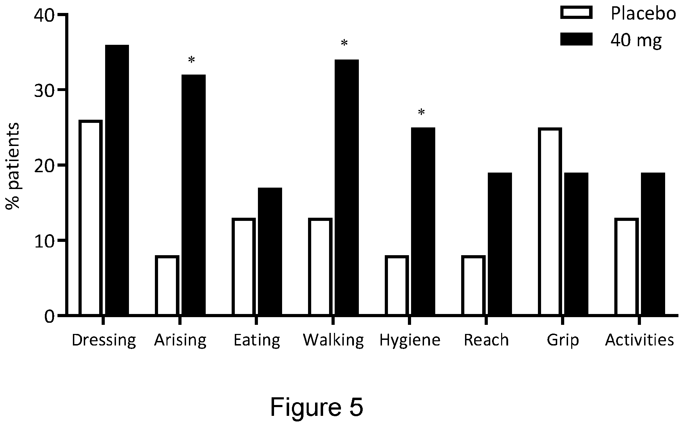

In patients with moderate to severe active RA, HMC-C-01-A showed no clear effect on tender and swollen joint counts, or patient's assessment of pain VAS compared to patients treated with placebo (Table 2). By contrast, approved DMARDs reduce tender and swollen joints, pain VAS and patient assessments, and show pronounced effects on clinical scores such as ACR20/50/70 and joint counts (e.g. tender joint counts (TJC) and swollen joint counts (SJC)). It is noteworthy that while patients did not report an overall reduction of pain in the clinic, when asked to assess pain associated with daily activities, such as dressing, arising and walking using the HAQ-DI scoring method, patients did report reduced pain associated with these activities, with a large number reporting no pain following treat (Figure 5).

**Table 2. Effect of MCIM compounds on joints.**

| Table 2 | | | | | | | | |
|---|---|---|---|---|---|---|---|---|
| Parameter | Baseline | | | | Week 12 | | | |
| | Placebo | 5 mg | 20 mg | 40 mg | Placebo | 5 mg | 20 mg | 40 mg |
| ACR20 | N/A | | | | 48.8 | 50.9 | 46.9 | 54.7 |
| DAS28-CRP | 6.37 (1.0) | 6.38 (0.9) | 6.35 (1.0) | 6.22 (0.9) | -1.26 (1.46) | -1.44 (1.16) | -1.33 (1.05) | -1.47 (1.45) |
| Tender joint count 68 | 22.0 (11.3) | 21.2 (9.1) | 22.2 (10.2) | 22.0 (11.3) | -7.5 (12.3) | -8.6 (8.3) | -8.8 (9.8) | -7.9 (12.1) |
| Swollen Joint count 66 | 17.3 (9.3) | 14.8 (7.8) | 16.3 (7.3) | 15.9 (7.7) | -5.5 (11.4) | -6.2 (7.1) | -6.3 (8.4) | -5.6 (10.4) |
| Pain VAS | 64.8 (20.9) | 66 (19.0) | 63.5 (18.3) | 63.8 (18.7) | -25.5 (33.9) | -18.3 (27.8) | -18.7 (23.4) | -23.5 (27.1) |
| Patient assessment of disease | 67.8 (13.0) | 66.9 (15.4) | 67.0 (15.9) | 67.6 (14.0) | -24.7 (31.5) | -21.2 (29.6) | -22.9 (23.0) | -27.5 (26.0) |

### EXAMPLE 2

### RAMRIS

For most patients RA remains a lifelong condition with impaired quality of life (QoL), the burden of long-term therapy, and the potential to suffer disease flares. Up to 75% of patients with RA fail to achieve clinical remission with current therapy, and even fewer achieve sustained remission (Olsen, 2019). Increasing evidence supports a role for ongoing sub-clinical inflammation and the presence of erosions in the ongoing symptoms, pain, and propensity to flare experienced by RA patients either with or without treatment. Up to 96% of patients in clinical remission receiving disease modifying antirheumatic drugs (DMARDs) showed sub-clinical inflammation (Brown, 2006), and patients achieving low disease activity scores (DAS) may nevertheless commonly present with 2-3 swollen joints (Felson, 2022). One reason for this may be the emergence of bone erosions as immunologically active units that can propagate synovitis and act as a key source of bone pain.

Current draft US Food and Drug Administration (FDA) (FDA, 2013) and European Medicine Agency (EMA) guidelines (EMA, 2017) recognize that the reduction in progression of structural damage is an important predictor of long-term benefits in delaying or preventing the progression to disability related to RA. The primary imaging modality for the evaluation of structural joint damage in clinical practice and clinical trials in RA has been conventional radiography (CR), with analysis using validated scoring methods such as the Sharp/van der Heijde method (Van der Heijde, 1996), which is hereby incorporated by reference in its entirety. However, this method is associated with several disadvantages, including:
1. insensitivity for bone erosions - both small early bone erosions, and large established erosions with intramedullary extension.
2. low sensitivity for subchondral bone abnormalities, an important site of pathology, including pre-erosive osteitis and intramedullary extension of bone erosions.
3. inability to visualize hyaline cartilage directly, resulting in insensitivity and inaccuracy for articular cartilage loss, an important cause of disability in RA.
4. inability to visualize intra-articular synovitis, tenosynovitis, and other soft tissue abnormalities and inflammation, including intramedullary osteitis.
5. projectional superimposition because of the 2D representation of 3D pathology
6. indirect evaluation of cartilage loss by joint-space narrowing, rather than direct assessment.
7. the long duration typically needed to demonstrate inhibition of structural progression (24-52 weeks).

Given these limitations, a validated imaging method that more reliably detects structural progression within a 3-month time frame and enables the assessment of additional features of disease would strongly aid drug development and the ability to assess modification of underlying disease in RA, with the aim of addressing the ongoing unmet needs of patients. Magnetic resonance imaging (MRI) allows the monitoring of structural damage and soft tissue features of RA and is more sensitive to detect change than CR assessment.

The OMERACT RAMRIS was developed and validated from 1998 to 2002 by the OMERACT MRI Working Group as described in Østergaard (2003a). The OMERACT RAMRIS methodology is described in the OMERACT Handbook (Beaton et al, version 2.1, 2021), which is hereby incorporated by reference in its entirety. A core set of MRI acquisitions, joint pathology definitions, and a scoring system for semi-quantitative evaluation of bone erosion, bone marrow edema (osteitis), and synovitis were provided, which were further revised and updated in 2016 to define areas for assessment, a recommended scoring system, and image capture guidance described in Østergaard (2017), which is hereby incorporated by reference in its entirety. The approach is now the standard MRI method used in RA clinical trials (Østergaard, 2005; Peterfy, 2013; ACR/OMERACT, 2013).

The following definitions of RA joint pathologies of synovitis, bone erosion, joint space narrowing, and osteitis/bone marrow edema were recommended (Østergaard, 2017) as shown in **Error! Reference s**

### ource not found.:

- Synovitis: An area in the synovial compartment that shows above-normal post-gadolinium enhancement (signal intensity increase) of a thickness greater than the width of the normal synovium.
- MRI bone erosion: A sharply marginated bone lesion, with correct juxta-articular localization and typical signal characteristics, which is visible in 2 planes with a cortical break seen in at least 1 plane.
- MRI osteitis/bone marrow edema: A lesion within the trabecular bone, with ill-defined margins and signal characteristics consistent with increased water content.
- MRI joint space narrowing: Reduced joint space width compared to normal, as assessed in a slice perpendicular to the joint surface.

The 2016 updated OMERACT RAMRIS system is detailed below.

| | |
|---|---|
| Bone erosion | • Each bone (wrists: distal radius, distal ulna, carpal bones, metacarpal bases; MCP joints: metacarpal heads, phalangeal bases) is scored separately. |
| | • The scale is 0-10, based on the proportion of eroded bone compared to the "assessed bone volume" judged on all available images: 0 = no erosion; 1 = 1-10% of bone eroded; 2 = 11-20%, etc. For long bones, the "assessed bone volume" is from the articular surface (or its best estimated position if absent) to a depth of 1 cm, while in carpal bones it is the whole bone. |
| | • In case a bone is fused with another bone, bone erosion is scored as 10 in the bone. |
| Osteitis/bone marrow edema | • Each bone is scored separately (as for erosions). |
| | • The scale is 0-3 based on the proportion of bone with osteitis, as follows: 0 = no osteitis; 1 = 1-33% of bone with osteitis; 2 = 34-66%; 3 = 67-100%. |
| Synovitis | • Synovitis is assessed in 3 wrist regions (1. The distal radioulnar joint; 2. The radiocarpal joint; 3. The intercarpal and carpometacarpal joints) and in each MCP joint. The first carpometacarpal joint is not scored. |
| | • The scale is 0-3. Score 0 is normal, while 1-3 (mild, moderate, severe) are by thirds of the presumed maximum volume of enhancing tissue in the synovial compartment. |
| Joint space narrowing | • Joint space narrowing is assessed at 17 locations in the wrist, between distal radius and carpal bones (2 sites), between the carpal bones (except the pisiform; 10 sites), and between carpal bones and each metacarpal bone (5 sites), and in each MCP joint. |
| | • The scale is 0-4, as follows: 0 = no narrowing; 1 = focal or mild (< 33%) narrowing; 2 = moderate (34-66%) narrowing; 3 = moderate to severe (67-99%) narrowing; 4 = ankylosis. |

OMERACT=Outcome Measures in Rheumatology; RAMRIS=Rheumatoid Arthritis Magnetic Resonance Imaging Scoring system; MCP=metacarpophalangeal joints.

The OMERACT RAMRIS has high intra and inter-reader reliability with a high level of sensitivity to change. For example, 8 out 9 RCTs that used MRI scoring, high inter- and intra-reader intraclass correlation coefficients (ICCs) of 0.68-0.98 and 0.73-0.98, respectively, were observed (ACR/OMERACT, 2013). In addition, RAMRIS showed good reliability and sensitivity to change in multi-reader and longitudinal settings for parameters of synovitis, erosion, and bone marrow edema/osteitis (Haavardsholm, 2008).

Images are captured from the worst affected hand at the baseline and week 12 visits and transferred to an independent blinded central MRI reading facility for review and scoring. Two independent readers assessed bone erosion, synovitis, bone marrow edema/osteitis and cartilage loss by means of the OMERACT RAMRIS system, using the method described by Conaghan et al. (2016), which is hereby incorporated by reference in its entirety. The mean value between readers for each image is then calculated, and from this a change from baseline value calculation. The data are presented as the mean value at baseline and the mean change from baseline by treatment group.

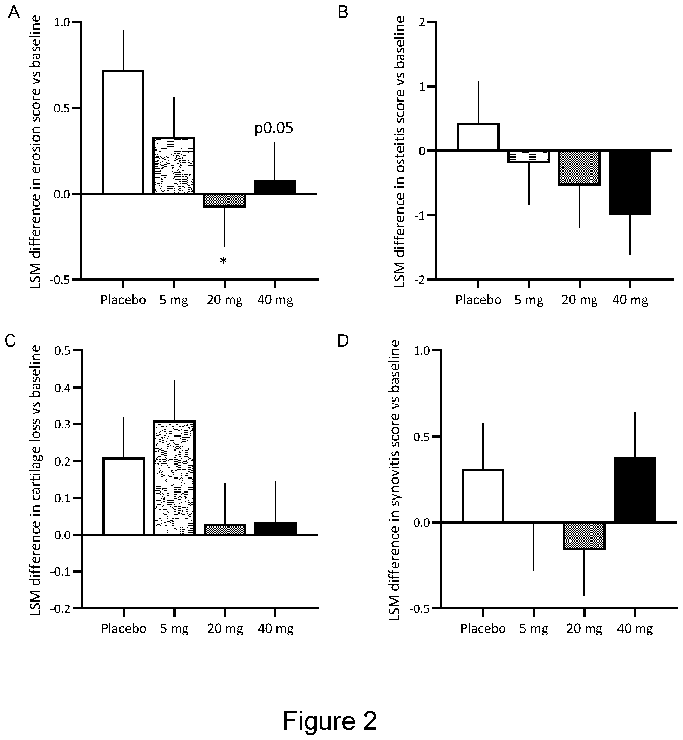

While HMC-C-01-A did not show a clear effect on tender or swollen joint counts or the pain VAS in this study, it did have a pronounced effect on indicators of structural damage such as bone erosions and cartilage loss, as well as reducing osteitis (bone inflammation), although synovitis (inflammation of synovial membrane) was not clearly impacted (Figure 2 and Table 3). These structural changes are accompanied by a reduction in the acute phase reactant, C-reactive protein (CRP) and other inflammatory markers such as rheumatoid factor (Table 4).

**Table 3. Effect of MCIM compounds on damage indicators. * indicates statistical significance, p<0.05. ^{a} p = 0.1**

| Table 3 | | | | | | | | |
|---|---|---|---|---|---|---|---|---|
| Parameter | Baseline | | | | Change from baseline | | | |
| | Placebo | 5 mg | 20 mg | 40 mg | Placebo | 5 mg | 20 mg | 40 mg |
| Osteitis | 5.24 (8.0) | 5.92 (8.4) | 5.44 (8.6) | 4.96 (8.7) | 0.4 (6.6) | -0.2 (3.2) | -0.7(2.1) | -0.9(6.0) |
| Erosion | 13.3 (10.9) | 11.9 (15.2) | 11.6 (12.0) | 9.9 (9.6) | 0.7 (2.0) | 0.3 (1.3) | -0.1 (1.7)* | 0.1 (1.4)a |
| Synovitis | 7.11 (4.6) | 7.70 (6.1) | 7.56 (5.1) | 7.17 (5.1) | 0.4 (2.0) | 0 (1.9) | -0.4(1.2) | 0.4 (2.2) |
| % No erosion progression | N/A | | | | 51.4 | 67.9 | 65.7 | 72.0* |

**Table 4. Effect of MCIM compounds on inflammatory biomarkers. * indicates statistical significance, p<0.05.**

| Table 4 | | | | | | | | |
|---|---|---|---|---|---|---|---|---|
| Parameter | Baseline | | | | Change from baseline | | | |
| | Placebo | 5 mg | 20 mg | 40 mg | Placebo | 5 mg | 20 mg | 40 mg |
| CRP- mean | 10.7 (9.4) | 20.3 (28.0) | 16.1 (30.0) | 14.7 (16.8) | 2.80 (11.2) | 0.88 (18.4) | -4.13 (20.6) | -5.73 (27.7) * |
| CRP- median | 7.3 | 8.6 | 7.0 | 8.4 | -0.30 | -0.20 | -0.10 | -2.50 * |
| Rheumatoid factor, mean | 165.8 (171.1) | 180.7 (210.7) | 184.3 (228.9) | 176.0 (186.4) | 10.4 | 6.8 (80.9) | -5.0 (60.4) | -11.3 (88.0) |

### EXAMPLE 3

Patient reported outcomes are improved following treatment with MCIMs. Accompanying the changes shown in Tables 3 and 4 are pronounced effects on quality of life, including the HAQ-disability index, EULAR-RA Impact of Disease (RAID) and the EQ-5D-5L VAS scale (Table 5).

**Table 5. Effect of MCIM compounds on quality-of-life indicators. BL= Baseline, CFB = change from baseline.**

| Table 5 | | | | | | |
|---|---|---|---|---|---|---|
| Mean (SD) | HAQ-DI | | EULAR-RAID | | FACIT-F | |
| | BL | CFB | BL | CFB | BL | CFB |
| Placebo | 1.6 (0.7) | 0.3 (0.6) | 6.04 (2.0) | -1.7 (2.4) | 28.7 (12.2) | 5.2 (11.4) |
| 5 mg | 1.7 (0.6) | -0.6 (0.5)* | 6.46 (1.6) | -2.1 (2.0) | 26.8 (9.8) | 8.9 (9.6) |
| 20 mg | 1.6 (0.5) | -0.4 (0.6) | 6.38 (1.4) | -1.7 (1.9) | 26.5 (8.5) | 6 (8.7) |
| 40 mg | 1.7 (0.6) | -0.6 (0.7)^{a} | 6.38 (1.8) | -2.5 (2.5) | 26.3 (10.4) | 8.8 (10.8) |

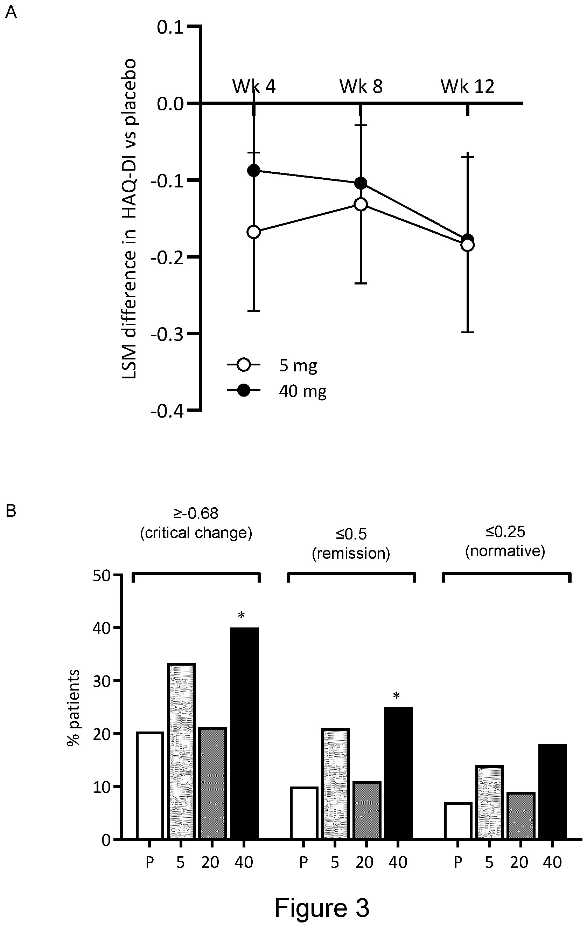

HAQ-DI shows a greater proportion of patients achieving minimal and critical improvements in disability (Figure 3A). In addition, there is a clear improvement in patients achieving a normative HAQ-DI score of 0.25. In addition, treatment with HMC-C-01-A led to an increase in the number of patients reporting a minimal clinically important difference (MCID; the smallest improvement considered worthwhile by a patient) when treated with both 5 mg and 40 mg HMC-C-01-A compared to those receiving placebo (Table 6). The difference in clinically important differences (CID; an improvement following treatment which is not necessarily minimal) is even more striking with up to -42% of patients reporting a CID compared to just -18% of those treated with placebo (Figure 3B and Table 6).

**Table 6. Effect of MCIM compounds on clinical differences. BL= Baseline, CFB = change from baseline, MCID = minimum clinically important difference, CID = clinically important difference. * indicates statistical significance, p<0.05 (Chi² test).**

| Table 6 | | | | |
|---|---|---|---|---|
| % patients at week 12 | MCID: ≥-0.22 | CID: ≥-0.68 | Remission (≤0.5) | Normative (≤0.25) |
| Placebo | 51.9 | 19.9 | 15.7 | 6.9 |
| 5 mg | 78.2 | 33.4 | 22.6 | 14.3 |
| 20 mg | 59.2 | 24.5 | 14.5 | 8.9 |
| 40 mg | 67.9 | 39.7 | 28.8 | 18.2 |

The HAQ-DI method includes questions related to whether a patient has difficulty in performing a range of everyday activities, the answer to which are indicative of the level of functional disability experienced by a patient. While this methodology can be used to assess functional disability specifically in RA patients, the scoring method can also be used to measure functional disability in other patient groups, such as patients with age-related functional decline/disability. The questions in the HAQ-DI questionnaire include the following:
Are you able to shampoo your hair? Are you able to stand up from a chair? Are you able to get in and out of bed? Are you able to cut your meat? Are you able to lift a full cup or glass to your mouth? Are you able to walk outdoors on flat ground? Are you able to climb up five steps? Are you able to take a tub bath? Are you able to reach and get down a 5 pound object (such as a bag of sugar) from just above your head? Are you able to bend down to pick up clothing from the floor? Are you able to open car doors? Are you able to do chores such as vacuuming or yard work?

Analysing these individual disability components of the HAQ-DI reveals that treatment with HMC-C-01-A significantly improves key measures for indicating an improvement in patient quality of life. Figure 4 shows that (in the full study analysis set), following a 12-week course of treatment with 40 mg/day HMC-C-01-A, an increase in patients reporting no problems with DG2 and AC3 by -15% compared to placebo, AR1, EA2, WA2 by -20%, AR2, EA1 WA1 and RE2 by -30%, GR1 by -25%. Moreover, the data in Figure 5 shows that more patients following treatment with HMC-C-01-A compared to placebo experienced no pain at all associated with dressing, arising, eating, walking, hygiene, reach and activities. The reduction in pain experienced is particularly pronounced in respect to arising, walking and hygiene. Together these data clearly shows that following treatment with HMC-C-01-A, a significant proportion of patients experience a reversal in functional decline and experience less pain associated with daily activities which taken together clearly indicate an increase in quality-of-life.

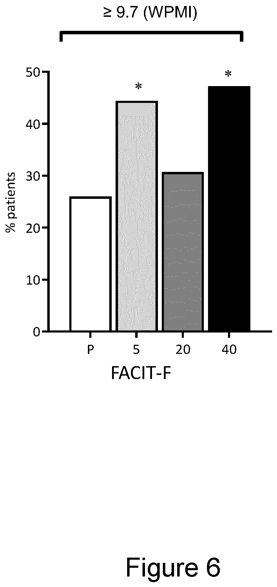

The above results were also reflected when patients were assessed using the patient global assessment of health (PGAH) and Patient global assessment of disease (PGAD) scoring methods in the full analysis set. PGAH and PGAD show an improvement in the proportion of patients achieving the MCID of a reduction of ≥-9.1 (Figure 6 and Table 7).

**Table 7. Effect of MCIM compounds on global health assessments. MCID = minimum clinically important difference, PGAH = patient global assessment of health, PGAD = patient global assessment of disease. Data shown as responders in the full analysis set with missing data treated as non-responders.**

| Table 7 | | |
|---|---|---|
| MCID: ≥-9.1 wk 12 | PGAH | PGAD |
| Placebo | 49.2 | 51.7 |
| 5 mg | 60.7 | 56.7 |
| 20 mg | 58.9 | 63.3 |
| 40 mg | 67.9* | 65.0* |

### EXAMPLE 4

Fatigue is common to all rheumatic conditions, and is a consistent feature of ageing. It occurs in varying degrees and can present a serious problem that has a major impact on patients' lives. FACIT-F is a commonly used clinical scoring system to measure fatigue and can be carried out as described in Hewlett et al (2011) which is hereby incorporated in its entirety by reference. The FACIT-F covers physical fatigue (e.g. I feel tired), functional fatigue (e.g. trouble finishing things), emotional fatigue (e.g. frustration) and social consequences of fatigue (e.g. limits social activity). The FACIT Fatigue Scale consists of 13 measures, that evaluate an individual's level of fatigue during their usual daily activities over the past week. The level of fatigue is measured on a four-point Likert scale (4 = not at all fatigued to 0 = very much fatigued). Subscale scores are calculated by first reversing negatively stated-items (subtracting the response from '4' ) and then summing the raw (0-4) scores. A total score is then derived by summing subscale scores. A higher score is better than a lower score.

The data are presented as the mean value for the group, and the mean change from the baseline value. A greater increase from baseline reflects a greater impact of treatment. Responder analysis to assess the minimum clinically important difference (CFB >4) and the within patient meaningful improvement (>9.7) are also applied to assess the depth of responses.

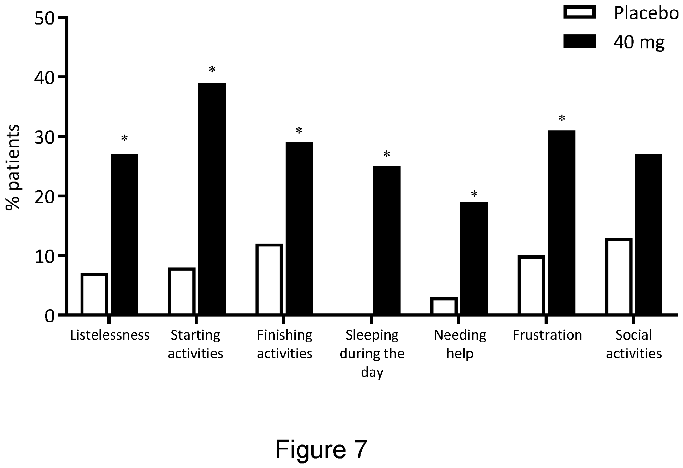

FACIT-F scores show a clear reduction in patients with severe fatigue following MCIM treatment. In the proportion of patients achieving the "within-patient meaningful improvement" (WPMI), strong effects on weakness, listlessness, tiredness, starting activities and frustration with tiredness was observed, indicative of an increase in quality of life (Figures 6, 7and Table 8). Looking at individual components of FACIT-F scores reveals particularly notable improvements in patient listlessness, ability to start activities, finishing activities, reduced sleeping during the day, reduced need for help, reduced frustration and increased participation in social activities, with an increase in patients treated with HMC-C-01-A achieving a score of over >20 (indicating reduction in severe fatigue) compared to placebo (Figure 7).

**Table 8. Effect of MCIM compounds on weakness, listlessness, tiredness, starting activities and frustration with tiredness. BL= Baseline, CFB = change from baseline, MCID = minimum clinically important difference, WPMI = within-patient meaningful improvement. * p<0.05. ** p<0.01, Chi2 test, full analysis set, missing data assumed as non-responder.**

| Table 8 | | | | | | | | |
|---|---|---|---|---|---|---|---|---|
| | Placebo | | 5 mg | | 20 mg | | 40 mg | |
| WPMI >9.7 | 63.9 | | 66.7 | | 59.2 | | 71.7 | |
| | BL | CFB | BL | CFB | BL | CFB | BL | CFB |
| Reduction in severe fatigue (≤20) | 22 | -10 | 22 | -18* | 21 | -7* | 32 | -21* |
| No problems | | | | | | | | |
| I feel fatigued | 5 | 15 | 2 | 7 | 0 | 6 | 2 | 14 |
| I feel weak all over | 10 | 12 | 3 | 17* | 3 | 6 | 2 | 19* |
| I feel listless | 25 | 8 | 12 | 22** | 15 | 5 | 19 | 19* |
| I feel tired | 10 | 3 | 2 | 10* | 3 | 6 | 2 | 10 |
| I have trouble starting things because I am tired | 17 | 10 | 10 | 22** | 2 | 16* | 7 | 22** |
| I have trouble finishing things because I am tired | 18 | 5 | 15 | 18* | 3 | 10 | 3 | 24* |
| I have energy | 12 | -2 | 12 | 3 | 3 | 2 | 12 | -5 |
| I am able to do my usual activities | 3 | 5 | 8 | -3 | 2 | 3 | 12 | -7 |
| I need to sleep during the day | 32 | 3 | 25 | 10 | 23 | 3 | 24 | 7* |
| I am too tired to eat | 52 | 10 | 43 | 23 | 50 | 6 | 49 | 10 |
| I need help doing my usual activities | 27 | 3 | 22 | 27 | 21 | 6 | 19 | 19 |
| I am frustrated by being too tired to do the things I want to do | 35 | 0 | 27 | 12 | 21 | 13 | 17 | 25** |
| I have to limit my social activity because I am tired | 17 | 15 | 20 | 27 | 15 | 15 | 15 | 22 |

### EXAMPLE 5

The Rheumatoid Arthritis Impact of Disease (RAID) score is a EULAR-initiated PROM developed in collaboration with patients with RA. It is a global composite measure of the impact of RA that takes into account the seven domains of impact of RA considered of utmost relevance by patients: pain, physical disability, fatigue, sleep disturbances, coping as well as physical and emotional well-being. Each domain is evaluated by patients using a single 0 (no symptom) to 10 numeric rating scale (NRS) and has a specific weight used to calculate a single composite RAID score using an online calculator (https://rheumcalc.com/raid/). The EULAR-RAID score was calculated for each subject at each visit. The data are presented as the mean value for the group, and the mean change in patients achieving disease thresholds reflecting different disease activity levels. Responder analysis to assess patients experiencing no problems (score of <2) are also applied to assess the depth of responses.

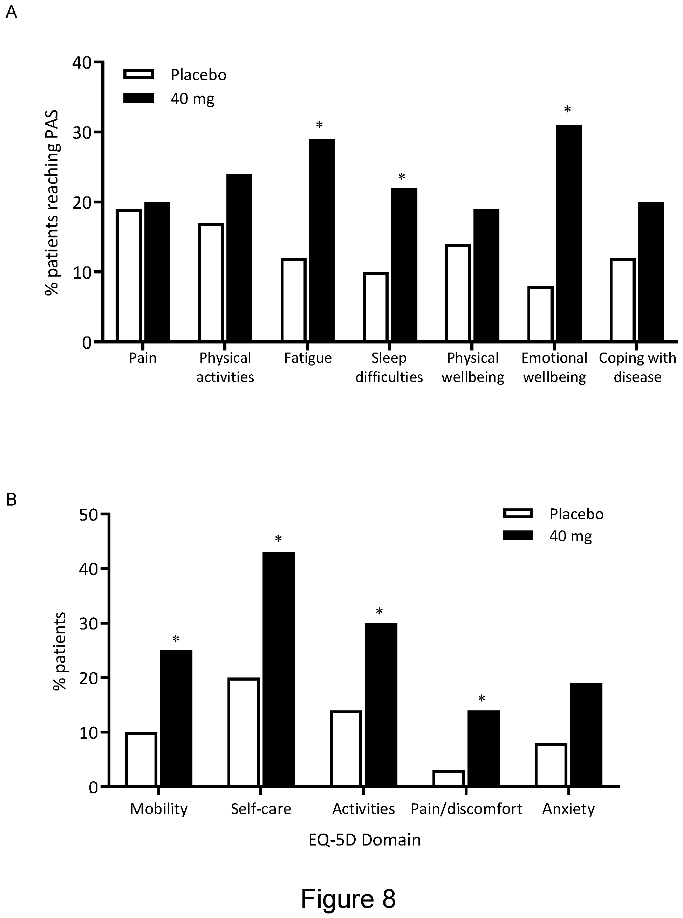

Patients treated with HMC-C-01-A show a clear improvement in all components of the EULAR-RAID scoring system (with the exception of pain), with particularly strong improvements in function, fatigue, sleep and emotional wellbeing, and a clear reduction in patients with a severe RAID score (Figure 8A and Table 9).

**Table 9. Effect of MCIM compounds on patients reporting a score of ≤2 on components of the EULAR-RAID score system.. * p<0.05, Chi2 test, full analysis set, missing data assumed as non-responder.**

| Table 9 | | | | |
|---|---|---|---|---|
| Patients achieving score of <2 | Placebo | 5 mg | 20 mg | 40 mg |
| | CFB | BL | CFB | BL |
| mild | 11.5 | 16.6 | 18.9 | 23.1* |
| moderate | 23.5 | 31.4 | 26.2 | 36.5* |
| severe | 16.9 | 23.3** | 16.1 | 26.8* |
| Pain | 18.6 | 15.0 | 8.1 | 20.3 |
| Physical activities | 16.9 | 28.3 | 6.6 | 23.7 |
| Fatigue | 11.9 | 25.0* | 9.8 | 28.8* |
| Sleep difficulties | 10.2 | 21.7* | 13.1 | 22.0* |
| Physical wellbeing | 13.6 | 21.7 | 6.6 | 18.6 |
| Emotional wellbeing | 8.5 | 26.7 | 3.3 | 30.5 |
| Coping | 11.9 | 35.0* | 23.0* | 20.3 |

Patients also show clear improvements in EQ-5D scoring criteria, with treatment eliciting a relatively large number of patients reporting no problems on all subscale dimensions compared to patients receiving placebo (Figure 8B and Table 10).

**Table 10. Effect of MCIM compounds on EQ-5D scoring criteria. BL= Baseline, CFB = change from baseline. * indicates p<0.1 , ** indicates p<0.05, Chi² test, modified full analysis set.**

| Table 10 | | | | |
|---|---|---|---|---|
| % increase in patients achieving no problems (week 12 -BL) | Placebo | 5 mg | 20 mg | 40 mg |
| | CFB | BL | CFB | BL |
| Mobility | 11.5 | 16.6 | 18.9 | 23.1 * |
| Self-care | 23.5 | 31.4 | 26.2 | 36.5* |
| Usual activities | 16.9 | 23.3** | 16.1 | 26.8* |
| Pain/Discomfort | 4.1 | 9.3 | 2.5 | 15.1 * |
| Anxiety/depression | 11.7 | 24.1 ** | 18.7 | 16.1 |

Generally, in the above trials, patients treated with either 5 mg or 40 mg HMC-C-01-A outperformed patients treated with 20 mg HMC-C-01-A in many of the patient reported outcomes. This may be explained by the observation of several patients within the 20 mg patient group experiencing an increase in pain, with a consequent impact on quality of life and function early on in the treatment course, at approximately week 4. Whilst this recovered to week 8 and week 12, this carried a consequent impact in the later patient responses.

### EXAMPLE 6

Following the 12 week course of treatment, erosion progression and/or repair of joint architecture was assessed. In the placebo treated cohort, approximately 50 percent of patients showed no progression of erosion over the 12-week period (Figure 9, "All"). This increased to approximately 65% of patients showing no erosion progression in cohorts treated with 5 or 20 mg/day HMC-C-01-A, and up to approximately 72% in patients treated with 40 mg/day HMC-C-01-A. Treatment with glucocorticoids or non-steroidal anti-inflammatory drugs (NSAIDs) can suppress normal bone repair (Chotiyarnwong, P., and McCloskey, E., 2020 and Pountos et al., 2021) and thus mask the potential to evaluate the effect of HMC-C-01-A and MCIM compounds on the bone. To avoid this potential masking effect, the full trial analysis set data were analysed according to steroid or NSAID use. In patients who did not receive co-treatment with steroids/glucocorticoids, treatment with HMC-C-01-A increased the proportion of patients who showed no progression of bone erosion at week 12 to 58-67% compared with placebo (35%), whilst in patients who did not report taking NSAIDs, treatment with HMC-C-01-A increased the proportion of patients who showed no progression of bone erosion at week 12 to 50-68% compared with placebo (0%). The presence of subclinical bone inflammation (osteitis) can predict a more erosive disease time course (Burgers et al, 2018). To evaluate how the presence of this type of inflammation impacts the ability of HMC-C-01-A and MCIM to arrest progression of erosive damage, the full trial analysis set data were analysed according to the presence or absence of osteitis. In patients who showed the presence of bone erosions with concurrent osteitis at baseline, treatment with HMC-C-01-A increased the proportion of patients who showed no progression of bone erosion at week 12 to 52-57% compared with placebo (35%).

Given the potential of co-treatment with steroids or NSAIDs to inhibit bone repair, the same subgroups (i.e. patients not receiving co-treatment with steroid, or taking NSAIDs) were evaluated for responders who showed reversal/repair of bone erosion. As seen in Figure 9, treatment with HMC-C-01-A increased the proportion of patients showing reversal/repair of their erosions compared with placebo in groups not receiving steroid or NSAID. In addition, treatment with HMC-C-01-A increased the proportion of patients who showed reduction irrespective of the presence or absence of osteitis; in the absence of osteitis at baseline, HMC-C-01-A increased the proportion of patients who showed reduction of bone erosion at week 12 to 15-22% compared with placebo (11%) whilst in the presence of osteitis at baseline, HMC-C-01-A increased the proportion of patients who showed reduction of bone erosion at week 12 to 20-23% compared with placebo (16%). This reversal/repair is a significant finding as no current treatments offer the prospect of inducing tissue repair in arthritis such as RA, or age-related bone degeneration. Thus, treatment with MCIM compounds are the first compounds to demonstrate reversal of existing pathological damage.

Patients treated with the MCIM, HMC-C-01A, show a different profile of efficacy and pattern of response to patients who are currently treated with DMARDs. The current DMARDs elicit a change in tender and swollen joint scores and pain, accompanied by a short-term improvement in function assessed by the HAQ-DI. Impacts on structural progression follow control of joint swelling, and pain control is associated with the reduction in these joint scores. However, several lines of evidence indicate that structural progression and functional loss continue despite the use of conventional DMARDs, which are effective in controlling joint swelling and/or tenderness and pain, resulting in a phenomenon known as silent progression (Sewerin et al., 2017). For example, Welsing at al (2001) showed that after an initial reduction in the extent of disease activity, the mean DAS remained more or less stable over the course of the disease whilst the functional capacity of the patients, as measured by the HAQ DI, worsened over the course of the disease after an initial improvement. Similar results have also been observed for structural progression (Emery et al., 2008 and Sewerin et al., 2017). In addition, it has been shown that even patients who achieve low disease activity rather than disease remission have worse structural and functional outcomes (Ruyssen-Witbrand et al., 2023). This suggests that additional pathological features of the disease may be involved in driving functional decline in patients with RA. However, no therapy has, to date, shown an ability to improve physical function and improve health-related quality of life absent control of joint tenderness/swelling and pain. For example, whilst the RANKL inhibitor, denosumab, was able to reduce structural progression it had no impact on disability suggesting that the relationship between disability and bone erosion is more complex than simple infilling of erosions or increasing bone mineral density. The results from this study are unexpected in the light of the considerable body of evidence that exists linking control of joint swelling/tenderness to effective treatment of disease, and identifies an alternative approach to improving disability and quality of life in RA, by establishing a structural and inflammatory control at the level of the bone pathology.

### EXAMPLE 7

MCIM compounds can affect mitochondrial morphology and modulate cellular metabolism as discussed in PCT/EP2024/057086, which is hereby incorporated by reference in its entirety. It was investigated whether the reparative properties of the MCIM compounds could be attributed to binding to/modulation of mitochondrial proteins and/or complexes. To this end, MCIM compounds were assessed using a cellular thermal shift assay (CETSA) coupled with quantitative mass spectroscopy (MS) to determine what pathways are modulated by the MCIM compounds.

Thp-1 cells were incubated in the presence of 2 µM MCIM compound (ABD900) or DMSO (vehicle control) for 4 hours. Following the incubation period, samples were heated to one of the following temperatures: 40.0, 42.9, 46.0, 49.6, 53.2, 56.8, 60.8, 64.0, 67.1, 70.0°C. Each test condition was performed in duplicate.

Following heating, cells were lysed, and the samples digested with trypsin. The digested soluble peptide fractions corresponding to individual temperatures were then labelled with a different isobaric tag, using the TMT10plex system as described in Bantscheff, M., et al (2007), Bantscheff, M., et al (2012) and Franken, H., et al (2015), each of which are incorporated by reference in their entirety. Labelling of fractions from individual temperatures with an individual TMT1 OPlex tag allows the samples to be pooled and analysed by mass spectroscopy in a single run.

Following TMT10Plex labelling and pooling, the samples were fractionated using hydrophilic strong anion exchange (hSAX) (24 fractions per sample) and Liquid Chromatography with tandem mass spectrometry (LC-MS/MS) performed. The LC-MS/MS data was analysed to identify proteins whose thermal stability was shifted in the presence of the MCIM compound using the TPP R Package (Franken, H., et al (2015)) with a procedure described in Savitski., MM et al (2014). Generally, upon compound binding, proteins become more stable and thus more resistant to thermal denaturation. Therefore, a shift in thermal stability of a protein in the described CETSA assay can indicate direct binding of a compound to the protein with shifted thermal stability. Alternatively, a shift on thermal stability may indicate the protein is involved in a downstream event from the bound protein, for example, a post-translation modification as a result of an altered metabolic or signalling pathway.

Briefly, criteria for target candidate selection were as follows:
- Min p-value <0.4 (Benjamini-Hochberg corrected)
- DTm of Run 1 (R1) and Run 2 (R2) have same sign
- DTm (drug vs DMSO) > DTm (DMSO R1 vs DMSO R2)
- Minimum slope less than -0.06

Using the above criteria, 105 proteins were identified as showing a thermal shift when incubated with the MCIM compound indicating that they were either stabilised or destabilised. Of these 105 proteins, 73 were classified as high confidence and 32 as medium confidence hits.

Functional protein interactions and/or associations of the 105 identified candidate proteins were retrieved using the freely available STRING software (https://string-db.org). STRING analysis identified candidates involved in oxidative phosphorylation (e.g. NDUFA6 and SDHB), mitochondrial function and the ER to Golgi apparatus interface.

In addition, thermal shifts were observed in proteins involved in adaptive stress responses (e.g. YME1L1, OXSR1, MKNK1) and other proteins which play a role in NF-κB signalling (e.g. OXSR1, RASA1 and BIRC2).

It was therefore concluded, that incubation with MCIM compounds alters the metabolic activity of cells specifically through modulation of oxidative phosphorylation, as indicated by the thermal shift of NDUFA6 (a Respiratory Complex 1 component). It was hypothesised that such modulation of Complex 1 and the oxidative phosphorylation pathway would alter NPκB and adaptive response pathways in cells, as indicated by the thermal shift observed for proteins such as YMEL1L1, OXSR1, MKNK1, RASA1 and BIRC2.

### EXAMPLE 8

To investigate the binding partner(s) of the MCIM compounds, and the cellular activities modulated by them, Photoaffinity labelling (PAL) and quantitative stable isotope labelling by amino acids in cell culture (SILAC) was performed. A clickable linker probe MCIM compound was generated to do this.

This identified NDUFS2, a subunit of Mitochondrial Complex I, as a binder of the clickable linker probe MCIM compound. Together, the CETSA and PAL/SILAC-MS results strongly indicate that MCIM compounds can modulate oxidative phosphorylation and stress response pathways by binding and modulating the activity of the Mitochondrial Complex I.

### EXAMPLE 9

Given that both CETSA and PAL/SILAC-MS methods identified Complex I subunits as targets of MCIM compounds, an *in silico* computer modelling approach was used to determine the binding site of MCIM compounds with the Complex I subunits.

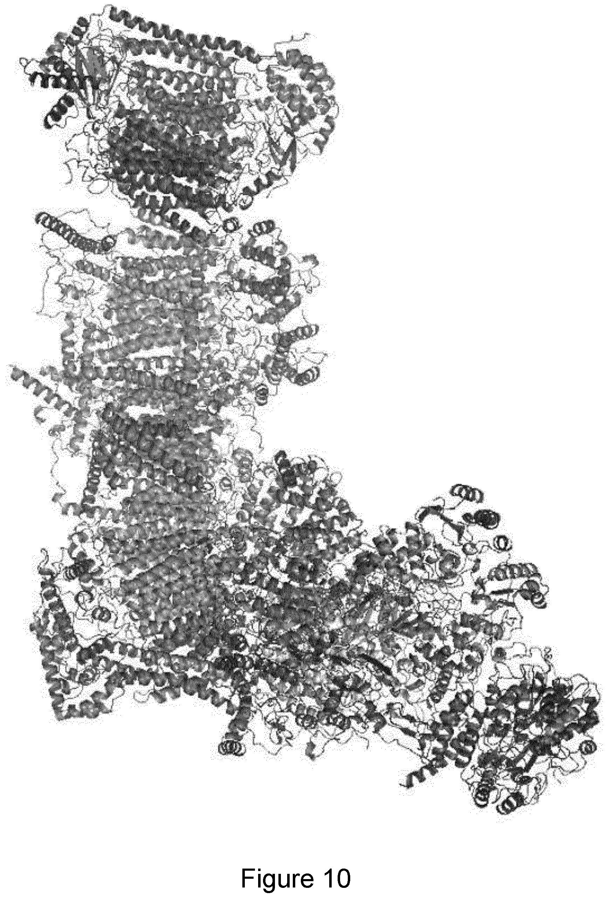

Initially, homology models were built from publicly available structures of mitochondrial Complex I from five organisms, including human (Table 11). The putative targets were fully resolve in 5 structures. Mitochondrial Complex 1 used for the following modelling approaches is show in Figure 10.

| **Table 11** | | | | | | | |
|---|---|---|---|---|---|---|---|
| Publicly available Complex I structures. FMN = Flavin mononucleotide. | | | | | | | |
| **Protein** | **Size** | **Type** | **Method** | **Liganded?** | | **Ref** | **Protein database ID** |
| | | | | Y/N | Site | | |
| Y. Lipolytica | 466 KD | Fungal | X-Ray | N | | Zickerman et al Science, 2015, 247, 44-49 | 4WZ7 |
| T. Thermophilus | | Bacterial | X-Ray | FMN | NADH-FMN | Efremov et al Nature, 2010, 465, 441-445 | 3M9S |
| T. Thermophilus | 536 KD | Bacterial | X-Ray | FMN, Y | NADH-FMN, Quinone | Sazanov et a Nature, 2013, 2013, 494, 443-450 | 4HEA |
| T. Thermophilus | 570 KD | Bacterial | X-Ray | NADH, FMN | NAD-FMN | Sazanov et al JBC, 2009, 284, 29773 | 3IAM |
| Bos Taurus | 517 KD | Mammalian | Cryo-EM | N | | Hirst et al Nature, 2014, 515, 80-84 | 4UQ8 |
| Bos Taurus | 891 KD | Mammalian | Cryo-EM | NADH, FMN | NADH-FMN | Hirst et al Nature, 2016, 536, 354 | 5LDW, 5LDX, 5LC5 |
| Ovis Aries | 978 KD | Mammalian | Cryo-EM | N | | Sazanov et al Nature, 2016, 538, 406 | 5LNK |
| Ovis Aries | 1.28 MD | Mammalian | Cryo-EM | N | | Sazanov et al Nature, 2016, 537, 644 | 5J4Z, 5J7Y, 5J8K |
| Homo Sapiens | | Mammalian | Cryo-EM | NADP, FMN | NADH-FMN | Guo et al Cell, 2017, 170, 1247-1257 | 5XTD, 5XTH, 5XTI |

The homology modelling revealed a lid pocket in the NDUFS2 subunit of Complex I which is in contact with the Q-tunnel. In four of the models the lid pocket was seen to be in an "open" conformation while in the remaining model it was in a "closed" conformation.

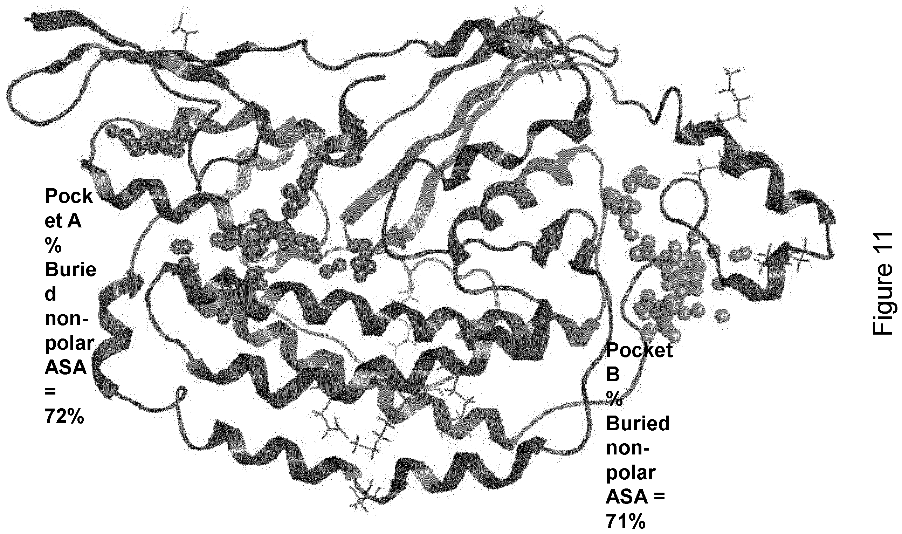

A homology model of the "open" confirmation was constructed and then SiteFinder was used to map NDUFS2 in the open and closed conformations. In particular, SiteFinder (Halgren T. A., 2009) was used to build a model of the "drugability" of NDUFS2, as measured by the volume of buried non-polar available surface area (ASA; Figure 11). This model identified two binding sites on NDUFS2. The first binding site ("Pocket A") is located on the lid pocket in the open conformation and is represented on Figure 11 by a cluster of spheres. Pocket A has a percentage buried non-polar available surface area of 72%. The second binding site ("Pocket B") is located at the "back" of the mitochondrial Complex I, relative to the position of the mitochondrial Complex I subunits NDUFS7 and ND1 and is represented by a second cluster of spheres. Pocket B has a percentage buried non-polar available surface area of 71%.

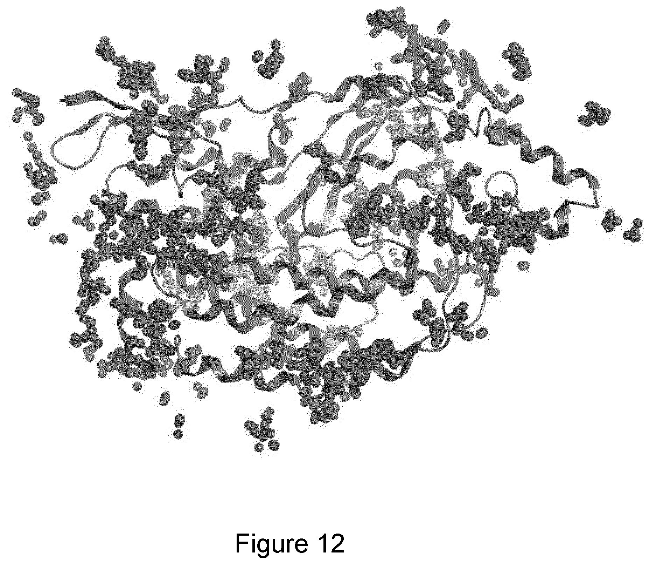

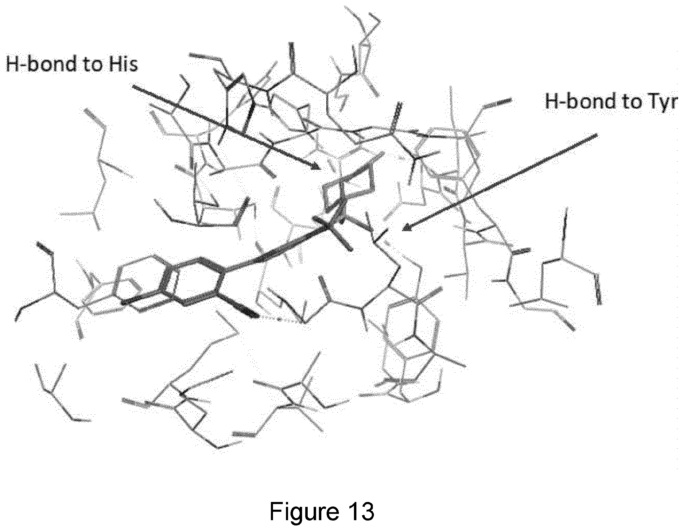

Further modelling of Q10, the natural ligand of the Q-tunnel, within the Q-site of Complex 1 using SiteFinder indicated that the space for binding in this pocket is limited (Figure 12). The spheres in Figure 12 illustrate the space and channels around NDUFS2 when NDUFS2 is associated with Complex 1. This modelling approach also revealed a further ligand binding site at the junction between NDUFS2 and NDUFS7 (Figure 13). In particular, the MCIM compound is predicted to interact with His38 and Tyr141 of NDUFS2.

Using these protein models it was determined that the optimal binding site of each of ABD900, HMC-C-01-A and HMC-N-01-A was the NDUFS2 lid pocket (as illustrated in Figure 11, "Pocket A"), specifically the lid pocket in the open conformation (Glide score ~6), which is close to the Q-site. Even more particularly, the inventors were able to predict which amino acid residues in NDUFS2 contributed to the binding of the compounds:
- H-bonding with backbone carbonyl of Gly85, backbone amine of Leu95 and carboxylic acid of Asp193
- H-bonding to Tyr141 and His38
- π- π stacking with Phe458 and His88

In conclusion, analysis of the Q-site of NDUFS2 indicates that this site needs to be in the open confirmation to accommodate Q10 or small drug-like compounds.

### EXAMPLE 10

As discussed in Example 9, is has been determined that the optimal binding site for MCIM compounds is in the lid pocket of NDUFS2 which is in close proximity to the Q-tunnel. From the data in Example 9, a ligand-protein pharmacophore model was built which identified 9 pharmacophoric features (Figure 14 and Tables 12A to12C). Table 12-A describes the relationship between the type of pharmacophoric feature and the permissible variation in 3D space for the location of the given pharmacophoric feature. Table 12-B shows a distance matrix describing the 3D relationship between the centre of each pharmacophoric feature. Table 12-C describes the angle between each triplet combination of pharmacophoric features, wherein column "Y" describes the vertex of each angle. Using the parameters described in Tables 12-A to 12-C a visual representation of the pharmacophore model has been produced using the unified annotation scheme in *Molecular Operating Environment (MOE)* software tool (Figure 14). Using *Molecular Operating Environment (MOE),* the parameters can also be used to determine if a molecule conforms to the pharmacophore model - i.e. if 4 or more of the annotation points of the pharmacophore model are occupied by a corresponding annotation point located on a test molecule.

| **Table 12-A** | | |
|---|---|---|
| Pharmacophore feature annotations, and radii | | |
| *Feature Number* | *Feature Annotation* | *Radius (Å)* |
| F1 | Acc | 1.2 |
| F2 | Acc | 1.2 |
| F3 | Acc | 1.2 |
| F4 | Acc2 | 1.4 |
| F5 | Aro | 1.4 |
| F6 | Hyd | 1.4 |
| F7 | Hyd | 1.4 |
| F8 | Hyd | 1.4 |
| F9 | Don2 | 1.4 |

| **Table 12-B** | | | | | | | | | | |
|---|---|---|---|---|---|---|---|---|---|---|
| Pharmacophore feature distances (Å) | | | | | | | | | | |
| *Dist. (Å)* | *Feature Number* | *F1* | *F2* | *F3* | *F4* | *F5* | *F6* | *F7* | *F8* | *F9* |
| *Feature Number* | *Feature Ann* | Acc | Acc | Acc | Acc2 | Aro | Hyd | Hyd | Hyd | Don2 |
| *F1* | Acc | 0.00 | 2.64 | 8.50 | 8.16 | 8.11 | 4.07 | 4.77 | 3.13 | 4.82 |
| *F2* | Acc | 2.64 | 0.00 | 7.21 | 9.80 | 8.48 | 4.60 | 3.85 | 3.85 | 3.46 |
| *F3* | Acc | 8.50 | 7.21 | 0.00 | 16.55 | 14.19 | 10.57 | 4.37 | 10.95 | 9.58 |
| *F4* | Acc2 | 8.16 | 9.80 | 16.55 | 0.00 | 6.76 | 6.87 | 12.41 | 6.05 | 8.88 |
| *F5* | Aro | 8.11 | 8.48 | 14.19 | 6.76 | 0.00 | 4.12 | 9.96 | 6.41 | 5.82 |
| *F6* | Hyd | 4.07 | 4.60 | 10.57 | 6.87 | 4.12 | 0.00 | 6.26 | 3.31 | 3.22 |
| *F7* | Hyd | 4.77 | 3.85 | 4.37 | 12.41 | 9.96 | 6.26 | 0.00 | 7.12 | 5.90 |
| *F8* | Hyd | 3.13 | 3.85 | 10.95 | 6.05 | 6.41 | 3.31 | 7.12 | 0.00 | 3.77 |
| *F9* | Don2 | 4.82 | 3.46 | 9.58 | 8.88 | 5.82 | 3.22 | 5.90 | 3.77 | 0.00 |

| **Table 12-C** | | | |
|---|---|---|---|
| Pharmacophore feature angles between features X-Y-Z (°), | | | |
| *X* | *Y* | *Z* | *Angle (°)* |
| F1 | F2 | F3 | 110.39 |
| F1 | F2 | F4 | 45.38 |
| F1 | F2 | F5 | 72.94 |
| F1 | F2 | F6 | 61.46 |
| F1 | F2 | F7 | 92.58 |
| F1 | F2 | F8 | 53.87 |
| F1 | F2 | F9 | 103.51 |
| F1 | F3 | F4 | 6.61 |
| F1 | F3 | F5 | 30.48 |
| F1 | F3 | F6 | 21.30 |
| F1 | F3 | F7 | 22.60 |
| F1 | F3 | F8 | 11.59 |
| F1 | F3 | F9 | 30.18 |
| F1 | F4 | F5 | 65.03 |
| F1 | F4 | F6 | 29.85 |
| F1 | F4 | F7 | 12.32 |
| F1 | F4 | F8 | 18.96 |
| F1 | F4 | F9 | 32.52 |
| F1 | F5 | F6 | 7.99 |
| F1 | F5 | F7 | 28.34 |
| F1 | F5 | F8 | 21.01 |
| F1 | F5 | F9 | 35.98 |
| F1 | F6 | F7 | 49.67 |
| F1 | F6 | F8 | 48.90 |
| F1 | F6 | F9 | 81.99 |
| F1 | F7 | F8 | 20.48 |
| F1 | F7 | F9 | 52.40 |
| F1 | F8 | F9 | 88.17 |

| **Table 12-C** | | | |
|---|---|---|---|
| Pharmacophore feature angles between features X-Y-Z (°), | | | |
| *X* | *Y* | *Z* | *Angle (°)* |
| F2 | F3 | F4 | 15.65 |
| F2 | F3 | F5 | 27.53 |
| F2 | F3 | F6 | 20.78 |
| F2 | F3 | F7 | 26.84 |
| F2 | F3 | F8 | 5.82 |
| F2 | F3 | F9 | 17.47 |
| F2 | F4 | F5 | 58.21 |
| F2 | F4 | F6 | 24.99 |
| F2 | F4 | F7 | 14.75 |
| F2 | F4 | F8 | 6.62 |
| F2 | F4 | F9 | 20.61 |
| F2 | F5 | F6 | 14.35 |
| F2 | F5 | F7 | 22.35 |
| F2 | F5 | F8 | 25.41 |
| F2 | F5 | F9 | 18.14 |
| F2 | F6 | F7 | 37.84 |
| F2 | F6 | F8 | 55.34 |
| F2 | F6 | F9 | 48.68 |
| F2 | F7 | F8 | 22.45 |
| F2 | F7 | F9 | 34.01 |
| F2 | F8 | F9 | 54.05 |
| F3 | F4 | F5 | 58.15 |
| F3 | F4 | F6 | 22.97 |
| F3 | F4 | F7 | 5.71 |
| F3 | F4 | F8 | 18.05 |
| F3 | F4 | F9 | 27.40 |
| F3 | F5 | F6 | 24.17 |
| F3 | F5 | F7 | 5.20 |

| **Table 12-C** | | | |
|---|---|---|---|
| Pharmacophore feature angles between features X-Y-Z (°), | | | |
| *X* | *Y* | *Z* | *Angle (°)* |
| F3 | F5 | F8 | 47.65 |
| F3 | F5 | F9 | 29.69 |
| F3 | F6 | F7 | 5.20 |
| F3 | F6 | F8 | 87.80 |
| F3 | F6 | F9 | 63.56 |
| F3 | F7 | F8 | 143.73 |
| F3 | F7 | F9 | 137.11 |
| F3 | F8 | F9 | 59.09 |
| F4 | F5 | F6 | 73.80 |
| F4 | F5 | F7 | 93.94 |
| F4 | F5 | F8 | 54.64 |
| F4 | F5 | F9 | 89.42 |
| F4 | F6 | F7 | 142.01 |
| F4 | F6 | F8 | 61.79 |
| F4 | F6 | F9 | 118.85 |
| F4 | F7 | F8 | 17.94 |
| F4 | F7 | F9 | 41.28 |
| F4 | F8 | F9 | 127.89 |
| F5 | F6 | F7 | 146.38 |
| F5 | F6 | F8 | 118.68 |
| F5 | F6 | F9 | 104.33 |
| F5 | F7 | F8 | 39.90 |
| F5 | F7 | F9 | 31.63 |
| F5 | F8 | F9 | 63.74 |
| F6 | F7 | F8 | 27.72 |
| F6 | F7 | F9 | 30.48 |
| F6 | F8 | F9 | 53.57 |
| F7 | F8 | F9 | 55.96 |

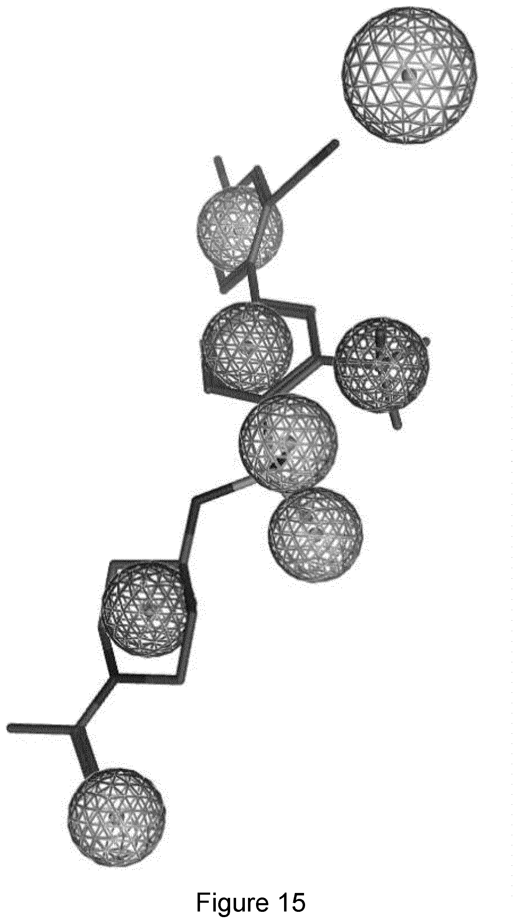

Using the Molecular Operating Environment (MOE), 2022.02 Chemical Computing Group ULC, 1010 Sherbooke St. West, Suite #910, Montreal, QC, Canada, H3A 2R7, 2022 MOE docking using the pharmacophore algorithm for ligand placement and the GBVI/WSA dG scoring function, 117 compounds were assessed for their ability to dock in the predicted binding site using the pharmacophore model described above. To be treated as a successful docking in the predicted binding site, it is required that a molecule hits at least 4 features of the pharmacophore model shown in Figure 15 and has a half-maximal inhibitory concentration (IC₅₀) ≤ 1 µM.

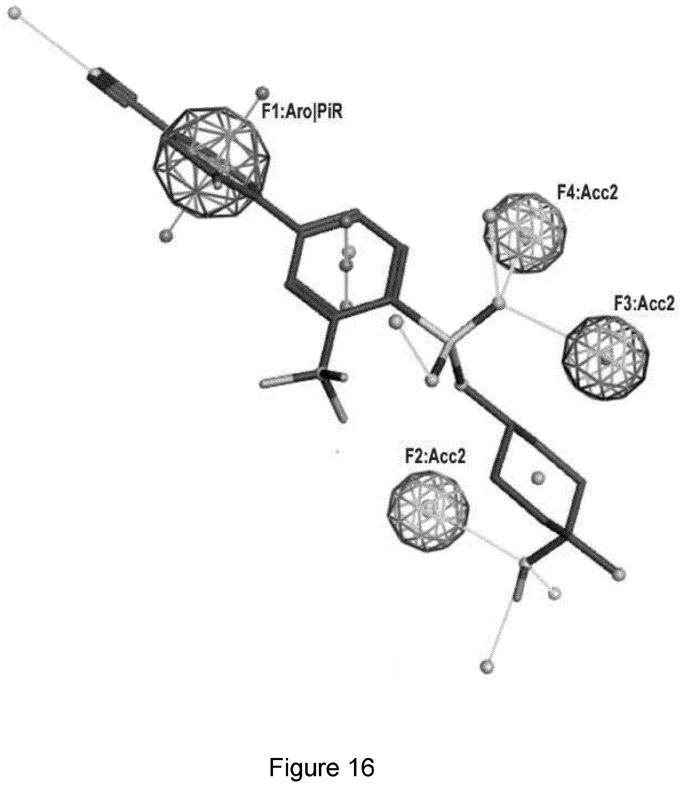

Figure 15 shows a MCIM compound that conforms to the pharmacophore model and satisfies 7 out of the 9 annotation points determined to be important for binding to Complex I. Surprisingly, out of the 117 compounds assessed for their ability to bind in the pharmacophore model, only 13 compounds had an IC₅₀ > 1 µM, which indicates that these compounds would fail to dock in the predicted binding sites. The remaining 104 compounds hit at least 4 of the pharmacophore features and are predicted to have an IC₅₀ ≤ 1 µM. Figure 16 shows representative compound CHMSA-02-A, which fulfils the pharmacophore model and is predicted to have a pAct (-Log(IC₅₀) of ~7.

### EXAMPLE 11

Following building of the pharmacophore model, the inventors next wanted to identify additional NDUFS2 binders using this model.

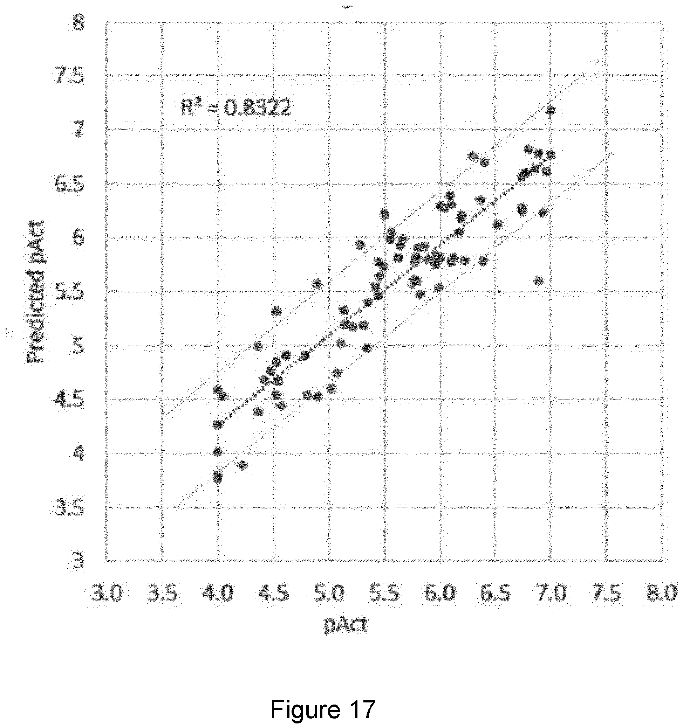

The 3D model of the entire Complex 1 described in Example 9 (Figure 10) was validated by docking Q10 and active MCIM compounds. This allowed establishment of the bioactive conformation of the MCIM compounds when docked in the Q-tunnel of Complex I. This further allowed a structure-based hypothesis to be generated and to rationalise the structure-activity relationship (SAR) of the compounds and to build the pharmacophore model described in Example 10 and a QSAR model (Figure 17) for activity prediction. The QSAR model is a linear regression model which includes docking scores and parameters related to ligand energies and electrostatics.

For uHTVS, a library of compounds was screened against the pharmacophore model described in Example 13. The model identified 37.6 million compounds from the library of compounds which generally hit 3-6 of the pharmacophore features. Of these 37.6 million compounds, those up to a molecular weight of -350 Da were then virtually docked in the 3D Complex I model without imposing pharmacophore constraints. 67,000 compounds were predicted to dock in Complex I and were retained for further screening.

The retained compounds were then filtered through a more theoretically rigorous docking method and evaluated with a QSAR model to predict biological activity of each compound (Figure 17). The GBVI/WSA ΔG forcefield-based scoring function (Naim *et al.* 2007) on the MOE dock (Molecular Operating Environment (MOE), 2022.02 Chemical Computing Group ULC, 1010 Sherbooke St. West, Suite #910, Montreal, QC, Canada, H3A 2R7) was used. Using the screening process described, 756 compounds were identified as potential drug candidates for targeting NDUFS2/NDUFS7 of Complex 1.

### References

A number of publications are cited above in order to more fully describe and disclose the invention and the state of the art to which the invention pertains. Full citations for these references are provided below. The entirety of each of these references is incorporated herein.
Albert Sanchez-Niubo, Carlos G Forero, Yu-Tzu Wu, lago Giné-Vázquez, Matthew Prina, Javier De La Fuente, Christina Daskalopoulou, Elena Critselis, Alejandro De La Torre-Luque, Demosthenes Panagiotakos, Holger Arndt, José Luis Ayuso-Mateos, Ivet Bayes-Marin, Jerome Bickenbach, Martin Bobak, Francisco Félix Caballero, Somnath Chatterji, Laia Egea-Cortés, Esther García-Esquinas, Matilde Leonardi, Seppo Koskinen, Ilona Koupil, Blanca Mellor-Marsá, Beatriz Olaya, Andrzej Pajak, Martin Prince, Alberto Raggi, Fernando Rodríguez-Artalejo, Warren Sanderson, Sergei Scherbov, Abdonas Tamosiunas, Beata Tobias-Adamczyk, Stefanos Tyrovolas, Josep Maria Haro, the ATHLOS Consortium, Development of a common scale for measuring healthy ageing across the world: results from the ATHLOS consortium, International Journal of Epidemiology, Volume 50, Issue 3, June 2021, Pages 880-892, https://doi.org/10.1093/ije/dyaa236
Aletaha D, Smolen JS. Diagnosis and Management of Rheumatoid Arthritis: A Review. JAMA. 2018 Oct 2;320(13):1360-1372. doi: 10.1001/jama.2018.13103. PMID: 30285183.
Allanore Y, Bozzi S, Terlinden A, Huscher D, Amand C, Soubrane C, Siegert E, Czirják L, Carreira PE, Hachulla E, Zanatta E, Li M, Airò P, Mendoza FA, Rosato E, Distler O; EUSTAR Collaborators. Health Assessment Questionnaire-Disability Index (HAQ-DI) use in modelling disease progression in diffuse cutaneous systemic sclerosis: an analysis from the EUSTAR database. Arthritis Res Ther. 2020 Oct 28;22(1):257. doi: 10.1186/s13075-020-02329-2. PMID: 33115544; PMCID: PMC7592571.
Almutairi K, Nossent J, Preen D, Keen H, Inderjeeth C. The global prevalence of rheumatoid arthritis: a meta-analysis based on a systematic review. Rheumatol Int. 2021 May;41(5):863-877. doi: 10.1007/s00296-020-04731-0. Epub 2020 Nov 11. PMID: 33175207.
Altintas D, Melikoglu MA. The frequency of fibromyalgia in familial Mediterranean fever and its impact on the quality of life. Int J Rheum Dis. 2022 Oct;25(10):1123-1128. doi: 10.1111/1756-185X.14389. Epub 2022 Jul 18. PMID: 35851993.
Alvarez-Hernández E, Peláez-Ballestas I, Vázquez-Mellado J, Terán-Estrada L, Bernard-Medina AG, Espinoza J, Aceves-Avila FJ, Goycochea-Robles MV, Garza M, Ventura L, Burgos-Vargas R;
REUMAIMPACT. Validation of the Health Assessment Questionnaire disability index in patients with gout. Arthritis Rheum. 2008 May 15;59(5):665-9. doi: 10.1002/art.23575. PMID: 18438898.
Beaton, D et al (2021) The OMERACT Handbook for establishing and implementing core outcomes in clinical trials across the spectrum of rheumatologic conditions.
Berardi S, Corrado A, Maruotti N, Cici D, Cantatore FP. Osteoblast role in the pathogenesis of rheumatoid arthritis. Mol Biol Rep. 2021 Mar;48(3):2843-2852. doi: 10.1007/s11033-021-06288-y. Epub 2021 Mar 27. PMID: 33774802; PMCID: PMC8060181.
Behrens, F., Koehm, M., Schwaneck, E.C. et al. Use of a "critical difference" statistical criterion improves the predictive utility of the Health Assessment Questionnaire-Disability Index score in patients with rheumatoid arthritis. BMC Rheumatol 3, 51 (2019). https://doi.org/10.1186/s41927-019-0095-2
Bizzi E, Massafra U, Laganà B, Bruzzese V, Diamanti AP, Cassol M, Migliore A. Radiological outcomes in randomized controlled trials on biologic therapies for rheumatoid arthritis: a narrative review. Clin Rheumatol. 2014 Jul;33(7):877-84. doi: 10.1007/s10067-014-2504-7. Epub 2014 Feb 8. PMID: 24510026. Bonafede MM, Curtis JR, McMorrow D, Mahajan P, Chen CI. Treatment effectiveness and treatment patterns among rheumatoid arthritis patients after switching from a tumor necrosis factor inhibitor to another medication. Clinicoecon Outcomes Res. 2016 Dec 2;8:707-715. doi: 10.2147/CEOR.S115706. PMID: 27980429; PMCID: PMC5144914.
Brazier JE, Harper R, Jones NM, O'Cathain A, Thomas KJ, Usherwood T, Westlake L. Validating the SF-36 health survey questionnaire: new outcome measure for primary care. BMJ. 1992 Jul 18;305(6846):160-4. doi: 10.1136/bmj.305.6846.160. PMID: 1285753; PMCID: PMC1883187.
Brown AK, Quinn MA, Karim Z, Conaghan PG, Peterfy CG, Hensor E, Wakefield RJ, "Connor PJ, Emery P. Presence of significant synovitis in rheumatoid arthritis patients with disease-modifying antirheumatic drug-induced clinical remission: evidence from an imaging study may explain structural progression. Arthritis Rheum. 2006 Dec;54(12):3761-73. doi: 10.1002/art.22190. PMID: 17133543.
Burgers, L.E., Boeters, D.M., Reijnierse, M. et al. Does the presence of magnetic resonance imaging-detected osteitis at diagnosis with rheumatoid arthritis lower the risk for achieving disease-modifying antirheumatic drug-free sustained remission: results of a longitudinal study. Arthritis Res Ther 20, 68 (2018). https://doi.org/10.1186/s13075-018-1553-8
Cipolletta E, Incorvaia A, Mashadi Mirza R, et alSAT0031 CORRELATION BETWEEN IRREVERSIBLE PHYSICAL DISABILITY AND JOINT DAMAGE IN RHEUMATOID ARTHRITISAnnals of the Rheumatic Diseases 2020;79:945-946.
Chotiyarnwong, P., McCloskey, E. Pathogenesis of glucocorticoid-induced osteoporosis and options for treatment. Nat Rev Endocrinol 16, 437-447 (2020). https://doi.org/10.1038/s41574-020-0341-0 Clements PJ, Wong WK, Hurwitz EL, Furst DE, Mayes M, White B, Wigley F, Weisman M, Barr W, Moreland L, Medsger TA Jr, Steen V, Martin R, Collier D, Weinstein A, Lally E, Varga J, Weiner S, Andrews B, Abeles M, Seibold J. Correlates of the disability index of the health assessment questionnaire: a measure of functional impairment in systemic sclerosis. Arthritis Rheum. 1999 Nov;42(11):2372-80. doi: 10.1002/1529-0131(199911)42:11<2372::AID-ANR16>3.0.CO;2-J. PMID: 10555033.
Colangelo KJ, Pope JE, Peschken C. The minimally important difference for patient reported outcomes in systemic lupus erythematosus including the HAQ-DI, pain, fatigue, and SF-36. J Rheumatol. 2009 Oct;36(10):2231-7. doi: 10.3899/jrheum.090193. Epub 2009 Sep 1. PMID: 19723907.
Conaghan PG, Østergaard M, Bowes MA, Wu C, Fuerst T, van der Heijde D, Irazoque-Palazuelos F, Soto-Raices O, Hrycaj P, Xie Z, Zhang R, Wyman BT, Bradley JD, Soma K, Wilkinson B. Comparing the effects of tofacitinib, methotrexate and the combination, on bone marrow oedema, synovitis and bone erosion in methotrexate-naive, early active rheumatoid arthritis: results of an exploratory randomised MRI study incorporating semiquantitative and quantitative techniques. Ann Rheum Dis. 2016 Jun;75(6):1024-33. doi: 10.1136/annrheumdis-2015-208267. Epub 2016 Jan 25. PMID: 27002108; PMCID: PMC4893111.
Čota S, Delimar V, Žagar I, Kovač Durmiš K, Kristić Cvitanović N, Žura N, Perić P, Laktašić Žerjavić N. Efficacy of therapeutic ultrasound in the treatment of chronic calcific shoulder tendinitis: a randomized trial. Eur J Phys Rehabil Med. 2023 Feb;59(1):75-84. doi: 10.23736/51973-9087.22.07715-2. Epub 2023 Feb 1. PMID: 36723056; PMCID: PMC10035437.
Danieli MG, Paladini A, Longhi E, Tonacci A, Gangemi S. A machine learning analysis to evaluate the outcome measures in inflammatory myopathies. Autoimmun Rev. 2023 Jul;22(7):103353. doi: 10.1016/j.autrev.2023.103353. Epub 2023 May 2. PMID: 37142194.
Dahir KM, Kishnani PS, Martos-Moreno GÁ, Linglart A, Petryk A, Rockman-Greenberg C, Martel SE, Ozono K, Högler W, Seefried L. Impact of muscular symptoms and/or pain on disease characteristics, disability, and quality of life in adult patients with hypophosphatasia: A cross-sectional analysis from the Global HPP Registry. Front Endocrinol (Lausanne). 2023 Mar 27;14:1138599. doi: 10.3389/fendo.2023.1138599. PMID: 37051203; PMCID: PMC10083387.
DEANDRADE JR, CASAGRANDE PA. A SEVEN-DAY VARIABILITY STUDY OF 499 PATIENTS WITH PERIPHERAL RHEUMATOID ARTHRITIS. Arthritis Rheum. 1965 Apr;8:302-34. PMID: 14280261.
P. Emery, I. B. Mclnnes, R. van Vollenhoven, M. C. Kraan, Clinical identification and treatment of a rapidly progressing disease state in patients with rheumatoid arthritis, Rheumatology, Volume 47, Issue 4, April 2008, Pages 392-398, https://doi.org/10.1093/rheumatology/kem257
De la Corte-Rodriguez H, Rodriguez-Merchan EC. The Health Assessment Questionnaire Disability Index (HAQ-DI) as a valid alternative for measuring the functional capacity of people with haemophilia.
Thromb Res. 2017 May;153:51-56. doi: 10.1016/j.thromres.2017.03.016. Epub 2017 Mar 14. PMID: 28324767.
Dougados M, Brault Y, Logeart I, van der Heijde D, Gossec L, Kvien T. Defining cut-off values for disease activity states and improvement scores for patient-reported outcomes: the example of the Rheumatoid Arthritis Impact of Disease (RAID). Arthritis Res Ther. 2012 May 30;14(3):R129. doi: 10.1186/ar3859. PMID: 22647431; PMCID: PMC3446510.
Duarte C, Santos EJF, Ferreira RJO, Kvien TK, Dougados M, de Wit M, da Silva JAP, Gossec L. Validity and reliability of the EULAR instrument RAID.7 as a tool to assess individual domains of impact of disease in rheumatoid arthritis: a cross-sectional study of 671 patients. RMD Open. 2021 Feb;7(1):e001539. doi: 10.1136/rmdopen-2020-001539. PMID: 33547229; PMCID: PMC7871340.
Einarsson JT, Willim M, Ernestam S, Saxne T, Geborek P, Kapetanovic MC. Prevalence of sustained remission in rheumatoid arthritis: impact of criteria sets and disease duration, a Nationwide Study in Sweden. Rheumatology (Oxford). 2019 Feb 1;58(2):227-236. doi: 10.1093/rheumatology/key054. PMID: 29538755.
Farquhar H, Vassallo R, Edwards AL, Matteson EL. Pulmonary Complications of Rheumatoid Arthritis.
Semin Respir Crit Care Med. 2019 Apr;40(2):194-207. doi: 10.1055/s-0039-1683995. Epub 2019 May 28. PMID: 31137060.
Fazaa A, Boussaa H, Ouenniche K, et alOPTIMAL ASSESSMENT OF FATIGUE IN RHEUMATOID ARTHRITIS: VISUAL ANALOG SCALE VERSUS FUNCTIONAL ASSESSMENT OF CHRONIC ILLNESS THERAPY - FATIGUEAnnals of the Rheumatic Diseases 2021;80:1113-1114.
Felson, D. and (2007), A proposed revision to the ACR20: The hybrid measure of American College of Rheumatology responset. Arthritis & Rheumatism, 57: 193-202. https://doi.org/10.1002/art.22552.
Grote C, Reinhardt D, Zhang M, Wang J. Regulatory mechanisms and clinical manifestations of musculoskeletal aging. J Orthop Res. 2019 Jul;37(7):1475-1488. doi: 10.1002/jor.24292. Epub 2019 Apr 3. PMID: 30919498; PMCID: PMC9202363.
Ferrari M, Onuoha SC, Pitzalis C. Trojan horses and guided missiles: targeted therapies in the war on arthritis. Nat Rev Rheumatol. 2015 Jun;11(6):328-37. doi: 10.1038/nrrheum.2015.17. Epub 2015 Mar 3. PMID: 25734971.Fleischmann R, Schiff M, van der Heijde D, Ramos-Remus C, Spindler A, Stanislav M, Zerbini CA, Gurbuz S, Dickson C, de Bono S, Schlichting D, Beattie S, Kuo WL, Rooney T, Macias W, Takeuchi T. Baricitinib, Methotrexate, or Combination in Patients With Rheumatoid Arthritis and No or Limited Prior Disease-Modifying Antirheumatic Drug Treatment. Arthritis Rheumatol. 2017a Mar;69(3):506-517. doi: 10.1002/art.39953. PMID: 27723271; PMCID: PMC5347954.
Fleischmann R, Alam J, Arora V, Bradley J, Schlichting DE, Muram D, Smolen JS. Safety and efficacy of baricitinib in elderly patients with rheumatoid arthritis. RMD Open. 2017b Oct 10;3(2):e000546. doi: 10.1136/rmdopen-2017-000546. PMID: 29071120; PMCID: PMC5640108.
Fleischmann R, Takeuchi T, Schiff M, Schlichting D, Xie L, Issa M, Stoykov I, Lisse J, Martinez-Osuna P, Rooney T, Zerbini CAF. Efficacy and Safety of Long-Term Baricitinib With and Without Methotrexate for the Treatment of Rheumatoid Arthritis: Experience With Baricitinib Monotherapy Continuation or After Switching From Methotrexate Monotherapy or Baricitinib Plus Methotrexate. Arthritis Care Res (Hoboken). 2020 Aug;72(8):1112-1121. doi: 10.1002/acr.24007. PMID: 31233281.
Fuchs HA, Brooks RH, Callahan LF, Pincus T. A simplified twenty-eight-joint quantitative articular index in rheumatoid arthritis. Arthritis Rheum. 1989 May;32(5):531-7. doi: 10.1002/anr.1780320504. PMID: 2719728.
Gossec L, Paternotte S, Aanerud GJ, Balanescu A, Boumpas DT, Carmona L, de Wit M, Dijkmans BA, Dougados M, Englbrecht M, Gogus F, Heiberg T, Hernandez C, Kirwan JR, Mola EM, Cerinic MM, Otsa K, Schett G, Scholte-Voshaar M, Sokka T, von Krause G, Wells GA, Kvien TK. Finalisation and validation of the rheumatoid arthritis impact of disease score, a patient-derived composite measure of impact of rheumatoid arthritis: a EULAR initiative. Ann Rheum Dis. 2011 Jun;70(6):935-42. doi: 10.1136/ard.2010.142901. PMID: 21540201.
Guidelli GM, Barskova T, Brizi MG, Lepri G, Parma A, Talarico R, Cantarini L, Frediani B. One year in review: novelties in the treatment of rheumatoid arthritis. Clin Exp Rheumatol. 2015 Jan-Feb;33(1):102-8. Epub 2015 Feb 26. PMID: 25719499.
Guo Q, Wang Y, Xu D, Nossent J, Pavlos NJ, Xu J. Rheumatoid arthritis: pathological mechanisms and modern pharmacologic therapies. Bone Res. 2018 Apr 27;6:15. doi: 10.1038/s41413-018-0016-9. PMID: 29736302; PMCID: PMC5920070.
Health Assessment Questionnaire Disability Index (HAD-QI). (2023, September 21). Physiopedia, . Retrieved 13:21, April 26, 2024 from https://www.physiopedia.com/index.php?title=Health_Assessment_Questionnaire_Disability_Index_(HAD-QI)&oldid=341627.
Heinlen L, Humphrey MB. Skeletal complications of rheumatoid arthritis. Osteoporos Int. 2017 Oct;28(10):2801-2812. doi: 10.1007/s00198-017-4170-5. Epub 2017 Aug 4. PMID: 28779302.
Hewlett, S., Dures, E. and Almeida, C. (2011), Measures of fatigue: Bristol Rheumatoid Arthritis Fatigue Multi-Dimensional Questionnaire (BRAF MDQ), Bristol Rheumatoid Arthritis Fatigue Numerical Rating Scales (BRAF NRS) for Severity, Effect, and Coping, Chalder Fatigue Questionnaire (CFQ), Checklist Individual Strength (CIS20R and CIS8R), Fatigue Severity Scale (FSS), Functional Assessment Chronic Illness Therapy (Fatigue) (FACIT-F), Multi-Dimensional Assessment of Fatigue (MAF), Multi-Dimensional Fatigue Inventory (MFI), Pediatric Quality Of Life (PedsQL) Multi-Dimensional Fatigue Scale, Profile of Fatigue (ProF), Short Form 36 Vitality Subscale (SF-36 VT), and Visual Analog Scales (VAS). Arthritis Care Res, 63: S263-S286. https://doi.org/10.1002/acr.20579
Hu Q, Zhong X, Tian H, Liao P. The Efficacy of Denosumab in Patients With Rheumatoid Arthritis: A Systematic Review and Pooled Analysis of Randomized or Matched Data. Front Immunol. 2022 Jan 5;12:799575. doi: 10.3389/fimmu.2021.799575. PMID: 35069583; PMCID: PMC8766643.
Jinesh S. Pharmaceutical aspects of anti-inflammatory TNF-blocking drugs. Inflammopharmacology. 2015 Jun;23(2-3):71-7. doi: 10.1007/s10787-015-0229-0. Epub 2015 Feb 17. PMID: 25687751. Katchamart W, Narongroeknawin P, Chanapai W, Thaweeratthakul P. Health-related quality of life in patients with rheumatoid arthritis. BMC Rheumatol. 2019 Aug 14;3:34. doi: 10.1186/s41927-019-0080-9. PMID: 31428740; PMCID: PMC6694487.
Khanna D, Clements PJ, Postlethwaite AE, Furst DE. Does incorporation of aids and devices make a difference in the score of the health assessment questionnaire-disability index? Analysis from a scleroderma clinical trial. J Rheumatol. 2008 Mar;35(3):466-8. Epub 2007 Dec 15. PMID: 18085737.
Kleyer A, Schett G. Arthritis and bone loss: a hen and egg story. Curr Opin Rheumatol. 2014 Jan;26(1):80-4. doi: 10.1097/BOR.0000000000000007. PMID: 24276089.
Krishnan E, Sokka T, Häkkinen A, Hubert H, Hannonen P. Normative values for the Health Assessment Questionnaire disability index: benchmarking disability in the general population. Arthritis Rheum. 2004 Mar;50(3):953-60. doi: 10.1002/art.20048. PMID: 15022339.
Kitchen h, Bekker Hansen B, Abetz L, Højbjerre L and Strandberg-Larsen M, Endpoint Development & Outcomes Assessment, Adelphi Values Ltd, Health Economics & HTA, Novo Nordisk A/S (2013) Patient-Reported Outcome Measures For Rheumatoid Arthritis: Minimal Important Differences Review. 2013 ACR/ARHP Annual Meeting. https://acrabstracts.org/abstract/patient-reported-outcome-measures-for-rheumatoid-arthritis-minimal-important-differences-review/.
Lee KE, Choi SE, Xu H, Kang JH, Park DJ, Lee SS. HAQ score is an independent predictor of sustained remission in patients with rheumatoid arthritis. Rheumatol Int. 2017 Dec;37(12):2027-2034. doi: 10.1007/s00296-017-3833-z. Epub 2017 Sep 27. PMID: 28956118.
Leong KP, Tan JWL, Gao X, Koh ET; TTSH Rheumatoid Arthritis Study Group. Conversion among the 28-joint count activity indices for rheumatoid arthritis. Eur J Rheumatol. 2020 Jul;7(3):105-111. doi: 10.5152/eurjrheum.2020.19199. Epub 2020 Jul 1. PMID: 32809931; PMCID: PMC7431355.
Leung YY, Tillett W, Hojgaard P, Orbai AM, Holland R, Mathew AJ, Goel N, Chau J, Lindsay CA, Ogdie A, Coates LC, Christensen R, Mease PJ, Strand V, Gladman DD. Test-retest Reliability for HAQ-DI and SF-36 PF for the Measurement of Physical Function in Psoriatic Arthritis. J Rheumatol. 2021 Oct;48(10):1547-1551. doi: 10.3899/jrheum.210175. Epub 2021 Apr 15. PMID: 33858982; PMCID: PMC8487911.
Leung JL, Twohig H, Muller S, Maxwell L, Mackie SL, Neill LM, Owen CE; OMERACT PMR Working Group. Test-retest reliability of pain VAS/NRS, stiffness VAS/NRS, HAQ-DI and mHAQ in polymyalgia rheumatica: An OMERACT study. Semin Arthritis Rheum. 2023 Oct;62:152239. doi: 10.1016/j.semarthrit.2023.152239. Epub 2023 Jul 5. PMID: 37429139.
Lin J, Yang L, Chen L, et alTHU0069 RHEUMATOID CACHEXIA ASSESSED BY BODY COMPOSITION IS ASSOCIATED WITH WORSE DISEASE IN RHEUMATOID ARTHRITIS PATIENTS WITH NORMAL BODY MASS INDEXAnnals of the Rheumatic Diseases 2019;78:303.
Liu H, Zhu Y, Gao Y, Qi D, Zhao L, Zhao L, Liu C, Tao T, Zhou C, Sun X, Guo F, Xiao J. NR1D1 modulates synovial inflammation and bone destruction in rheumatoid arthritis. Cell Death Dis. 2020 Feb 18;11(2):129. doi: 10.1038/s41419-020-2314-6. PMID: 32071294; PMCID: PMC7028921.
Lundquist LM, Cole SW, Sikes ML. Efficacy and safety of tofacitinib for treatment of rheumatoid arthritis. World J Orthop. 2014 Sep 18;5(4):504-11. doi: 10.5312/wjo.v5.i4.504. PMID: 25232526; PMCID: PMC4133456.
Mainland, D. (1965), A Seven-Day variability study of 499 patients with peripheral rheumatoid arthritis. Arthritis & Rheumatism, 8: 302-334. https://doi.org/10.1002/art.1780080214Mclnnes IB, Schett G.
Pathogenetic insights from the treatment of rheumatoid arthritis. Lancet. 2017 Jun 10;389(10086):2328-2337. doi: 10.1016/50140-6736(17)31472-1. PMID: 28612747.
Michaud, K., Pope, J., van de Laar, M., Curtis, J.R., Kannowski, C., Mitchell, S., Bell, J., Workman, J., Paik, J., Cardoso, A. and Taylor, P.C. (2021), Systematic Literature Review of Residual Symptoms and an Unmet Need in Patients With Rheumatoid Arthritis. Arthritis Care Res, 73: 1606-1616. https://doi.org/10.1002/acr.24369
Michaud K, Pope J, van de Laar M, Curtis JR, Kannowski C, Mitchell S, Bell J, Workman J, Paik J, Cardoso A, Taylor PC. Systematic Literature Review of Residual Symptoms and an Unmet Need in Patients With Rheumatoid Arthritis. Arthritis Care Res (Hoboken). 2021 Nov;73(11):1606-1616. doi: 10.1002/acr.24369. Epub 2021 Sep 9. PMID: 32619340; PMCID: PMC8596735.
Mistry J, Sharif M, Prideaux A, Smith C, Sumbwanyambe M, Sibley M, Carpenter L, Sweeney M, Kiely P. Use of rheumatoid arthritis impact of disease (RAID) in routine care; identification of DAS28 remission and unmet patient-reported outcomes. Rheumatol Adv Pract. 2020 Apr 27;4(2):rkaa013. doi: 10.1093/rap/rkaa013. PMID: 32685911; PMCID: PMC7359769.
Miwa Y, Saito M, Furuya H, Yanai R, Ikari Y, Hayashi T, Kasama T, Toyoshima Y, Inagaki K. Clinical Characteristics of Rheumatoid Arthritis Patients Achieving Functional Remission after Six Months of Non-tumor Necrosis Factor Biological Disease-Modifying Antirheumatic Drugs (DMARDs) Treatment.
Intern Med. 2017 Sep 1;56(17):2271-2275. doi: 10.2169/internalmedicine.8723-16. Epub 2017 Aug 10. PMID: 28794381; PMCID: PMC5635297.
Morinobu A. JAK inhibitors for the treatment of rheumatoid arthritis. Immunol Med. 2020 Dec;43(4):148-155. doi: 10.1080/25785826.2020.1770948. Epub 2020 Jun 5. PMID: 32501149.
Nam JL, Takase-Minegishi K, Ramiro S, Chatzidionysiou K, Smolen JS, van der Heijde D, Bijlsma JW, Burmester GR, Dougados M, Scholte-Voshaar M, van Vollenhoven R, Landewé R. Efficacy of biological disease-modifying antirheumatic drugs: a systematic literature review informing the 2016 update of the EULAR recommendations for the management of rheumatoid arthritis. Ann Rheum Dis. 2017 Jun;76(6):1113-1136. doi: 10.1136/annrheumdis-2016-210713. Epub 2017 Mar 10. PMID: 28283512.
Nehring SM, Goyal A, Patel BC. C Reactive Protein. [Updated 2023 Jul 10]. In: StatPearls [Internet]. Treasure Island (FL): StatPearls Publishing; 2024 Jan-. Available from: https://www.ncbi.nlm.nih.gov/books/NBK441843/
Nikiphorou, E., Radner, H., Chatzidionysiou, K. et al. Patient global assessment in measuring disease activity in rheumatoid arthritis: a review of the literature. Arthritis Res Ther 18, 251 (2016). https://doi.org/10.1186/s13075-016-1151-6.
Olsen IC, Lie E, Vasilescu R, Wallenstein G, Strengholt S, Kvien TK. Assessments of the unmet need in the management of patients with rheumatoid arthritis: analyses from the NOR-DMARD registry.
Rheumatology (Oxford). 2019 Mar 1;58(3):481-491. doi: 10.1093/rheumatology/key338. PMID: 30508189; PMCID: PMC6381770.
Østergaard M, Peterfy C, Conaghan P, McQueen F, Bird P, Ejbjerg B, Shnier R, O'Connor P, Klarlund M, Emery P, Genant H, Lassere M, Edmonds J. OMERACT Rheumatoid Arthritis Magnetic Resonance Imaging Studies. Core set of MRI acquisitions, joint pathology definitions, and the OMERACT RA-MRI scoring system. J Rheumatol. 2003 Jun;30(6):1385-6. Erratum in: J Rheumatol. 2004 Jan;31(1):198. PMID: 12784422.
Østergaard M, Peterfy CG, Bird P, Gandjbakhch F, Glinatsi D, Eshed I, Haavardsholm EA, Lillegraven S, Bøyesen P, Ejbjerg B, Foltz V, Emery P, Genant HK, Conaghan PG. The OMERACT Rheumatoid Arthritis Magnetic Resonance Imaging (MRI) Scoring System: Updated Recommendations by the OMERACT MRI in Arthritis Working Group. J Rheumatol. 2017 Nov;44(11):1706-1712. doi: 10.3899/jrheum.161433. Epub 2017 Aug 15. PMID: 28811353.
Panagopoulos PK, Lambrou GI. Bone erosions in rheumatoid arthritis: recent developments in pathogenesis and therapeutic implications. J Musculoskelet Neuronal Interact. 2018 Sep 1;18(3):304-319. PMID: 30179207; PMCID: PMC6146189.
Peterfy C, Emery P, Tak PP, et alMRI assessment of suppression of structural damage in patients with rheumatoid arthritis receiving rituximab: results from the82andomizedd, placebo-controlled, double-blind RA-SCORE studyAnnals of the Rheumatic Diseases 2016;75:170-177.
Pierreisnard A, Issa N, Barnetche T, Richez C, Schaeverbeke T. Meta-analysis of clinical and radiological efficacy of biologics in rheumatoid arthritis patients naive or inadequately responsive to methotrexate. Joint Bone Spine. 2013 Jul;80(4):386-92. doi: 10.1016/j.jbspin.2012.09.023. Epub 2012 Nov 7. PMID: 23141718.
Ippokratis Pountos, Michalis Panteli, Gavin Walters, Peter V. Giannoudis. NSAIDs inhibit bone healing through the downregulation of TGF-β3 expression during endochondral ossification. Injury, Volume 52, Issue 6, 2021, Pages 1294-1299, ISSN 0020-1383, https://doi.org/10.1016/j.injury.2021.01.007.
Prevoo ML, van"t Hof MA, Kuper HH, van Leeuwen MA, van de Putte LB, van Riel PL. Modified disease activity scores that include twenty-eight-joint counts. Development and validation in a prospective longitudinal study of patients with rheumatoid arthritis. Arthritis Rheum. 1995 Jan;38(1):44-8. doi: 10.1002/art.1780380107. PMID: 7818570.
Rakieh C, Conaghan PG. Tofacitinib for treatment of rheumatoid arthritis. Adv Ther. 2013 Aug;30(8):713-26. doi: 10.1007/s12325-013-0047-y. Epub 2013 Aug 14. PMID: 23943546.
Rawla P. Cardiac and vascular complications in rheumatoid arthritis. Reumatologia. 2019;57(1):27-36. doi: 10.5114/reum.2019.83236. Epub 2019 Feb 28. PMID: 30858628; PMCID: PMC6409824.
Rendas-Baum, R., Lin, X., Kosinski, M. et al. Meaningful score changes for SF-36v2, FACIT-fatigue, and RASIQ in rheumatoid arthritis. J Patient Rep Outcomes 8, 9 (2024). https://doi.org/10.1186/s41687-024-00685-0
Riise T, Jacobsen BK, Gran JT, Haga HJ, Arnesen E. Total mortality is increased in rheumatoid arthritis. A 17-year prospective study. Clin Rheumatol. 2001;20(2):123-7. doi: 10.1007/pl00011191. PMID: 11346224.
Romaniello CE, Falls AC, Ricketts P, Dega A, Elliott JO, Jordan KM. Driving Impairment and Healthcare Provider Counseling in Rheumatoid Arthritis and Osteoarthritis Patients: A Cross-Sectional Survey.
Cureus. 2022 Jun 24;14(6):e26280. doi: 10.7759/cureus.26280. PMID: 35911308; PMCID: PMC9312789.
Rubbert-Roth A, Szabó MZ, Kedves M, Nagy G, Atzeni F, Sarzi-Puttini P. Failure of anti-TNF treatment in patients with rheumatoid arthritis: The pros and cons of the early use of alternative biological agents. Autoimmun Rev. 2019 Dec;18(12):102398. doi: 10.1016/j.autrev.2019.102398. Epub 2019 Oct 19. PMID: 31639514.
Ruyssen-Witrand, A., Guernec, G., Dupont, J. et al. Ten-year radiographic and functional outcomes in rheumatoid arthritis patients in remission compared to patients in low disease activity. Arthritis Res Ther 25, 207 (2023). https://doi.org/10.1186/s13075-023-03176-7
Serhal L, Edwards CJ. Upadacitinib for the treatment of rheumatoid arthritis. Expert Rev Clin Immunol. 2019 Jan;15(1):13-25. doi: 10.1080/1744666X.2019.1544892. Epub 2018 Nov 19. PMID: 30394138 Sewerin P, Vordenbaeumen S, Hoyer A, Brinks R, Buchbender C, Miese F, Schleich C, Klein S, Schneider M, Ostendorf B. Silent progression in patients with rheumatoid arthritis: is DAS28 remission an insufficient goal in RA? Results from the German Remission-plus cohort. BMC Musculoskelet Disord.
2017 Apr 19;18(1):163. doi: 10.1186/s12891-017-1528-y. PMID: 28420375; PMCID: PMC5395882.
Siebert S, Tsoukas A, Robertson J, Mclnnes I. Cytokines as therapeutic targets in rheumatoid arthritis and other inflammatory diseases. Pharmacol Rev. 2015;67(2):280-309. doi: 10.1124/pr.114.009639. PMID: 25697599.
Smolen JS, Aletaha D, Bijlsma JW, Breedveld FC, Boumpas D, Burmester G, Combe B, Cutolo M, de Wit M, Dougados M, Emery P, Gibofsky A, Gomez-Reino JJ, Haraoui B, Kalden J, Keystone EC, Kvien TK, Mclnnes I, Martin-Mola E, Montecucco C, Schoels M, van der Heijde D; T2T Expert Committee. Treating rheumatoid arthritis to target: recommendations of an international task force. Ann Rheum Dis. 2010 Apr;69(4):631-7. doi: 10.1136/ard.2009.123919. Epub 2010 Mar 9. Erratum in: Ann Rheum Dis. 2011 Aug;70(8):1519. Erratum in: Ann Rheum Dis. @011 Jul;70(7):1349. van der Heijde, Desirée [corrected to van der Heijde, Désirée]. PMID: 20215140; PMCID: PMC3015099.
Smolen JS, Landewé R, Breedveld FC, Buch M, Burmester G, Dougados M, Emery P, Gaujoux-Viala C, Gossec L, Nam J, Ramiro S, Winthrop K, de Wit M, Aletaha D, Betteridge N, Bijlsma JW, Boers M, Buttgereit F, Combe B, Cutolo M, Damjanov N, Hazes JM, Kouloumas M, Kvien TK, Mariette X, Pavelka K, van Riel PL, Rubbert-Roth A, Scholte-Voshaar M, Scott DL, Sokka-Isler T, Wong JB, van der Heijde D.
EULAR recommendations for the management of rheumatoid arthritis with synthetic and biological disease-modifying antirheumatic drugs: 2013 update. Ann Rheum Dis. 2014 Mar;73(3):492-509. doi: 10.1136/annrheumdis-2013-204573. Epub 2013 Oct 25. PMID: 24161836; PMCID: PMC3933074.
Smolen JS, Aletaha D. Rheumatoid arthritis therapy reappraisal: strategies, opportunities and challenges. Nat Rev Rheumatol. 2015 May;11(5):276-89. doi: 10.1038/nrrheum.2015.8. Epub 2015 Feb 17. PMID: 25687177.
Smolen JS, Landewé R, Bijlsma J, Burmester G, Chatzidionysiou K, Dougados M, Nam J, Ramiro S, Voshaar M, van Vollenhoven R, Aletaha D, Aringer M, Boers M, Buckley CD, Buttgereit F, Bykerk V, Cardiel M, Combe B, Cutolo M, van Eijk-Hustings Y, Emery P, Finckh A, Gabay C, Gomez-Reino J, Gossec L, Gottenberg JE, Hazes JMW, Huizinga T, Jani M, Karateev D, Kouloumas M, Kvien T, Li Z, Mariette X, Mclnnes I, Mysler E, Nash P, Pavelka K, Poór G, Richez C, van Riel P, Rubbert-Roth A, Saag K, da Silva J, Stamm T, Takeuchi T, Westhovens R, de Wit M, van der Heijde D. EULAR recommendations for the management of rheumatoid arthritis with synthetic and biological disease-modifying antirheumatic drugs: 2016 update. Ann Rheum Dis. 2017 Jun;76(6):960-977. doi: 10.1136/annrheumdis-2016-210715. Epub 2017 Mar 6. PMID: 28264816.
Smolen JS, Aletaha D, Barton A, Burmester GR, Emery P, Firestein GS, Kavanaugh A, Mclnnes IB, Solomon DH, Strand V, Yamamoto K. Rheumatoid arthritis. Nat Rev Dis Primers. 2018 Feb 8;4:18001. doi: 10.1038/nrdp.2018.1. PMID: 29417936.
Smolen JS, Landewé RBM, Bijlsma JWJ, Burmester GR, Dougados M, Kerschbaumer A, Mclnnes IB, Sepriano A, van Vollenhoven RF, de Wit M, Aletaha D, Aringer M, Askling J, Balsa A, Boers M, den Broeder AA, Buch MH, Buttgereit F, Caporali R, Cardiel MH, De Cock D, Codreanu C, Cutolo M, Edwards CJ, van Eijk-Hustings Y, Emery P, Finckh A, Gossec L, Gottenberg JE, Hetland ML, Huizinga TWJ, Koloumas M, Li Z, Mariette X, Müller-Ladner U, Mysler EF, da Silva JAP, Poór G, Pope JE, Rubbert-Roth A, Ruyssen-Witrand A, Saag KG, Strangfeld A, Takeuchi T, Voshaar M, Westhovens R, van der Heijde D. EULAR recommendations for the management of rheumatoid arthritis with synthetic and biological disease-modifying antirheumatic drugs: 2019 update. Ann Rheum Dis. 2020 Jun;79(6):685-699. doi: 10.1136/annrheumdis-2019-216655. Epub 2020 Jan 22. PMID: 31969328.
Sokka T, Kautiainen H, Hannonen P, Pincus T. Changes in Health Assessment Questionnaire disability scores over five years in patients with rheumatoid arthritis compared with the general population.
Arthritis Rheum. 2006 Oct;54(10):3113-8. doi: 10.1002/art.22130. PMID: 17009231.
Sokka T, Pincus T. Joint counts to assess rheumatoid arthritis for clinical research and usual clinical care: advantages and limitations. Rheum Dis Clin North Am. 2009 Nov;35(4):713-22, v-vi. doi: 10.1016/j.rdc.2009.10.004. PMID: 19962615.
Sousa KH, Kwok OM, Ryu E, Cook SW. Confirmation of the validity of the HAQ-DI in two populations living with chronic illnesses. J Nurs Meas. 2008;16(1):31-42. doi: 10.1891/1061-3749.16.1.31. PMID: 18578108.
Spence J, Bosch J, Chongsi E, Lee SF, Thabane L, Mendoza P, Belley-Côté E, Whitlock R, Brady K, McIntyre WF, Lamy A, Devereaux PJ. Standardized Assessment of Global activities in the Elderly scale in adult cardiac surgery patients. Br J Anaesth. 2021 Oct;127(4):539-546. doi: 10.1016/j.bja.2021.05.037. Epub 2021 Jul 28. PMID: 34330417.
Strand V, Mysler E, Moots RJ, et alPatient-reported outcomes for tofacitinib with and without methotrexate, or adalimumab with methotrexate, in rheumatoid arthritis: a phase IIIB/IV trialRMD Open 2019;5:e001040. doi: 10.1136/rmdopen-2019-001040
Strand V, Tundia N, Bergman M, Ostor A, Durez P, Song IH, Enejosa J, Schlacher C, Song Y, Fleischmann R. Upadacitinib improves patient-reported outcomes vs placebo or adalimumab in patients with rheumatoid arthritis: results from SELECT-COMPARE, Rheumatology, Volume 60, Issue 12, December 2021, Pages 5583-5594, https://doi.org/10.1093/rheumatology/keab158
Studenic P, Aletaha D, de Wit M, Stamm TA, Alasti F, Lacaille D, Smolen JS, Felson DT. American College of Rheumatology/EULAR Remission Criteria for Rheumatoid Arthritis: 2022 Revision. Arthritis Rheumatol. 2023 Jan;75(1):15-22. doi: 10.1002/art.42347. Epub 2022 Oct 23. PMID: 36274193; PMCID: PMC10092655.
Taylor PC, Moore A, Vasilescu R, Alvir J, Tarallo M. A structured literature review of the burden of illness and unmet needs in patients with rheumatoid arthritis: a current perspective. Rheumatol Int. 2016 May;36(5):685-95. doi: 10.1007/s00296-015-3415-x. Epub 2016 Jan 8. PMID: 26746843; PMCID: PMC4839053.
Taylor PC. Clinical efficacy of launched JAK inhibitors in rheumatoid arthritis. Rheumatology (Oxford). 2019 Feb 1;58(Suppl 1):i17-i26. doi: 10.1093/rheumatology/key225. PMID: 30806707; PMCID: PMC6390878.
van der Heijde How to read radiographs according to the Sharp/van der Heijde method J Rheumatol 2000; 27:261-3
Welsing PM, van Gestel AM, Swinkels HL, Kiemeney LA, van Riel PL. The relationship between disease activity, joint destruction, and functional capacity over the course of rheumatoid arthritis. Arthritis Rheum. 2001 Sep;44(9):2009-17. doi: 10.1002/1529-0131(200109)44:9<2009::AID-ART349>3.0.CO;2-L. PMID: 11592361.
van der Heijde DM. Plain X-rays in rheumatoid arthritis: overview of scoring methods, their reliability and applicability. Baillieres Clin Rheumatol. 1996 Aug;10(3):435-53. doi: 10.1016/s0950-3579(96)80043-4. PMID: 8876953.
Wells G, Becker JC, Teng J, Dougados M, Schiff M, Smolen J, Aletaha D, van Riel PL. Validation of the 28-joint Disease Activity Score (DAS28) and European League Against Rheumatism response criteria based on C-reactive protein against disease progression in patients with rheumatoid arthritis, and comparison with the DAS28 based on erythrocyte sedimentation rate. Ann Rheum Dis. 2009 Jun;68(6):954-60. doi: 10.1136/ard.2007.084459. Epub 2008 May 19. PMID: 18490431; PMCID: PMC2674547.
Welsing PM, van Gestel AM, Swinkels HL, Kiemeney LA, van Riel PL. The relationship between disease activity, joint destruction, and functional capacity over the course of rheumatoid arthritis. Arthritis Rheum. 2001 Sep;44(9):2009-17. doi: 10.1002/1529-0131(200109)44:9<2009::AID-ART349>3.0.CO;2-L. PMID: 11592361.
Wolfe F, Hawley DJ. The longterm outcomes of rheumatoid arthritis: Work disability: a prospective 18 year study of 823 patients. J Rheumatol. 1998 Nov;25(11):2108-17. PMID: 9818651.
Wolfe F, Michaud K, Gefeller O, et al. Predicting mortality in patients with rheumatoid arthritis. Arthritis Rheum 2003;48:1530-42.
Yilmaz V, Umay E, Gündo 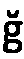 du i, Karaahmet ZÖ, Öztürk AE. Rheumatoid Arthritis: Are psychological factors effective in disease flare? Eur J Rheumatol. 2017 Jun;4(2):127-132. doi: 10.5152/eurjrheum.2017.16100. Epub 2017 Jun 1. PMID: 28638686; PMCID: PMC5473448.
Zhang X, Lin L, Sun X, Lei X, Liu GG, Raat H, Zeng Y. Development and Validation of the Disability Index Among Older Adults. J Gerontol A Biol Sci Med Sci. 2023 Jan 26;78(1):111-119. doi: 10.1093/gerona/glac059. PMID: 35271717; PMCID: PMC9879748.
Zochling J. Measures of symptoms and disease status in ankylosing spondylitis: Ankylosing Spondylitis Disease Activity Score (ASDAS), Ankylosing Spondylitis Quality of Life Scale (ASQoL), Bath Ankylosing Spondylitis Disease Activity Index (BASDAI), Bath Ankylosing Spondylitis Functional Index (BASFI), Bath Ankylosing Spondylitis Global Score (BAS-G), Bath Ankylosing Spondylitis Metrology Index (BASMI), Dougados Functional Index (DFI), and Health Assessment Questionnaire for the Spondylarthropathies (HAQ-S). Arthritis Care Res (Hoboken). 2011 Nov;63 Suppl 11:547-58. doi: 10.1002/acr.20575. PMID: 22588768.
For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A mitochondrial complex I modulator (MCIM) compound for use in the treatment of arthritis in a patient, wherein the treatment; i) increases mobility in one or more joints of the patient, ii) inhibits loss of mobility in one or more joints of the patient, iii) reduces patient fatigue, iv) reduces pain intensity experienced by a patient, and/or v) improves a patients' Quality of Life (QoL) as measured using a clinical scoring method,
wherein the MCIM compound is a compound, or a pharmaceutically acceptable salt, hydrate, or solvate thereof, of the following formula: 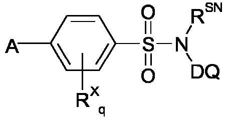 wherein:
-A is independently: 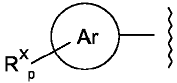
-Ar is independently phenyl, pyridinyl, or pyrimidinyl; and
p is independently an integer from 0 to 3;
and wherein:
q is independently an integer from 0 to 3;
and wherein:
-R^{SN} is independently -H or saturated aliphatic C₁₋₄alkyl;
and wherein:
-DQ is independently -D¹-Q¹ or -D²=O;
-D¹- is independently cyclopentane-di-yl, cyclohexane-di-yl, cycloheptane-di-yl, bicyclo[3.1.1]heptane-di-yl, or bicyclo[3.2.1]octane-di-yl, and is optionally substituted with one or more groups -R^{D};
-D²= is independently cyclopentane-yl-ylidene, cyclohexane-yl-ylidene, cycloheptane-yl-ylidene, bicyclo[3.1.1]heptane-yl-ylidene, or bicyclo[3.2.1]octane-yl-ylidene, and is optionally substituted with one or more groups -R^{D};
each -R^{D} is independently selected
from -F, -Cl, -Br, -I, -R^{DD}, -CF₃, -OH, -OR^{DD}, -NH₂, -NHR^{DD}, and -NR^{DD}₂; and
each -R^{DD} is independently saturated aliphatic C₁₋₄alkyl;
and wherein -Q¹ is independently selected from: 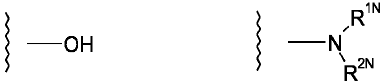 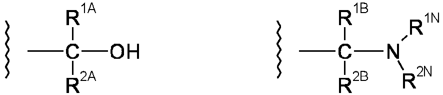 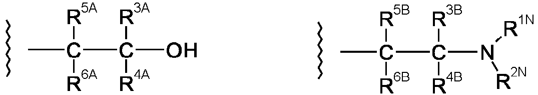 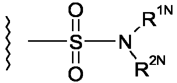
wherein:
each -R^{1N} is independently -H, -R^{CN}, or -R^{CF};
each -R^{2N} is independently -H, -R^{CN}, or -R^{CF};
each -R^{CN} is independently saturated aliphatic C₁₋₄alkyl; and
each -R^{CF}, if present, is independently saturated aliphatic C₁₋₄fluoroalkyl;
or:
-NR^{1N}R^{2N} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
-R^{1A} is independently -H, -R^{C}, or -R^{F}; and
-R^{2A} is independently -H, -R^{C}, or -R^{F};
or -R^{1A} and -R^{2A} together form a saturated aliphatic C₂₋₄alkylene group;
-R^{1B} is independently -H, -R^{C}, or -R^{F}; and
-R^{2B} is independently -H, -R^{C}, or -R^{F};
or -R^{1B} and -R^{2B} together form a saturated aliphatic C₂₋₄alkylene group;
or -R^{1B} and -R^{2B} together form =O;
-R^{3A} is independently -H, -R^{C}, or -R^{F}; and
-R^{4A} is independently -H, -R^{C}, or -R^{F};
or -R^{3A} and -R^{4A} together form a saturated aliphatic C₂₋₄alkylene group;
-R^{5A} is independently -H, -R^{C}, -R^{F}, or -R^{J}; and
-R^{6A} is independently -H, -R^{C}, or -R^{F};
or -R^{5A} and -R^{6A} together form a saturated aliphatic C₂₋₄alkylene group;
-R^{3B} is independently -H, -R^{C}, or -R^{F}; and
-R^{4B} is independently -H, -R^{C}, or -R^{F};
or -R^{3B} and -R^{4B} together form a saturated aliphatic C₂₋₄alkylene group;
-R^{5B} is independently -H, -R^{C}, -R^{F}, -OH, or -OR^{O}; and
-R^{6B} is independently -H, -R^{C}, or -R^{F};
or -R^{5B} and -R^{6B} together form a saturated aliphatic C₂₋₄alkylene group;
each -R^{C} is independently saturated aliphatic C₁₋₄alkyl;
each -R^{F} is independently saturated aliphatic C₁₋₄fluoroalkyl;
-R^{O} is independently saturated aliphatic C₁₋₄alkyl;
-R^{J} is independently -NH₂, -NHR^{JN1}, -NR^{JN1}₂, or -NR^{JN2}R^{JN3};
each -R^{JN1} is independently saturated aliphatic C₁₋₄alkyl; and
-NR^{JN2}R^{JN3} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
and wherein each -R^{X} is independently:
-F, -Cl, -Br, -I,
-R^{XX},
-OH, -OR^{XX},
-SH, -SR^{XX},
-CF₃, -OCF₃, -SCF₃,
-NH₂, -NHR^{XX}, -NR^{XX}₂, -NR^{YY}R^{ZZ},
-C(=O)R^{XX}, -OC(=O)R^{XX},
-C(=O)OH, -C(=O)OR^{XX},
-C(=O)NH₂, -C(=O)NHR^{XX}, -C(=O)NR^{XX}₂, -C(=O)NR^{YY}R^{ZZ},
-OC(=O)NH₂, -OC(=O)NHR^{XX}, -OC(=O)NR^{XX}₂, -OC(=O)NR^{YY}R^{ZZ},
-NHC(=O)R^{XX}, -NR^{XX}C(=O)R^{XX},
-NHC(=O)OR^{XX}, -NR^{XX}C(=O)OR^{XX},
-NHC(=O)NH₂, -NHC(=O)NHR^{XX}, -NHC(=O)NR^{XX}₂, -NHC(=O)NR^{YY}R^{ZZ},
-NR^{XX}C(=O)NH₂, -NR^{XX}C(=O)NHR^{XX}, -NR^{XX}C(=O)NR^{XX}₂, -NR^{XX}C(=O)NR^{YY}R^{ZZ},
-CN,
-NO₂,
-S(=O)₂NH₂, -S(=O)₂NHR^{XX}, -S(=O)₂NR^{XX}₂, -S(=O)₂NR^{YY}R^{ZZ},
-S(=O)R^{XX}, -S(=O)₂R^{XX}, -OS(=O)₂R^{XX}, -S(=O)₂OH, or -S(=O)₂OR^{XX};
wherein:
each -R^{XX} is independently saturated aliphatic C₁₋₆alkyl, phenyl, or benzyl, wherein said phenyl and benzyl are optionally substituted with one or more groups selected
from: -F, -Cl, -Br, -I, -CF₃, -OCF₃, -R^{XXX}, -OH, -OR^{XXX}, or -SR^{XXX}, wherein each -R^{XXX} is independently saturated aliphatic C₁₋₄alkyl; and
each -NR^{YY}R^{ZZ} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl.

2. The MCIM compound for use according to claim 1, wherein the arthritis is DMARD-inadequate response (IR) arthritis, csDMARD-IR arthritis, methotrexate-IR (MTX-IR) arthritis, bDMARD-IR, tsDMARD-IR, DMARD-refractory arthritis, csDMARD-refractory arthritis, methotrexate-refractory arthritis, bDMARD-refractory arthritis, tsDMARD-refractory arthritis, difficult-to-treat arthritis.

3. The MCIM compound for use according to claim 1 or claim 2, wherein the arthritis is rheumatoid arthritis (RA), psoriatic arthritis, osteoarthritis, spondyloarthritis, reactive arthritis, infectious arthritis, or juvenile idiopathic arthritis, or wherein the arthritis is a symptom of or caused by systemic lupus erythematosus, osteoporosis, osteopenia, ankylosing spondylitis, gout, scleroderma, or Sjogren's syndrome.

4. The MCIM compound for use according to any one of the preceding claims, wherein the arthritis is rheumatoid arthritis.

5. The MCIM compound for use according to any one of the preceding claims, wherein the arthritis is active arthritis and classified as mild, moderate or severe according to the DAS28 scoring methodology, or wherein the arthritis is in remission.

6. The MCIM compound for use according to any one of the preceding claims, wherein treatment with the MCIM compound inhibits or reverses structural progression of arthritis, inhibits or reverses bone erosion, inhibits or reverses cartilage loss, reduces osteitis, reduces rheumatoid factor in patient blood, and/or reduces C-reactive protein in patient blood.

7. A mitochondrial complex I modulator (MCIM) compound for use in the treatment of physical functional decline in a patient and/or a functional disability in a patient, wherein the treatment i) increases mobility in one or more joints of the patient, ii) inhibits loss of mobility in one or more joints of the patient, iii) reduces patient fatigue, iv) reduces pain intensity experienced by the patient, and/or v) improves Quality of Life (QoL) of the patient as measured using a clinical scoring method,
wherein the MCIM compound is a compound, or a pharmaceutically acceptable salt, hydrate, or solvate thereof, of the following formula: 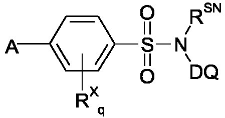
wherein:
-A is independently: 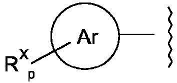
-Ar is independently phenyl, pyridinyl, or pyrimidinyl; and
p is independently an integer from 0 to 3;
and wherein:
q is independently an integer from 0 to 3;
and wherein:
-R^{SN} is independently -H or saturated aliphatic C₁₋₄alkyl;
and wherein:
-DQ is independently -D¹-Q¹ or -D²=O;
-D¹- is independently cyclopentane-di-yl, cyclohexane-di-yl, cycloheptane-di-yl, bicyclo[3.1.1]heptane-di-yl, or bicyclo[3.2.1]octane-di-yl, and is optionally substituted with one or more groups -R^{D};
-D²= is independently cyclopentane-yl-ylidene, cyclohexane-yl-ylidene, cycloheptane-yl-ylidene, bicyclo[3.1.1]heptane-yl-ylidene, or bicyclo[3.2.1]octane-yl-ylidene, and is optionally substituted with one or more groups -R^{D};
each -R^{D} is independently selected
from -F, -Cl, -Br, -I, -R^{DD}, -CF₃, -OH, -OR^{DD}, -NH₂, -NHR^{DD}, and -NR^{DD}₂; and
each -R^{DD} is independently saturated aliphatic C₁₋₄alkyl;
and wherein -Q¹ is independently selected from: 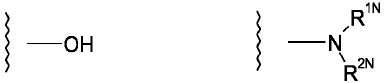 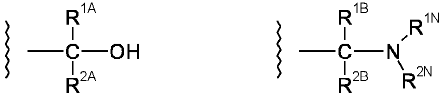 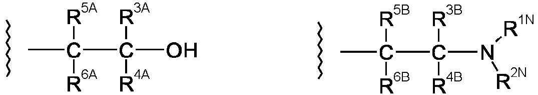 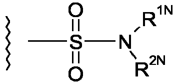 wherein:
each -R^{1N} is independently -H, -R^{CN}, or -R^{CF};
each -R^{2N} is independently -H, -R^{CN}, or -R^{CF};
each -R^{CN} is independently saturated aliphatic C₁₋₄alkyl; and
each -R^{CF}, if present, is independently saturated aliphatic C₁₋₄fluoroalkyl;
or:
-NR^{1N}R^{2N} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
-R^{1A} is independently -H, -R^{C}, or -R^{F}; and
-R^{2A} is independently -H, -R^{C}, or -R^{F};
or -R^{1A} and -R^{2A} together form a saturated aliphatic C₂₋₄alkylene group;
-R^{1B} is independently -H, -R^{C}, or -R^{F}; and
-R^{2B} is independently -H, -R^{C}, or -R^{F};
or -R^{1B} and -R^{2B} together form a saturated aliphatic C₂₋₄alkylene group;
or -R^{1B} and -R^{2B} together form =O;
-R^{3A} is independently -H, -R^{C}, or -R^{F}; and
-R^{4A} is independently -H, -R^{C}, or -R^{F};
or -R^{3A} and -R^{4A} together form a saturated aliphatic C₂₋₄alkylene group;
-R^{5A} is independently -H, -R^{C}, -R^{F}, or -R^{J}; and
-R^{6A} is independently -H, -R^{C}, or -R^{F};
or -R^{5A} and -R^{6A} together form a saturated aliphatic C₂₋₄alkylene group;
-R^{3B} is independently -H, -R^{C}, or -R^{F}; and
-R^{4B} is independently -H, -R^{C}, or -R^{F};
or -R^{3B} and -R^{4B} together form a saturated aliphatic C₂₋₄alkylene group;
-R^{5B} is independently -H, -R^{C}, -R^{F}, -OH, or -OR^{O}; and
-R^{6B} is independently -H, -R^{C}, or -R^{F};
or -R^{5B} and -R^{6B} together form a saturated aliphatic C₂₋₄alkylene group;
each -R^{C} is independently saturated aliphatic C₁₋₄alkyl;
each -R^{F} is independently saturated aliphatic C₁₋₄fluoroalkyl;
-R^{O} is independently saturated aliphatic C₁₋₄alkyl;
-R^{J} is independently -NH₂, -NHR^{JN1}, -NR^{JN1}₂, or -NR^{JN2}R^{JN3};
each -R^{JN1} is independently saturated aliphatic C₁₋₄alkyl; and
-NR^{JN2}R^{JN3} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl;
and wherein each -R^{X} is independently:
-F, -Cl, -Br, -I,
-R^{XX},
-OH, -OR^{XX},
-SH, -SR^{XX},
-CF₃, -OCF₃, -SCF₃,
-NH₂, -NHR^{XX}, -NR^{XX}₂, -NR^{YY}R^{ZZ},
-C(=O)R^{XX}, -OC(=O)R^{XX},
-C(=O)OH, -C(=O)OR^{XX},
-C(=O)NH₂, -C(=O)NHR^{XX}, -C(=O)NR^{XX}₂, -C(=O)NR^{YY}R^{ZZ},
-OC(=O)NH₂, -OC(=O)NHR^{XX}, -OC(=O)NR^{XX}₂, -OC(=O)NR^{YY}R^{ZZ},
-NHC(=O)R^{XX}, -NR^{XX}C(=O)R^{XX},
-NHC(=O)OR^{XX}, -NR^{XX}C(=O)OR^{XX},
-NHC(=O)NH₂, -NHC(=O)NHR^{XX}, -NHC(=O)NR^{XX}₂, -NHC(=O)NR^{YY}R^{ZZ},
-NR^{XX}C(=O)NH₂, -NR^{XX}C(=O)NHR^{XX}, -NR^{XX}C(=O)NR^{XX}₂, -NR^{XX}C(=O)NR^{YY}R^{ZZ},
-CN,
-NO₂,
-S(=O)₂NH₂, -S(=O)₂NHR^{XX}, -S(=O)₂NR^{XX}₂, -S(=O)₂NR^{YY}R^{ZZ},
-S(=O)R^{XX}, -S(=O)₂R^{XX}, -OS(=O)₂R^{XX}, -S(=O)₂OH, or -S(=O)₂OR^{XX};
wherein:
each -R^{XX} is independently saturated aliphatic C₁₋₆alkyl, phenyl, or benzyl, wherein said phenyl and benzyl are optionally substituted with one or more groups selected
from: -F, -Cl, -Br, -I, -CF₃, -OCF₃, -R^{XXX}, -OH, -OR^{XXX}, or -SR^{XXX}, wherein each -R^{XXX} is independently saturated aliphatic C₁₋₄alkyl; and
each -NR^{YY}R^{ZZ} is independently azetidino, pyrrolidino, imidazolidino, pyrazolidino, piperidino, piperazino, morpholino, thiomorpholino, azepino, or diazepino, each optionally substituted with one or more groups independently selected from saturated aliphatic C₁₋₄alkyl.

8. The MCIM compound for use according to claim 7, wherein the physical functional decline comprises musculoskeletal decline and/or the functional disability comprises a musculoskeletal disability.

9. The MCIM compound for use according to claim 7 or claim 8, wherein the physical functional decline comprises degradation or damage of one or more bones and/or the functional disability comprises degradation or damage of one or more bones.

10. The MCIM compound for use according to any one of claims 7-9, wherein the physical functional decline comprises degradation or damage to one or more joints and/or the functional disability comprises degradation or damage to one or more joints.

11. The MCIM compound for use according to any one of claims 7-10, wherein the physical functional decline and/or the functional disability is the result of one or more of; an arthritis, optionally RA and/or osteoarthritis; osteoporosis; and/or osteopenia.

12. The MCIM compound for use according to any one of claims 7-10, wherein the physical function decline is age-related physical functional decline and/or the functional disability is an age-related functional disability.

13. The MCIM compound for use according to any one of the preceding claims, wherein the clinical scoring method is selected from one or more of the following: HAQ-DI, EQ-5D, EQ5D-3L, EQ-5D-5L, EULAR-RAID, and FACIT-F.

14. The MCIM compound for use according to any one of the preceding claims, wherein
QoL is assessed using one or more of the clinical scoring systems selected from HAQ-disability index, EULAR-RAID, FACIT-F and EQ-5D-5L;
joint mobility is assessed using RAMRIS and/or EQ-5D-5L; and/or
patient fatigue is assessed using FACIT-F.

15. The MCIM compound for use according to any one of the preceding claims, wherein:
-Ar is independently phenyl or pyridinyl;
p is independently an integer from 1 to 3;
q is independently 0 or 1;
-R^{SN} is -H;
-DQ is -D¹-Q¹;
-D¹- is cyclohexane-di-yl and is optionally substituted with one group -R^{D};
-R° is -R^{DD}; and -R^{DD} is methyl;
-Q¹ is: 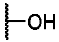
each -R^{X} is independently:
-F, -Cl, -Br, -R^{XX}, -OR^{XX}, -CF₃, or -CN; and
each -R^{XX} is independently methyl.

16. The MCIM compound for use according to claim 15, wherein:
-A is: 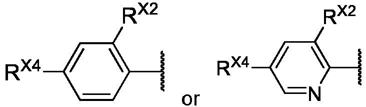
q is independently 0 or 1;
-R^{SN} is -H;
-DQ is: 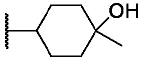
-R^{X} is independently -F or -Cl;
-R^{X2} is independently -F, -Cl, -CN, or -CF₃;
-R^{X4} is independently -F, -Cl, or -CN.

17. The MCIM compound for use according to any one of claims 1-16, wherein the MCIM compound is selected from compounds of the following formulae: 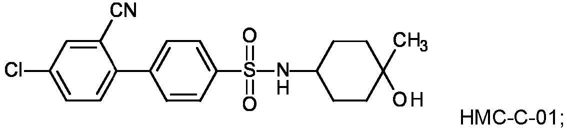 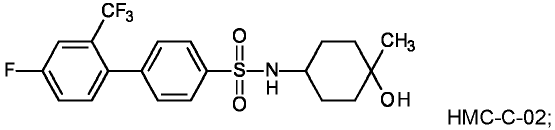 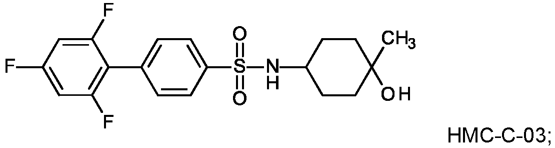 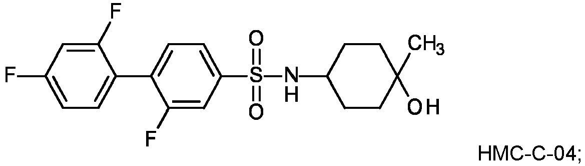 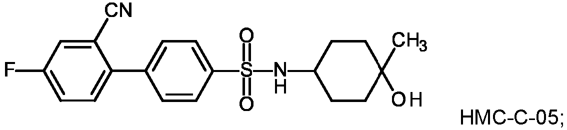 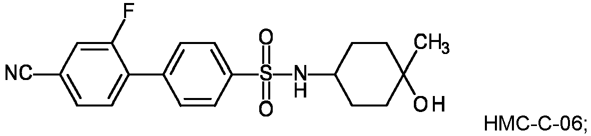 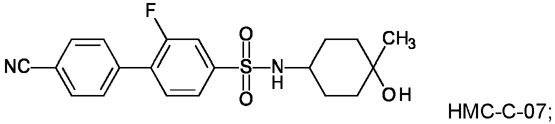 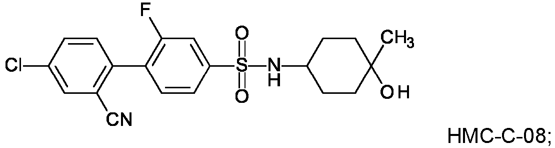 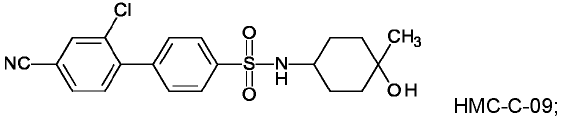 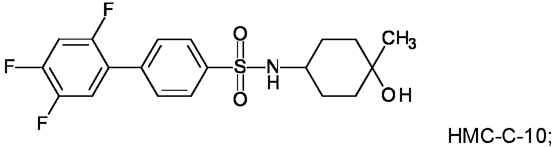 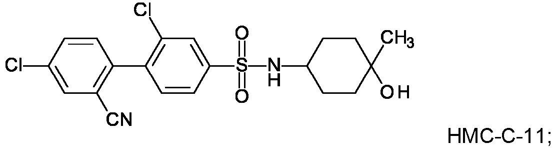 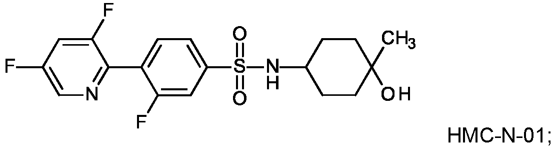 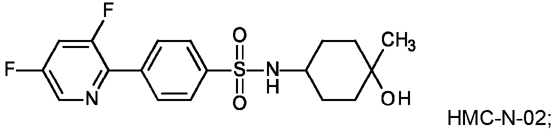 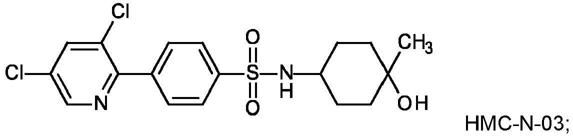 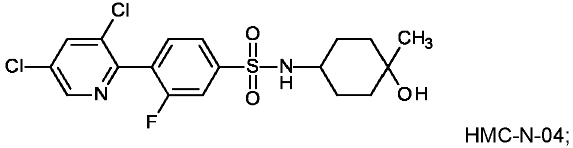 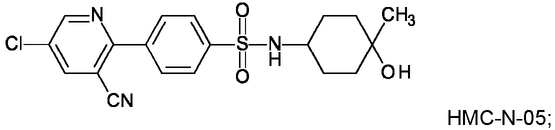 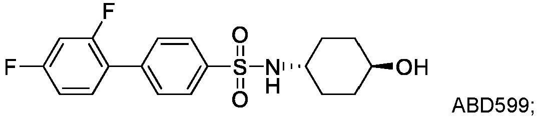 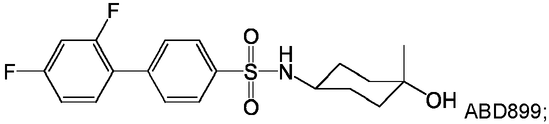 and 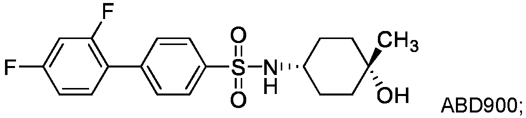 or a pharmaceutically acceptable salt, hydrate, or solvate thereof.

18. The MCIM compound for use according to any one of claims 1-16, wherein the MCIM compound is selected from compounds of the following formulae: 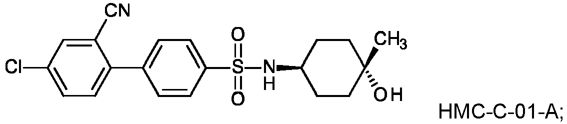 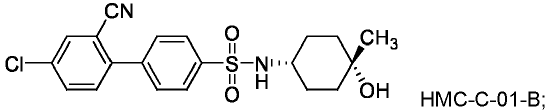 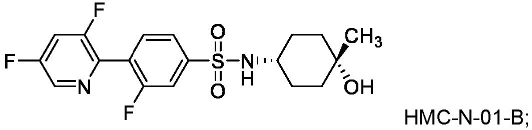 and 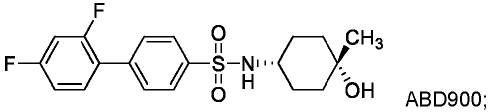 or a pharmaceutically acceptable salt, hydrate, or solvate thereof.

19. The MCIM compound for use according to any one of claims claim 1-16, wherein the MCIM compound is a compound of the following formula, or a pharmaceutically acceptable salt, hydrate, or solvate thereof:

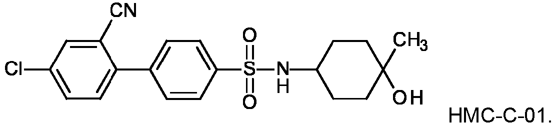

20. The MCIM compound for use according to any one of claims 1-16, wherein the MCIM compound is a compound of the following formula, or a pharmaceutically acceptable salt, hydrate, or solvate thereof:

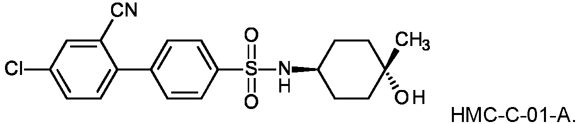

21. The MCIM compound for use according to any one of the preceding claims, wherein the treatment comprises the separate, sequential or simultaneous administration of one or more disease modifying antirheumatic drugs (DMARD).

22. The MCIM compound for use according to claim 21, wherein the one or more DMARDs comprise a csDMARD selected from methotrexate, azathioprine, chloroquine, hydroxychloroquine, ciclosporin, D-penicillamine, gold salt, sodium aurothiomalate, auranofin, leflunomide, sulfasalazine, and minocycline, or any combination of the foregoing.

23. The MCIM compound for use according to claim 22, wherein the one or more DMARDs comprise methotrexate.

24. The MCIM compound for use according to any of one claims 21 to 23, wherein the one or more DMARDs comprise one or more bDMARDs and/or tsDMARDs; optionally wherein
the bDMARD comprises one or more bDMARDs selected from adalimumab, certolizumab, etanercept, golimumab, infliximab, abatacept, anakinra, rituximab, sarilumab, tocilizumab, secukinumab, ixekizumab, bimekizumab, ustekinumab, risankizumab, guselkumab, and belimumab; and/or
the tsDMARD comprises one or more tsDMARDs selected from upadacitinib, baricitinib, filgotinib, tofacitinib, deucravacitinib, and apremilast.

25. The MCIM compound for use according to any one of the preceding claims, wherein the MCIM compound is administered at a dose of 0.5-500 mg/day, optionally at a dose of 5-40 mg/day, optionally at a dose of 5, 20 or 40 mg/day.

26. The MCIM compound for use according to any one of the preceding claims, wherein treatment with the MCIM compound does not cause immunosuppression.
